# EUROPEAN PATENT APPLICATION

(11) **EP 1 662 258 A1**
(43) Date of publication of application: **31.05.2006**
(21) Application number: 04028022.4
(22) Date of filing: 25.11.2004
(51) Int. Cl.: G01N 33/68, G01N 33/543, C12P 21/00

(54) **A substrate free in vitro protein kinase assay**

(71) Applicant: Sirenade Pharmaceuticals AG, 82152 Martinsried (DE)
(72) Inventor: Bieger, Boris, 81379 München (DE); Obermeier, Axel, 81379 München (DE)
(74) Representative: Graf von Stosch, Andreas

(57) **Abstract**

The present invention relates to methods for identifying, selecting and/or characterizing compounds capable of modulating auto-phosphorylation activity of protein-tyrosine kinases (PTKs), to compounds identified thereby and to their use in the treatment of diseases related to a protein tyrosine kinase activity, such as cancer or osteoporesis.

SEQUENCE LISTING

<110>  Sirenade Pharmaceuticals AG

<120>  A substrate free in vitro protein kinase assay

<130>  SI07P003EP

<160>  17

<170>  PatentIn version 3.1

<210>  1
<211>  475
<212>  PRT
<213>  Homo sapiens

<220>
<221>  MISC_FEATURE
<222>  (1)..(475)
<223>  Xaa = unknown or other

<220>
<221>  MISC_FEATURE
<223>  sequence of 6His-Src v1(human): (Figure 17)

<400>  1

Met Ser Tyr Tyr His His His His His His Asp Tyr Asp Ile Pro Thr
1               5                   10                  15

Thr Glu Asn Leu Tyr Phe Gln Gly Thr Xaa Thr Phe Val Ala Leu Tyr
            20                  25                  30

Asp Tyr Glu Ser Arg Thr Glu Thr Asp Leu Ser Phe Lys Lys Gly Glu

        35                  40                  45

Arg Leu Gln Ile Val Asn Asn Thr Glu Gly Asp Trp Trp Leu Ala His
    50                  55                  60

Ser Leu Ser Thr Gly Gln Thr Gly Tyr Ile Pro Ser Asn Tyr Val Ala
65                  70                  75                  80

Pro Ser Asp Ser Ile Gln Ala Glu Glu Trp Tyr Phe Gly Lys Ile Thr
                85                  90                  95

Arg Arg Glu Ser Glu Arg Leu Leu Leu Asn Ala Glu Asn Pro Arg Gly
            100                 105                 110

Thr Phe Leu Val Arg Glu Ser Glu Thr Thr Lys Gly Ala Tyr Cys Leu
        115                 120                 125

Ser Val Ser Asp Phe Asp Asn Ala Lys Gly Leu Asn Val Lys His Tyr
    130                 135                 140

Lys Ile Arg Lys Leu Asp Ser Gly Gly Phe Tyr Ile Thr Ser Arg Thr
145                 150                 155                 160

Gln Phe Asn Ser Leu Gln Gln Leu Val Ala Tyr Tyr Ser Lys His Ala
                165                 170                 175

Asp Gly Leu Cys His Arg Leu Thr Thr Val Cys Pro Thr Ser Lys Pro
            180                 185                 190

Gln Thr Gln Gly Leu Ala Lys Asp Ala Trp Glu Ile Pro Arg Glu Ser
        195                 200                 205

Leu Arg Leu Glu Val Lys Leu Gly Gln Gly Cys Phe Gly Glu Val Trp
    210                 215                 220

Met Gly Thr Trp Asn Gly Thr Thr Arg Val Ala Ile Lys Thr Leu Lys
225                 230                 235                 240

Pro Gly Thr Met Ser Pro Glu Ala Phe Leu Gln Glu Ala Gln Val Met
                245                 250                 255

Lys Lys Leu Arg His Glu Lys Leu Val Gln Leu Tyr Ala Val Val Ser
            260                 265                 270

Glu Glu Pro Ile Tyr Ile Val Thr Glu Tyr Met Ser Lys Gly Ser Leu
        275                 280                 285

Leu Asp Phe Leu Lys Gly Glu Thr Gly Lys Tyr Leu Arg Leu Pro Gln
    290                 295                 300

Leu Val Asp Met Ala Ala Gln Ile Ala Ser Gly Met Ala Tyr Val Glu
305                 310                 315                 320

Arg Met Asn Tyr Val His Arg Asp Leu Arg Ala Ala Asn Ile Leu Val
                325                 330                 335

Gly Glu Asn Leu Val Cys Lys Val Ala Asp Phe Gly Leu Ala Arg Leu
            340                 345                 350

Ile Glu Asp Asn Glu Tyr Thr Ala Arg Gln Gly Ala Lys Phe Pro Ile
        355                 360                 365

Lys Trp Thr Ala Pro Glu Ala Ala Leu Tyr Gly Arg Phe Thr Ile Lys
    370                 375                 380

Ser Asp Val Trp Ser Phe Gly Ile Leu Leu Thr Glu Leu Thr Thr Lys
385                 390                 395                 400

Gly Arg Val Pro Tyr Pro Gly Met Val Asn Arg Glu Val Leu Asp Gln
                405                 410                 415

Val Glu Arg Gly Tyr Arg Met Pro Cys Pro Pro Glu Cys Pro Glu Ser
            420                 425                 430

Leu His Asp Leu Met Cys Gln Cys Trp Arg Lys Glu Pro Glu Glu Arg
        435                 440                 445

Pro Thr Phe Glu Tyr Leu Gln Ala Phe Leu Glu Asp Tyr Phe Thr Ser
    450                 455                 460

Thr Glu Pro Gln Tyr Gln Pro Gly Glu Asn Leu
465                 470                 475

<210>  2

<211>  473

<212>  PRT

<213>  Homo sapiens


<220>

<221>  MISC_FEATURE

<222>  (1)..(473)

<223>  Xaa = unknown or other

<220>

<221>  MISC_FEATURE

<223>  sequence of 6His-Src v2 (human) (Figure 18)

<400>  2

Met Ser Tyr Tyr His His His His His His Asp Tyr Asp Ile Pro Thr
1               5                   10                  15

Thr Glu Asn Leu Tyr Phe Gln Gly Thr Xaa Thr Phe Val Ala Leu Tyr
            20                  25                  30

Asp Tyr Glu Ser Arg Thr Glu Thr Asp Leu Ser Phe Lys Lys Gly Glu
        35                  40                  45

Arg Leu Gln Ile Val Asn Asn Thr Glu Gly Asp Trp Trp Leu Ala His
    50                  55                  60

Ser Leu Ser Thr Gly Gln Thr Gly Tyr Ile Pro Ser Asn Tyr Val Ala
65                  70                  75                  80

Pro Ser Asp Ser Ile Gln Ala Glu Glu Trp Tyr Phe Gly Lys Ile Thr
                85                  90                  95

Arg Arg Glu Ser Glu Arg Leu Leu Leu Asn Ala Glu Asn Pro Arg Gly
            100                 105                 110

Thr Phe Leu Val Arg Glu Ser Glu Thr Thr Lys Gly Ala Tyr Cys Leu
        115                 120                 125

Ser Val Ser Asp Phe Asp Asn Ala Lys Gly Leu Asn Val Lys His Tyr
    130                 135                 140

Lys Ile Arg Lys Leu Asp Ser Gly Gly Phe Tyr Ile Thr Ser Arg Thr
145                 150                 155                 160

Gln Phe Asn Ser Leu Gln Gln Leu Val Ala Tyr Tyr Ser Lys His Ala
                165                 170                 175

Asp Gly Leu Cys His Arg Leu Thr Thr Val Cys Pro Thr Ser Lys Pro
            180                 185                 190

Gln Thr Gln Gly Leu Ala Lys Asp Ala Trp Glu Ile Pro Arg Glu Ser
            195                 200                 205

Leu Arg Leu Glu Val Lys Leu Gly Gln Gly Cys Phe Gly Glu Val Trp
        210                 215                 220

Met Gly Thr Trp Asn Gly Thr Thr Arg Val Ala Ile Lys Thr Leu Lys
225                 230                 235                 240

Pro Gly Thr Met Ser Pro Glu Ala Phe Leu Gln Glu Ala Gln Val Met
                245                 250                 255

Lys Lys Leu Arg His Glu Lys Leu Val Gln Leu Tyr Ala Val Val Ser
            260                 265                 270

Glu Glu Pro Ile Tyr Ile Val Thr Glu Tyr Met Ser Lys Gly Ser Leu
        275                 280                 285

Leu Asp Phe Leu Lys Gly Glu Thr Gly Lys Tyr Leu Arg Leu Pro Gln
    290                 295                 300

Leu Val Asp Met Ala Ala Gln Ile Ala Ser Gly Met Ala Tyr Val Glu
305                 310                 315                 320

Arg Met Asn Tyr Val His Arg Asp Leu Arg Ala Ala Asn Ile Leu Val
                325                 330                 335

Gly Glu Asn Leu Val Cys Lys Val Ala Asp Phe Gly Leu Ala Arg Leu
            340                 345                 350

Ile Glu Asp Asn Glu Tyr Thr Ala Arg Gln Gly Ala Lys Phe Pro Ile
        355                 360                 365

Lys Trp Thr Ala Pro Glu Ala Ala Leu Tyr Gly Arg Phe Thr Ile Lys
    370                 375                 380

Ser Asp Val Trp Ser Phe Gly Ile Leu Leu Thr Glu Leu Thr Thr Lys
385                 390                 395                 400

Gly Arg Val Pro Tyr Pro Gly Met Val Asn Arg Glu Val Leu Asp Gln
                405                 410                 415

Val Glu Arg Gly Tyr Arg Met Pro Cys Pro Pro Glu Cys Pro Glu Ser
            420                 425                 430

Leu His Asp Leu Met Cys Gln Cys Trp Arg Lys Glu Pro Glu Glu Arg
        435                 440                 445

Pro Thr Phe Glu Tyr Leu Gln Ala Phe Leu Glu Asp Tyr Phe Thr Ser
    450                 455                 460

Thr Glu Pro Gln Tyr Glu Glu Ile Pro
465                 470

<210>  3

<211>  470

<212>  PRT

<213>  Homo sapiens

<220>

<221>  MISC_FEATURE

<223>  sequence of 6His-hck (human) (Figure 19)

<400>  3

Met Ser Tyr Tyr His His His His His His Asp Tyr Asp Ile Pro Thr
1               5                   10                  15

Thr Glu Asn Leu Tyr Phe Gln Gly Ile Ile Val Val Ala Leu Tyr Asp
            20                  25                  30

Tyr Glu Ala Ile His His Glu Asp Leu Ser Phe Gln Lys Gly Asp Gln
        35                  40                  45

Met Val Val Leu Glu Glu Ser Gly Glu Trp Trp Lys Ala Arg Ser Leu
    50                  55                  60

Ala Thr Arg Lys Glu Gly Tyr Ile Pro Ser Asn Tyr Val Ala Arg Val
65                  70                  75                  80

Asp Ser Leu Glu Thr Glu Glu Trp Phe Phe Lys Gly Ile Ser Arg Lys
                85                  90                  95

Asp Ala Glu Arg Gln Leu Leu Ala Pro Gly Asn Met Leu Gly Ser Phe
            100                 105                 110

Met Ile Arg Asp Ser Glu Thr Thr Lys Gly Ser Tyr Ser Leu Ser Val
        115                 120                 125

Arg Asp Tyr Asp Pro Arg Gln Gly Asp Thr Val Lys His Tyr Lys Ile

130                 135                 140

Arg Thr Leu Asp Asn Gly Gly Phe Tyr Ile Ser Pro Arg Ser Thr Phe
145                 150                 155                 160

Ser Thr Leu Gln Glu Leu Val Asp His Tyr Lys Lys Gly Asn Asp Gly
                165                 170                 175

Leu Cys Gln Lys Leu Ser Val Pro Cys Met Ser Ser Lys Pro Gln Lys
            180                 185                 190

Pro Trp Glu Lys Asp Ala Trp Glu Ile Pro Arg Glu Ser Leu Lys Leu
        195                 200                 205

Glu Lys Lys Leu Gly Ala Gly Gln Phe Gly Glu Val Trp Met Ala Thr
    210                 215                 220

Tyr Asn Lys His Thr Lys Val Ala Val Lys Thr Met Lys Pro Gly Ser
225                 230                 235                 240

Met Ser Val Glu Ala Phe Leu Ala Glu Ala Asn Val Met Lys Thr Leu
                245                 250                 255

Gln His Asp Lys Leu Val Lys Leu His Ala Val Val Thr Lys Glu Pro
            260                 265                 270

Ile Tyr Ile Ile Thr Glu Phe Met Ala Lys Gly Ser Leu Leu Asp Phe
        275                 280                 285

Leu Lys Ser Asp Glu Gly Ser Lys Gln Pro Leu Pro Lys Leu Ile Asp
    290                 295                 300

Phe Ser Ala Gln Ile Ala Glu Gly Met Ala Phe Ile Glu Gln Arg Asn
305                 310                 315                 320

Tyr Ile His Arg Asp Leu Arg Ala Ala Asn Ile Leu Val Ser Ala Ser
                325                 330                 335

Leu Val Cys Lys Ile Ala Asp Phe Gly Leu Ala Arg Val Ile Glu Asp
            340                 345                 350

Asn Glu Tyr Thr Ala Arg Glu Gly Ala Lys Phe Pro Ile Lys Trp Thr
        355                 360                 365

Ala Pro Glu Ala Ile Asn Phe Gly Ser Phe Thr Ile Lys Ser Asp Val
    370                 375                 380

Trp Ser Phe Gly Ile Leu Leu Met Glu Ile Val Thr Tyr Gly Arg Ile
385                 390                 395                 400

Pro Tyr Pro Gly Met Ser Asn Pro Glu Val Ile Arg Ala Leu Glu Arg
                405                 410                 415

Gly Tyr Arg Met Pro Arg Pro Glu Asn Cys Pro Glu Glu Leu Tyr Asn
            420                 425                 430

Ile Met Met Arg Cys Trp Lys Asn Arg Pro Glu Glu Arg Pro Thr Phe
        435                 440                 445

Glu Tyr Ile Gln Ser Val Leu Asp Asp Phe Tyr Thr Ala Thr Glu Ser
    450                 455                 460

Gln Tyr Glu Glu Ile Pro
465                 470

<210>  4

<211>  536

<212>  PRT

<213>  mammalian

<220>

<221>  MISC_FEATURE

<223>  sequence of (wt) Src

<400>  4

<210>  5

<211>  543

<212>  PRT

<213>  mammalian

<220>

<221>  MISC_FEATURE

<223>  sequence of (wt) Yes

<400>  5

Met Gly Cys Ile Lys Ser Lys Glu Asn Lys Ser Pro Ala Ile Lys Tyr
1               5                   10                  15

Arg Pro Glu Asn Thr Pro Glu Pro Val Ser Thr Ser Val Ser His Tyr
            20                  25                  30

Gly Ala Glu Pro Thr Thr Val Ser Pro Cys Pro Ser Ser Ser Ala Lys
        35                  40                  45

Gly Thr Ala Val Asn Phe Ser Ser Leu Ser Met Thr Pro Phe Gly Gly
    50                  55                  60

Ser Ser Gly Val Thr Pro Phe Gly Gly Ala Ser Ser Ser Phe Ser Val
65                  70                  75                  80

Val Pro Ser Ser Tyr Pro Ala Gly Leu Thr Gly Gly Val Thr Ile Phe
                85                  90                  95

Val Ala Leu Tyr Asp Tyr Glu Ala Arg Thr Thr Glu Asp Leu Ser Phe
            100                 105                 110

Lys Lys Gly Glu Arg Phe Gln Ile Ile Asn Asn Thr Glu Gly Asp Trp
        115                 120                 125

Trp Glu Ala Arg Ser Ile Ala Thr Gly Lys Asn Gly Tyr Ile Pro Ser
    130                 135                 140

Asn Tyr Val Ala Pro Ala Asp Ser Ile Gln Ala Glu Glu Trp Tyr Phe
145                 150                 155                 160

Gly Lys Met Gly Arg Lys Asp Ala Glu Arg Leu Leu Leu Asn Pro Gly
                165                 170                 175

Asn Gln Arg Gly Ile Phe Leu Val Arg Glu Ser Glu Thr Thr Lys Gly
            180                 185                 190

Ala Tyr Ser Leu Ser Ile Arg Asp Trp Asp Glu Ile Arg Gly Asp Asn

195                 200                 205

Val Lys His Tyr Lys Ile Arg Lys Leu Asp Asn Gly Gly Tyr Tyr Ile
    210                 215                 220

Thr Thr Arg Ala Gln Phe Asp Thr Leu Gln Lys Leu Val Lys His Tyr
225                 230                 235                 240

Thr Glu His Ala Asp Gly Leu Cys His Lys Leu Thr Thr Val Cys Pro
                245                 250                 255

Thr Val Lys Pro Gln Thr Gln Gly Leu Ala Lys Asp Ala Trp Glu Ile
            260                 265                 270

Pro Arg Glu Ser Leu Arg Leu Glu Val Lys Leu Gly Gln Gly Cys Phe
        275                 280                 285

Gly Glu Val Trp Met Gly Thr Trp Asn Gly Thr Thr Lys Val Ala Ile
    290                 295                 300

Lys Thr Leu Lys Pro Gly Thr Met Met Pro Glu Ala Phe Leu Gln Glu
305                 310                 315                 320

Ala Gln Ile Met Lys Lys Leu Arg His Asp Lys Leu Val Pro Leu Tyr
                325                 330                 335

Ala Val Val Ser Glu Glu Pro Ile Tyr Ile Val Thr Glu Phe Met Ser
            340                 345                 350

Lys Gly Ser Leu Leu Asp Phe Leu Lys Glu Gly Asp Gly Lys Tyr Leu
        355                 360                 365

Lys Leu Pro Gln Leu Val Asp Met Ala Ala Gln Ile Ala Asp Gly Met
    370                 375                 380

Ala Tyr Ile Glu Arg Met Asn Tyr Ile His Arg Asp Leu Arg Ala Ala
385                 390                 395                 400

Asn Ile Leu Val Gly Glu Asn Leu Val Cys Lys Ile Ala Asp Phe Gly
                405                 410                 415

Leu Ala Arg Leu Ile Glu Asp Asn Glu Tyr Thr Ala Arg Gln Gly Ala
            420                 425                 430

Lys Phe Pro Ile Lys Trp Thr Ala Pro Glu Ala Ala Leu Tyr Gly Arg
        435                 440                 445

Phe Thr Ile Lys Ser Asp Val Trp Ser Phe Gly Ile Leu Gln Thr Glu
    450                 455                 460

Leu Val Thr Lys Gly Arg Val Pro Tyr Pro Gly Met Val Asn Arg Glu
465                 470                 475                 480

Val Leu Glu Gln Val Glu Arg Gly Tyr Arg Met Pro Cys Pro Gln Gly
                485                 490                 495

Cys Pro Glu Ser Leu His Glu Leu Met Asn Leu Cys Trp Lys Lys Asp
            500                 505                 510

Pro Asp Glu Arg Pro Thr Phe Glu Tyr Ile Gln Ser Phe Leu Glu Asp
        515                 520                 525

Tyr Phe Thr Ala Thr Glu Pro Gln Tyr Gln Pro Gly Glu Asn Leu
    530                 535                 540

<210>  6

<211>  537

<212>  PRT

<213>  mammalian

<220>

<221>  MISC_FEATURE

<223>  sequence of (wt) Fyn

<400>  6

Met Gly Cys Val Gln Cys Lys Asp Lys Glu Ala Thr Lys Leu Thr Glu
1               5                   10                  15

Glu Arg Asp Gly Ser Leu Asn Gln Ser Ser Gly Tyr Arg Tyr Gly Thr
            20                  25                  30

Asp Pro Thr Pro Gln His Tyr Pro Ser Phe Gly Val Thr Ser Ile Pro
        35                  40                  45

Asn Tyr Asn Asn Phe His Ala Ala Gly Gly Gln Gly Leu Thr Val Phe
    50                  55                  60

Gly Gly Val Asn Ser Ser Ser His Thr Gly Thr Leu Arg Thr Arg Gly
65                  70                  75                  80

Gly Thr Gly Val Thr Leu Phe Val Ala Leu Tyr Asp Tyr Glu Ala Arg
                85                  90                  95

Thr Glu Asp Asp Leu Ser Phe His Lys Gly Glu Lys Phe Gln Ile Leu
            100                 105                 110

Asn Ser Ser Glu Gly Asp Trp Trp Glu Ala Arg Ser Leu Thr Thr Gly
        115                 120                 125

Glu Thr Gly Tyr Ile Pro Ser Asn Tyr Val Ala Pro Val Asp Ser Ile
    130                 135                 140

Gln Ala Glu Glu Trp Tyr Phe Gly Lys Leu Gly Arg Lys Asp Ala Glu
145                 150                 155                 160

Arg Gln Leu Leu Ser Phe Gly Asn Pro Arg Gly Thr Phe Leu Ile Arg
                165                 170                 175

Glu Ser Glu Thr Thr Lys Gly Ala Tyr Ser Leu Ser Ile Arg Asp Trp
            180                 185                 190

Asp Asp Met Lys Gly Asp His Val Lys His Tyr Lys Ile Arg Lys Leu
        195                 200                 205

Asp Asn Gly Gly Tyr Tyr Ile Thr Thr Arg Ala Gln Phe Glu Thr Leu
    210                 215                 220

Gln Gln Leu Val Gln His Tyr Ser Glu Arg Ala Ala Gly Leu Cys Cys
225                 230                 235                 240

Arg Leu Val Val Pro Cys His Lys Gly Met Pro Arg Leu Thr Asp Leu
                245                 250                 255

Ser Val Lys Thr Lys Asp Val Trp Glu Ile Pro Arg Glu Ser Leu Gln
            260                 265                 270

Leu Ile Lys Arg Leu Gly Asn Gly Gln Phe Gly Glu Val Trp Met Gly
        275                 280                 285

Thr Trp Asn Gly Asn Thr Lys Val Ala Ile Lys Thr Leu Lys Pro Gly
    290                 295                 300

Thr Met Ser Pro Glu Ser Phe Leu Glu Glu Ala Gln Ile Met Lys Lys
305                 310                 315                 320

Leu Lys His Asp Lys Leu Val Gln Leu Tyr Ala Val Val Ser Glu Glu

325                 330                 335

Pro Ile Tyr Ile Val Thr Glu Tyr Met Asn Lys Gly Ser Leu Leu Asp
            340                 345                 350

Phe Leu Lys Asp Gly Glu Gly Arg Ala Leu Lys Leu Pro Asn Leu Val
        355                 360                 365

Asp Met Ala Ala Gln Val Ala Ala Gly Met Ala Tyr Ile Glu Arg Met
    370                 375                 380

Asn Tyr Ile His Arg Asp Leu Arg Ser Ala Asn Ile Leu Val Gly Asn
385                 390                 395                 400

Gly Leu Ile Cys Lys Ile Ala Asp Phe Gly Leu Ala Arg Leu Ile Glu
                405                 410                 415

Asp Asn Glu Tyr Thr Ala Arg Gln Gly Ala Lys Phe Pro Ile Lys Trp
            420                 425                 430

Thr Ala Pro Glu Ala Ala Leu Tyr Gly Arg Phe Thr Ile Lys Ser Asp
        435                 440                 445

Val Trp Ser Phe Gly Ile Leu Leu Thr Glu Leu Val Thr Lys Gly Arg
    450                 455                 460

Val Pro Tyr Pro Gly Met Asn Asn Arg Glu Val Leu Glu Gln Val Glu
465                 470                 475                 480

Arg Gly Tyr Arg Met Pro Cys Pro Gln Asp Cys Pro Ile Ser Leu His
                485                 490                 495

Glu Leu Met Ile His Cys Trp Lys Lys Asp Pro Glu Glu Arg Pro Thr
            500                 505                 510

Phe Glu Tyr Leu Gln Ser Phe Leu Glu Asp Tyr Phe Thr Ala Thr Glu
        515                 520                 525

Pro Gln Tyr Gln Pro Gly Glu Asn Leu
    530                 535

<210>  7

<211>  536

<212>  PRT

<213>  mammalian

<220>

<221>  MISC_FEATURE

<223>  sequence of (wt) Yrk

<400>  7

Met Gly Cys Val His Cys Lys Glu Lys Ile Ser Gly Lys Gly Gln Gly
1               5                   10                  15

Gly Ser Gly Thr Gly Thr Pro Ala His Pro Pro Ser Gln Tyr Asp Pro
            20                  25                  30

Asp Pro Thr Gln Leu Ser Gly Ala Phe Thr His Ile Pro Asp Phe Asn
        35                  40                  45

Asn Phe His Ala Ala Ala Val Ser Pro Pro Val Pro Phe Ser Gly Pro
    50                  55                  60

Gly Phe Tyr Pro Cys Asn Thr Leu Gln Ala His Ser Ser Ile Thr Gly
65                  70                  75                  80

Gly Gly Val Thr Leu Phe Ile Ala Leu Tyr Asp Tyr Glu Ala Arg Thr
                85                  90                  95

Glu Asp Asp Leu Ser Phe Gln Lys Gly Glu Lys Phe His Ile Ile Asn
            100                 105                 110

Asn Thr Glu Gly Asp Trp Trp Glu Ala Arg Ser Leu Ser Ser Gly Ala
        115                 120                 125

Thr Gly Tyr Ile Pro Ser Asn Tyr Val Ala Pro Val Asp Ser Ile Gln
    130                 135                 140

Ala Glu Glu Trp Tyr Phe Gly Lys Ile Gly Arg Lys Asp Ala Glu Arg
145                 150                 155                 160

Gln Leu Leu Cys His Gly Asn Cys Arg Gly Thr Phe Leu Ile Arg Glu
                165                 170                 175

Ser Glu Thr Thr Lys Gly Ala Tyr Ser Leu Ser Ile Arg Asp Trp Asp
            180                 185                 190

Glu Ala Lys Gly Asp His Val Lys His Tyr Lys Ile Arg Lys Leu Asp
        195                 200                 205

Ser Gly Gly Tyr Tyr Ile Thr Thr Arg Ala Gln Phe Asp Thr Ile Gln
    210                 215                 220

Gln Leu Val Gln His Tyr Ile Glu Arg Ala Ala Gly Leu Cys Cys Arg
225                 230                 235                 240

Leu Ala Val Pro Cys Pro Lys Gly Thr Pro Lys Leu Ala Asp Leu Ser
                245                 250                 255

Val Lys Thr Lys Asp Val Trp Glu Ile Pro Arg Glu Ser Leu Gln Leu
            260                 265                 270

Leu Gln Lys Leu Gly Asn Gly Gln Phe Gly Glu Val Trp Met Gly Thr
        275                 280                 285

Trp Asn Gly Thr Thr Lys Val Ala Val Lys Thr Leu Lys Pro Gly Thr
    290                 295                 300

Met Ser Pro Glu Ala Phe Leu Glu Glu Ala Gln Ile Met Lys Arg Leu
305                 310                 315                 320

Arg His Asp Lys Leu Val Gln Leu Tyr Ala Val Val Ser Glu Glu Pro
                325                 330                 335

Ile Tyr Ile Val Thr Glu Phe Met Ser Gln Gly Ser Leu Leu Asp Phe
            340                 345                 350

Leu Lys Asp Gly Asp Gly Arg Tyr Leu Lys Leu Pro Gln Leu Val Asp
        355                 360                 365

Met Ala Ala Gln Ile Ala Ala Gly Met Ala Tyr Ile Glu Arg Met Asn
    370                 375                 380

Tyr Ile His Arg Asp Leu Arg Ala Ala Asn Ile Leu Val Gly Asp Asn
385                 390                 395                 400

Leu Val Cys Lys Ile Ala Asp Phe Gly Leu Ala Arg Leu Ile Glu Asp
                405                 410                 415

Asn Glu Tyr Thr Ala Arg Gln Gly Ala Lys Phe Pro Ile Lys Trp Thr
            420                 425                 430

Ala Pro Glu Ala Ala Leu Phe Gly Lys Phe Thr Ile Lys Ser Asp Val
        435                 440                 445

Trp Ser Phe Gly Ile Leu Leu Thr Glu Leu Val Thr Lys Gly Arg Val
    450                 455                 460

Pro Tyr Pro Gly Met Asn Asn Arg Glu Val Leu Glu Gln Val Glu Arg
465                 470                 475                 480

Gly Tyr Arg Met Gln Cys Pro Gly Gly Cys Pro Pro Ser Leu His Asp
                485                 490                 495

Val Met Val Gln Cys Trp Lys Arg Glu Pro Glu Glu Arg Pro Thr Phe
            500                 505                 510

Glu Tyr Leu Gln Ser Phe Leu Glu Asp Tyr Phe Thr Ala Thr Glu Pro
        515                 520                 525

Gln Tyr Gln Pro Gly Asp Asn Gln
    530                 535

<210>  8

<211>  529

<212>  PRT

<213>  mammalian

<220>

<221>  MISC_FEATURE

<223>  sequence of (wt) Fgr

<400>  8

Met Gly Cys Val Phe Cys Lys Lys Leu Glu Pro Val Ala Thr Ala Lys
1               5                   10                  15

Glu Asp Ala Gly Leu Glu Gly Asp Phe Arg Ser Tyr Gly Ala Ala Asp
            20                  25                  30

His Tyr Gly Pro Asp Pro Thr Lys Ala Arg Pro Ala Ser Ser Phe Ala
        35                  40                  45

His Ile Pro Asn Tyr Ser Asn Phe Ser Ser Gln Ala Ile Asn Pro Gly
    50                  55                  60

Phe Leu Asp Ser Gly Thr Ile Arg Gly Val Ser Gly Ile Gly Val Thr
65                  70                  75                  80

Leu Phe Ile Ala Leu Tyr Asp Tyr Glu Ala Arg Thr Glu Asp Asp Leu

                85                  90                  95

Thr Phe Thr Lys Gly Glu Lys Phe His Ile Leu Asn Asn Thr Glu Gly
            100                 105                 110

Asp Trp Trp Glu Ala Arg Ser Leu Ser Ser Gly Lys Thr Gly Cys Ile
        115                 120                 125

Pro Ser Asn Tyr Val Ala Pro Val Asp Ser Ile Gln Ala Glu Glu Trp
    130                 135                 140

Tyr Phe Gly Lys Ile Gly Arg Lys Asp Ala Glu Arg Gln Leu Leu Ser
145                 150                 155                 160

Pro Gly Asn Pro Gln Gly Ala Phe Leu Ile Arg Glu Ser Glu Thr Thr
                165                 170                 175

Lys Gly Ala Tyr Ser Leu Ser Ile Arg Asp Trp Asp Gln Thr Arg Gly
            180                 185                 190

Asp His Val Lys His Tyr Lys Ile Arg Lys Leu Asp Met Gly Gly Tyr
        195                 200                 205

Tyr Ile Thr Thr Arg Val Gln Phe Asn Ser Val Gln Glu Leu Val Gln
    210                 215                 220

His Tyr Met Glu Val Asn Asp Gly Leu Cys Asn Leu Leu Ile Ala Pro
225                 230                 235                 240

Cys Thr Ile Met Lys Pro Gln Thr Leu Gly Leu Ala Lys Asp Ala Trp
                245                 250                 255

Glu Ile Ser Arg Ser Ser Ile Thr Leu Glu Arg Arg Leu Gly Thr Gly
            260                 265                 270

Cys Phe Gly Asp Val Trp Leu Gly Thr Trp Asn Gly Ser Thr Lys Val
        275                 280                 285

Ala Val Lys Thr Leu Lys Pro Gly Thr Met Ser Pro Lys Ala Phe Leu
    290                 295                 300

Glu Glu Ala Gln Val Met Lys Leu Leu Arg His Asp Lys Leu Val Gln
305                 310                 315                 320

Leu Tyr Ala Val Val Ser Glu Glu Pro Ile Tyr Ile Val Thr Glu Phe
                325                 330                 335

Met Cys His Gly Ser Leu Leu Asp Phe Leu Lys Asn Pro Glu Gly Gln
            340                 345                 350

Asp Leu Arg Leu Pro Gln Leu Val Asp Met Ala Ala Gln Val Ala Glu
        355                 360                 365

Gly Met Ala Tyr Met Glu Arg Met Asn Tyr Ile His Arg Asp Leu Arg
    370                 375                 380

Ala Ala Asn Ile Leu Val Gly Glu Arg Leu Ala Cys Lys Ile Ala Asp
385                 390                 395                 400

Phe Gly Leu Ala Arg Leu Ile Lys Asp Asp Glu Tyr Asn Pro Cys Gln
                405                 410                 415

Gly Ser Lys Phe Pro Ile Lys Trp Thr Ala Pro Glu Ala Ala Leu Phe
            420                 425                 430

Gly Arg Phe Thr Ile Lys Ser Asp Val Trp Ser Phe Gly Ile Leu Leu
        435                 440                 445

Thr Glu Leu Ile Thr Lys Gly Arg Ile Pro Tyr Pro Gly Met Asn Lys
    450                 455                 460

Arg Glu Val Leu Glu Gln Val Glu Gln Gly Tyr His Met Pro Cys Pro
465                 470                 475                 480

Pro Gly Cys Pro Ala Ser Leu Tyr Glu Ala Met Glu Gln Thr Trp Arg
                485                 490                 495

Leu Asp Pro Glu Glu Arg Pro Thr Phe Glu Tyr Leu Gln Ser Phe Leu
            500                 505                 510

Glu Asp Tyr Phe Thr Ser Ala Glu Pro Gln Tyr Gln Pro Gly Asp Gln
        515                 520                 525

Thr

<210>  9

<211>  525

<212>  PRT

<213>  mammalian

<220>

<221>  MISC_FEATURE

<223>  sequence of (wt) Hck

<400>  9

Gly Gly Arg Ser Ser Cys Glu Asp Pro Gly Cys Pro Arg Asp Glu Glu
1               5                   10                  15

Arg Ala Pro Arg Met Gly Cys Met Lys Ser Lys Phe Leu Gln Val Gly
            20                  25                  30

Gly Asn Thr Phe Ser Lys Thr Glu Thr Ser Ala Ser Pro His Cys Pro
        35                  40                  45

Val Tyr Val Pro Asp Pro Thr Ser Thr Ile Lys Pro Gly Pro Asn Ser
    50                  55                  60

His Asn Ser Asn Thr Pro Gly Ile Arg Glu Ala Gly Ser Glu Asp Ile
65                  70                  75                  80

Ile Val Val Ala Leu Tyr Asp Tyr Glu Ala Ile His His Glu Asp Leu
                85                  90                  95

Ser Phe Gln Lys Gly Asp Gln Met Val Val Leu Glu Glu Ser Gly Glu
            100                 105                 110

Trp Trp Lys Ala Arg Ser Leu Ala Thr Arg Lys Glu Gly Tyr Ile Pro
        115                 120                 125

Ser Asn Tyr Val Ala Arg Val Asp Ser Leu Glu Thr Glu Glu Trp Phe
    130                 135                 140

Phe Lys Gly Ile Ser Arg Lys Asp Ala Glu Arg Gln Leu Leu Ala Pro
145                 150                 155                 160

Gly Asn Met Leu Gly Ser Phe Met Ile Arg Asp Ser Glu Thr Thr Lys
                165                 170                 175

Gly Ser Tyr Ser Leu Ser Val Arg Asp Tyr Asp Pro Arg Gln Gly Asp
            180                 185                 190

Thr Val Lys His Tyr Lys Ile Arg Thr Leu Asp Asn Gly Gly Phe Tyr
        195                 200                 205

Ile Ser Pro Arg Ser Thr Phe Ser Thr Leu Gln Glu Leu Val Asp His

210                 215                 220

Tyr Lys Lys Gly Asn Asp Gly Leu Cys Gln Lys Leu Ser Val Pro Cys
225                 230                 235                 240

Met Ser Ser Lys Pro Gln Lys Pro Trp Glu Lys Asp Ala Trp Glu Ile
                245                 250                 255

Pro Arg Glu Ser Leu Lys Leu Glu Lys Lys Leu Gly Ala Gly Gln Phe
            260                 265                 270

Gly Glu Val Trp Met Ala Thr Tyr Asn Lys His Thr Lys Val Ala Val
        275                 280                 285

Lys Thr Met Lys Pro Gly Ser Met Ser Val Glu Ala Phe Leu Ala Glu
    290                 295                 300

Ala Asn Val Met Lys Thr Leu Gln His Asp Lys Leu Val Lys Leu His
305                 310                 315                 320

Ala Val Val Thr Lys Glu Pro Ile Tyr Ile Ile Thr Glu Phe Met Ala
                325                 330                 335

Lys Gly Ser Leu Leu Asp Phe Leu Lys Ser Asp Glu Gly Ser Lys Gln
            340                 345                 350

Pro Leu Pro Lys Leu Ile Asp Phe Ser Ala Gln Ile Ala Glu Gly Met
        355                 360                 365

Ala Phe Ile Glu Gln Arg Asn Tyr Ile His Arg Asp Leu Arg Ala Ala
    370                 375                 380

Asn Ile Leu Val Ser Ala Ser Leu Val Cys Lys Ile Ala Asp Phe Gly
385                 390                 395                 400

Leu Ala Arg Val Ile Glu Asp Asn Glu Tyr Thr Ala Arg Glu Gly Ala
                405                 410                 415

Lys Phe Pro Ile Lys Trp Thr Ala Pro Glu Ala Ile Asn Phe Gly Ser
            420                 425                 430

Phe Thr Ile Lys Ser Asp Val Trp Ser Phe Gly Ile Leu Leu Met Glu
        435                 440                 445

Ile Val Thr Tyr Gly Arg Ile Pro Tyr Pro Gly Met Ser Asn Pro Glu
    450                 455                 460

Val Ile Arg Ala Leu Glu Arg Gly Tyr Arg Met Pro Arg Pro Glu Asn
465                 470                 475                 480

Cys Pro Glu Glu Leu Tyr Asn Ile Met Met Arg Cys Trp Lys Asn Arg
                485                 490                 495

Pro Glu Glu Arg Pro Thr Phe Glu Tyr Ile Gln Ser Val Leu Asp Asp
            500                 505                 510

Phe Tyr Thr Ala Thr Glu Ser Gln Tyr Gln Gln Gln Pro
        515                 520                 525

<210>  10

<211>  511

<212>  PRT

<213>  mammalian

<220>

<221>  MISC_FEATURE

<223>  sequence of (wt) Lyn

<400>  10

Met Gly Cys Ile Lys Ser Lys Gly Lys Asp Ser Leu Ser Asp Asp Gly
1               5                   10                  15

Val Asp Leu Lys Thr Gln Pro Val Arg Asn Thr Glu Arg Thr Ile Tyr
            20                  25                  30

Val Arg Asp Pro Thr Ser Asn Lys Gln Gln Arg Pro Val Pro Glu Ser
        35                  40                  45

Gln Leu Leu Pro Gly Gln Arg Phe Gln Thr Lys Asp Pro Glu Glu Gln
    50                  55                  60

Gly Asp Ile Val Val Ala Leu Tyr Pro Tyr Asp Gly Ile His Pro Asp
65                  70                  75                  80

Asp Leu Ser Phe Lys Lys Gly Glu Lys Met Lys Val Leu Glu Glu His
                85                  90                  95

Gly Glu Trp Trp Lys Ala Lys Ser Leu Leu Thr Lys Lys Glu Gly Phe
            100                 105                 110

Ile Pro Ser Asn Tyr Val Ala Lys Leu Asn Thr Leu Glu Thr Glu Glu
        115                 120                 125

Trp Phe Phe Lys Asp Ile Thr Arg Lys Asp Ala Glu Arg Gln Leu Leu
    130                 135                 140

Ala Pro Gly Asn Ser Ala Gly Ala Phe Leu Ile Arg Glu Ser Glu Thr
145                 150                 155                 160

Leu Lys Gly Ser Phe Ser Leu Ser Val Arg Asp Phe Asp Pro Val His
                165                 170                 175

Gly Asp Val Ile Lys His Tyr Lys Ile Arg Ser Leu Asp Asn Gly Gly
            180                 185                 190

Tyr Tyr Ile Ser Pro Arg Ile Thr Phe Pro Cys Ile Ser Asp Met Ile
        195                 200                 205

Lys His Tyr Gln Lys Gln Ala Asp Gly Leu Cys Arg Arg Leu Glu Lys
    210                 215                 220

Ala Cys Ile Ser Pro Lys Pro Gln Lys Pro Trp Asp Lys Asp Ala Trp
225                 230                 235                 240

Glu Ile Pro Arg Glu Ser Ile Lys Leu Val Lys Arg Leu Gly Ala Gly
                245                 250                 255

Gln Phe Gly Glu Val Trp Met Gly Tyr Tyr Asn Asn Ser Thr Lys Val
            260                 265                 270

Ala Val Lys Thr Leu Lys Pro Gly Thr Met Ser Val Gln Ala Phe Leu
        275                 280                 285

Glu Glu Ala Asn Leu Met Lys Thr Leu Gln His Asp Lys Leu Val Arg
    290                 295                 300

Leu Tyr Ala Val Val Thr Arg Glu Glu Pro Ile Tyr Ile Ile Thr Glu
305                 310                 315                 320

Tyr Met Ala Lys Gly Leu Leu Asp Phe Leu Lys Ser Asp Glu Gly Gly
                325                 330                 335

Lys Val Leu Leu Pro Lys Leu Ile Asp Phe Ser Ala Gln Ile Ala Glu
            340                 345                 350

Gly Met Ala Tyr Ile Glu Arg Lys Asn Tyr Ile His Arg Asp Leu Arg

        355                 360                 365

Ala Ala Asn Val Leu Val Ser Glu Ser Leu Met Cys Lys Ile Ala Asp
    370                 375                 380

Phe Gly Leu Ala Arg Val Ile Glu Asp Asn Glu Tyr Thr Ala Arg Glu
385                 390                 395                 400

Gly Ala Lys Phe Pro Ile Lys Trp Thr Ala Pro Glu Ala Ile Asn Phe
                405                 410                 415

Gly Cys Phe Thr Ile Lys Ser Asp Val Trp Ser Phe Gly Ile Leu Leu
            420                 425                 430

Tyr Glu Ile Val Thr Tyr Gly Lys Ile Pro Tyr Pro Gly Arg Thr Asn
        435                 440                 445

Ala Asp Val Met Thr Ala Leu Ser Gln Gly Tyr Arg Met Pro Arg Val
    450                 455                 460

Glu Asn Cys Pro Asp Glu Leu Tyr Asp Ile Met Lys Met Cys Trp Lys
465                 470                 475                 480

Glu Lys Ala Glu Glu Arg Pro Thr Phe Asp Tyr Leu Gln Ser Val Leu
                485                 490                 495

Asp Asp Phe Tyr Thr Ala Thr Glu Gly Gln Tyr Gln Gln Gln Pro
            500                 505                 510

<210> 11

<211> 509

<212> PRT

<213> mammalian

<220>

<221> MISC_FEATURE

<223> sequence of (wt) Lck

<400> 11

Met Gly Cys Gly Cys Ser Ser His Pro Glu Asp Asp Trp Met Glu Asn
1               5                   10                  15

Ile Asp Val Cys Glu Asn Cys His Tyr Pro Ile Val Pro Leu Asp Gly
            20                  25                  30

Lys Gly Thr Leu Leu Ile Arg Asn Gly Ser Glu Val Arg Asp Pro Leu
        35                  40                  45

Val Thr Tyr Glu Gly Ser Asn Pro Pro Ala Ser Pro Leu Gln Asp Asn
    50                  55                  60

Leu Val Ile Ala Leu His Ser Tyr Glu Pro Ser His Asp Gly Asp Leu
65                  70                  75                  80

Gly Phe Glu Lys Gly Glu Pro Leu Arg Ile Leu Glu Gln Ser Gly Glu
                85                  90                  95

Trp Trp Lys Ala Gln Ser Leu Thr Thr Gly Gln Glu Gly Phe Ile Pro
            100                 105                 110

Phe Asn Phe Val Ala Lys Ala Asn Ser Leu Glu Pro Glu Pro Trp Phe
        115                 120                 125

Phe Lys Asn Leu Ser Arg Lys Asp Ala Glu Arg Gln Leu Leu Ala Pro
    130                 135                 140

Gly Asn Thr His Gly Ser Phe Leu Ile Arg Glu Ser Glu Ser Thr Ala
145                 150                 155                 160

Gly Ser Phe Ser Leu Ser Val Arg Asp Phe Asp Gln Asn Gln Gly Glu
                165                 170                 175

Val Val Lys His Tyr Lys Ile Arg Asn Leu Asp Asn Gly Gly Phe Tyr
            180                 185                 190

Ile Ser Pro Arg Ile Thr Phe Pro Gly Leu His Glu Leu Val Arg His
        195                 200                 205

Tyr Thr Asn Ala Ser Asp Gly Leu Cys Thr Arg Leu Ser Arg Pro Cys
    210                 215                 220

Gln Thr Gln Lys Pro Gln Lys Pro Trp Trp Glu Asp Glu Trp Glu Val
225                 230                 235                 240

Pro Arg Glu Thr Leu Lys Leu Val Glu Arg Leu Gly Ala Gly Gln Phe
                245                 250                 255

Gly Glu Val Trp Met Gly Tyr Tyr Asn Gly His Thr Lys Val Ala Val
            260                 265                 270

Lys Ser Leu Lys Gln Gly Ser Met Ser Pro Asp Ala Phe Leu Ala Glu
        275                 280                 285

Ala Asn Leu Met Lys Gln Leu Gln His Gln Arg Leu Val Arg Leu Tyr
    290                 295                 300

Ala Val Val Thr Gln Glu Pro Ile Tyr Ile Ile Thr Glu Tyr Met Glu
305                 310                 315                 320

Asn Gly Ser Leu Val Asp Phe Leu Lys Thr Pro Ser Gly Ile Lys Leu
                325                 330                 335

Thr Ile Asn Lys Leu Leu Asp Met Ala Ala Gln Ile Ala Glu Gly Met
            340                 345                 350

Ala Phe Ile Glu Glu Arg Asn Tyr Ile His Arg Asp Leu Arg Ala Ala
        355                 360                 365

Asn Ile Leu Val Ser Asp Thr Leu Ser Cys Lys Ile Ala Asp Phe Gly
    370                 375                 380

Leu Ala Arg Leu Ile Glu Asp Asn Glu Tyr Thr Ala Arg Glu Gly Ala
385                 390                 395                 400

Lys Phe Pro Ile Lys Trp Thr Ala Pro Glu Ala Ile Asn Tyr Gly Thr
                405                 410                 415

Phe Thr Ile Lys Ser Asp Val Trp Ser Phe Gly Ile Leu Leu Thr Glu
            420                 425                 430

Ile Val Thr His Gly Arg Ile Pro Tyr Pro Gly Met Thr Asn Pro Glu
        435                 440                 445

Val Ile Gln Asn Leu Glu Arg Gly Tyr Arg Met Val Arg Pro Asp Asn
    450                 455                 460

Cys Pro Glu Glu Leu Tyr Gln Leu Met Arg Leu Cys Trp Lys Glu Arg
465                 470                 475                 480

Pro Glu Asp Arg Pro Thr Phe Asp Tyr Leu Arg Ser Val Leu Glu Asp
                485                 490                 495

Phe Phe Thr Ala Thr Glu Gly Gln Tyr Gln Pro Gln Pro
            500                 505

<210> 12

<211>  505

<212>  PRT

<213>  mammalian

<220>

<221>  MISC_FEATURE

<223>  sequence of (wt) Blk

<400>  12

Met Gly Leu Val Ser Ser Lys Lys Pro Asp Lys Glu Lys Pro Ile Lys
1               5                   10                  15

Glu Lys Asp Lys Gly Gln Trp Ser Pro Leu Lys Val Ser Ala Gln Asp
            20                  25                  30

Lys Asp Ala Pro Pro Leu Pro Pro Leu Val Val Phe Asn His Leu Thr
        35                  40                  45

Pro Pro Pro Pro Asp Glu His Leu Asp Glu Asp Lys His Phe Val Val
    50                  55                  60

Ala Leu Tyr Asp Tyr Thr Ala Met Asn Asp Arg Asp Leu Gln Met Leu
65                  70                  75                  80

Lys Gly Glu Lys Leu Gln Val Leu Lys Gly Thr Gly Asp Trp Trp Leu
                85                  90                  95

Ala Arg Ser Leu Val Thr Gly Arg Glu Gly Tyr Val Pro Ser Asn Phe
            100                 105                 110

Val Ala Arg Val Glu Ser Leu Glu Met Glu Arg Trp Phe Phe Arg Ser
        115                 120                 125

Gln Gly Arg Lys Glu Ala Glu Arg Gln Leu Leu Ala Pro Ile Asn Lys
    130                 135                 140

Ala Gly Ser Phe Leu Ile Arg Glu Ser Glu Thr Asn Lys Gly Ala Phe
145                 150                 155                 160

Ser Leu Ser Val Lys Asp Val Thr Thr Gln Gly Glu Leu Ile Lys His
                165                 170                 175

Tyr Lys Ile Arg Cys Leu Asp Glu Gly Gly Tyr Tyr Ile Ser Pro Arg

180                 185                 190

Ile Thr Phe Pro Ser Leu Gln Ala Leu Val Gln His Tyr Ser Lys Lys
        195                 200                 205

Gly Asp Gly Leu Cys Gln Arg Leu Thr Leu Pro Cys Val Arg Pro Ala
    210                 215                 220

Pro Gln Asn Pro Trp Ala Gln Asp Glu Trp Glu Ile Pro Arg Gln Ser
225                 230                 235                 240

Leu Arg Leu Val Arg Lys Leu Gly Ser Gly Gln Phe Gly Glu Val Trp
                245                 250                 255

Met Gly Tyr Tyr Lys Asn Asn Met Lys Val Ala Ile Lys Thr Leu Lys
            260                 265                 270

Glu Gly Thr Met Ser Pro Glu Ala Phe Leu Gly Glu Ala Asn Met Met
        275                 280                 285

Lys Ala Leu Gln His Glu Arg Leu Val Arg Leu Tyr Ala Val Val Thr
    290                 295                 300

Lys Glu Pro Ile Tyr Ile Val Thr Glu Tyr Met Ala Arg Gly Cys Leu
305                 310                 315                 320

Leu Asp Phe Leu Lys Thr Asp Glu Gly Ser Arg Leu Ser Leu Pro Arg
                325                 330                 335

Leu Ile Asp Met Ser Ala Gln Ile Ala Glu Gly Met Ala Tyr Ile Glu
            340                 345                 350

Arg Met Asn Ser Ile His Arg Asp Leu Arg Ala Ala Asn Ile Leu Val
        355                 360                 365

Ser Glu Ala Leu Cys Cys Lys Ile Ala Asp Phe Gly Leu Ala Arg Ile
    370                 375                 380

Ile Asp Ser Glu Tyr Thr Ala Gln Glu Gly Ala Lys Phe Pro Ile Lys
385                 390                 395                 400

Trp Thr Ala Pro Glu Ala Ile His Phe Gly Val Phe Thr Ile Lys Ala
                405                 410                 415

Asp Val Trp Ser Phe Gly Val Leu Leu Met Glu Val Val Thr Tyr Gly
            420                 425                 430

Arg Val Pro Tyr Pro Gly Met Ser Asn Pro Glu Val Ile Arg Asn Leu
        435                 440                 445

Glu Arg Gly Tyr Arg Met Pro Arg Pro Asp Thr Cys Pro Pro Glu Leu
    450                 455                 460

Tyr Arg Gly Val Ile Ala Glu Cys Trp Arg Ser Arg Pro Glu Glu Arg
465                 470                 475                 480

Pro Thr Phe Glu Phe Leu Gln Ser Val Leu Glu Asp Phe Tyr Thr Ala
                485                 490                 495

Thr Glu Arg Gln Tyr Glu Leu Gln Pro
            500                 505

<210> 13

<211> 308

<212> PRT

<213> Homo sapiens

<220>

<221> MISC_FEATURE

<223> sequence of 6His-Src catalytic domain (human)

<400> 13

Met Ser Tyr Tyr His His His His His His Asp Tyr Asp Ile Pro Thr
1               5                   10                  15

Thr Glu Asn Leu Tyr Phe Gln Gly Ala Gln Thr Gln Gly Leu Ala Lys
            20                  25                  30

Asp Ala Trp Glu Ile Pro Arg Glu Ser Leu Arg Leu Glu Val Lys Leu
        35                  40                  45

Gly Gln Gly Cys Phe Gly Glu Val Trp Met Gly Thr Trp Asn Gly Thr
    50                  55                  60

Thr Arg Val Ala Ile Lys Thr Leu Lys Pro Gly Thr Met Ser Pro Glu
65                  70                  75                  80

Ala Phe Leu Gln Glu Ala Gln Val Met Lys Lys Leu Arg His Glu Lys
                85                  90                  95

Leu Val Gln Leu Tyr Ala Val Val Ser Glu Glu Pro Ile Phe Ile Val
            100                 105                 110

Thr Glu Tyr Met Ser Lys Gly Ser Leu Leu Asp Phe Leu Lys Gly Glu
        115                 120                 125

Thr Gly Lys Ser Leu Arg Leu Pro Gln Leu Val Asp Met Ala Ala Gln
    130                 135                 140

Ile Ala Ser Gly Met Ala Tyr Val Glu Arg Met Asn Tyr Val His Arg
145                 150                 155                 160

Asp Leu Arg Ala Ala Asn Ile Leu Val Gly Glu Asn Leu Val Cys Lys
                165                 170                 175

Val Ala Asp Phe Gly Leu Ala Arg Leu Ile Glu Asp Asn Glu Tyr Thr
            180                 185                 190

Ala Arg Gln Gly Ala Lys Phe Pro Ile Lys Trp Thr Ala Pro Glu Ala
        195                 200                 205

Ala Leu Tyr Gly Arg Phe Thr Ile Lys Ser Asp Val Trp Ser Phe Gly
    210                 215                 220

Ile Leu Leu Thr Glu Leu Thr Thr Lys Gly Arg Val Pro Tyr Pro Gly
225                 230                 235                 240

Met Val Asn Arg Glu Val Leu Asp Gln Val Glu Arg Gly Tyr Arg Met
                245                 250                 255

Pro Cys Pro Pro Glu Cys Pro Glu Ser Leu His Asp Leu Met Cys Gln
            260                 265                 270

Cys Trp Arg Lys Glu Pro Glu Glu Arg Pro Thr Phe Glu Tyr Leu Gln
        275                 280                 285

Ala Phe Leu Glu Asp Tyr Phe Thr Ser Thr Glu Pro Gln Tyr Gln Pro
    290                 295                 300

Gly Glu Asn Leu
305

<210> 14

<211> 31

<212> DNA

<213>  Artificial

<220>

<221>  misc_feature

<223>  Description of sequence: primer L-1611hSrc-Hind3+

<400>  14
atataagctt ctagaggttc tccccgggct g                                    31

<210>  15

<211>  32

<212>  DNA

<213>  Artificial

<220>

<221>  misc_feature

<223>  Description of sequence: primer U-src-259blunt

<400>  15
accacctttg tggccctcta tgactatgag tc                                   32

<210>  16

<211>  63

<212>  DNA

<213>  Artificial

<220>

<221>  misc_feature

<223>  Description of sequence: primer U-hSrc-Var4

<400>  16
atatccatgg gtctggccgg tggagtgacc acctttgtgg ccctctatga ctatgagtct     60

agg                                                                   63

<210>  17

<211>  62

<212>  DNA

<213>  Artificial

<220>

<221>  misc_feature

<223>  description of sequence: primer L-hSrc-Var4

<400>  17
tatactcgag ttaagggatc tcttcgtact ggggctcggt ggacgtgaag tagtcctcca      60

gg                                                                      62

## Description

The present invention relates to methods for identifying, selecting and/or characterizing compounds capable of modulating auto-phosphorylation activity of protein-tyrosine kinases (PTKs), to compounds identified thereby and to their use in the treatment of diseases related to a protein tyrosine kinase activity, such as cancer or osteoporesis.

Protein tyrosine kinases (PTKs) are widely spread and regulate a plurality of cellular processes including proliferation and transformation, survival, differentiation and metabolism. PTKs play a key role in normal cell and tissue development. Also, PTKs are assumed to be involved in the generation and/or progression of many different types of diseases. Enhanced PTK activity, for instance, is intimately correlated with proliferative diseases, such as cancers, leukemias, psoriasis, and restenosis, as well as with immune system dysfunction and bone remodelling diseases. For general reviews see Thomas and Brugge, Annu. Rev. Cell Dev. Biol. (1997) 13, 513; Lawrence and Niu, Pharmacol. Ther. (1998) 77, 81; Tatosyan and Mizenina, Biochemistry (Moscow) (2000) 65, 49; Boschelli et al., Drugs of the Future 2000, 25(7), 717, (2000). Accordingly, an increased need exists for suitable therapies for the treatment of diseases related to dysfunctional PTK activity. Such therapies may be based on compounds modifying or even inhibiting protein tyrosine kinases, in particular PTK trans-phosphorylation activity.

PTKs represent a subclass of protein kinases, wherein PTKs catalyze the transfer of the γ-phosphoryl group from a donor molecule (usually ATP) to tyrosine hydroxyls of an acceptor protein. Phosphorylation via protein kinases is important for signal transduction and the regulation of enzymes, whereby a phosphorylation reaction can activate (or inhibit) the activity of an enzyme. Particularly for eukaryotes, protein phosphorylation is probably the most important regulatory event, since many enzymes and receptors are switched "on" or "off" by phosphorylation and dephosphorylation.

PTKs undergo auto-phosphorylation or trans-phosphorylation reactions, i.e. PTKs can use their own sequence as a substrate and thus lack the need of providing additional substrate such as proteins or peptides. Auto-phosphorylation of a PTK is a monomolecular autocatalytic reaction. ln an auto-phosphorylation reaction a non-phosphorylated PTK phosphorylates itself by an intramolecular mechanism. This reaction is typically not dependent upon protein concentrations or ATP concentrations, if ATP is available in excess during auto-phosphorylation. Then, this reaction may be regarded as a 0^{th} order reaction, which can be described by a single-exponential kinetic equation. In contrast, trans-phosphorylation involves a bimolecular auto-catalytic 1^{st} order reaction, i.e. one protein tyrosine kinase phosphorylates a second protein tyrosine kinase. Irrespective of other regulatory ligands, the rate of trans-phosphorylation is typically dependent on the concentration of protein kinase, which may alternatively regulate specific biological processes.

Before the trans-phosphorylation reaction can take place, non-phosphorylated PTKs have to undergo auto-phosphorylation at a particular site of their sequence, e.g. at a tyrosine residue of the kinase domain, which is non-phosphorylated prior to this step. Non-phosphorylated PTKs may be present in an open or a closed conformation, whereby the open conformation allows the kinase to be phosphorylated at a tyrosine a tyrosine residue of the kinase domain. Typically, the functional transition from a closed to an open conformation is based on a conformation alteration.

PTKs represent a large multigene family. A search of the human genome for PTK encoding elements identified ninety unique kinase genes in the human genome, along with five pseudogenes. Of the ninety tyrosine kinases encoded thereby, fifty-eight are assumed to be of a tyrosine kinase receptor type, which, in turn, are distributed over twenty subfamilies. The remaining thirty-two non-receptor tyrosine kinases have been distributed over ten subfamilies. Additionally, mouse orthologs were identified for nearly all human tyrosine kinases. (Robinson et al., Oncogene (2000) 19, 5548-5557).

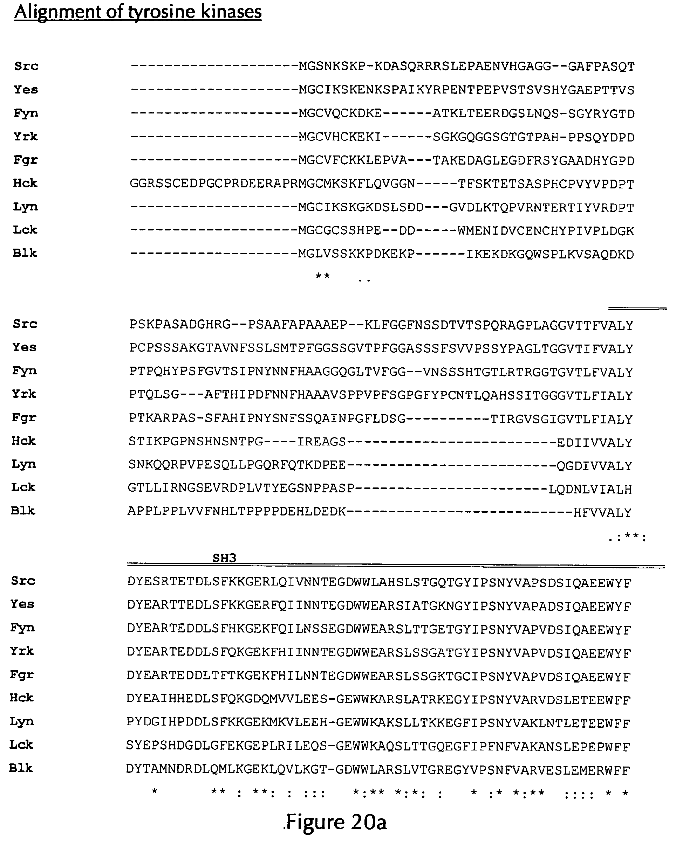

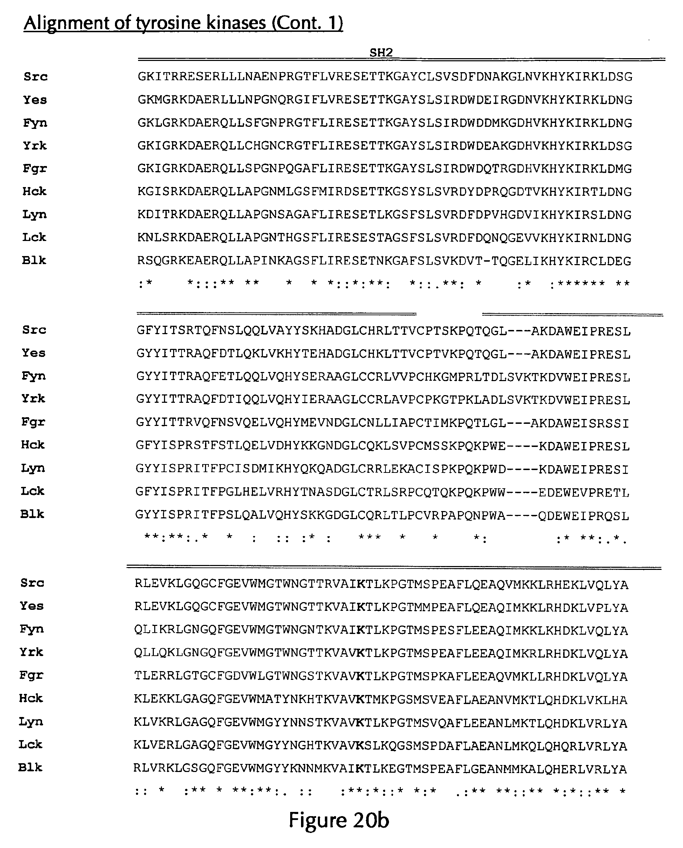

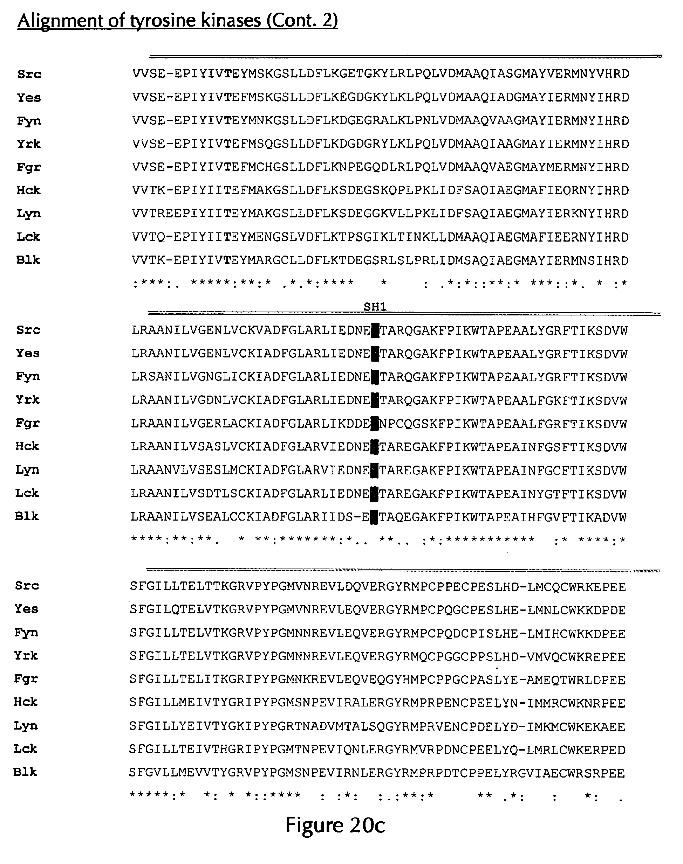

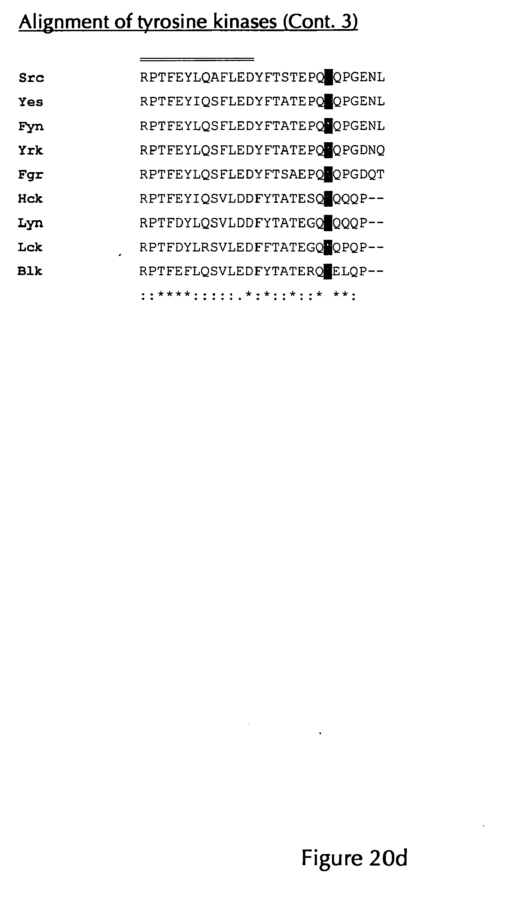

The Src family is an example for non-receptor PTKs. Members of the Src family include at least nine closely related PTKs in mammals, being Src, Fyn, Yes, Fgr, Lyn, Hck, Lck, Blk and Yrk, having a molecular weight from about 52 to 62 kD (Kefalas et al. (1995), *Int. J. Biochem. Cell Biol.* 27, 551-63.). All these Src kinases are characterized by a common structural organization that has six distinct functional domains: Src homology domain 4 (SH4), a unique domain, SH3 domain, SH2 domain, a catalytic domain (SH1), and a C-terminal regulatory region (Tatosyan et al. Biochemistry (Moscow) 65, 49-58 (2000)). An alignment of the sequences of the Src family members as mentioned above is given in Figures 20a-d. All sequences shown in these figures are human except Yrk (being derived from chicken).

Src (a member of the Src kinase family), in particular pp60^{c-src} kinase, was the first PTK to be identified and represents a prototype for the above mentioned Src-subfamily of at least nine closely related non-receptor PTKs (Kefalas et al. (1995); *Int. J. Biochem. Cell Biol.* 27, 551-63.). Its identification was due to the discovery of an oncogenic variant, "v-Src", in the genome of Rous sarcoma virus which was considered to be responsible for the rapid eruption of tumors in animals infected with this avian retrovirus (Jove and Hanafusa (1987), Annu. Rev. Cell Biol. 3, 31-56.). Its cellular homolog, c-Src or Src, is involved in cell division (probably through interaction with different growth factor receptors), as well as cell shape changes, adhesion and motility through regulation of integrin signaling and cytoskeletal architecture (Roche et al. (1995), Science 269, 1567-9; Mao et al., 1997, Oncogene 15, 3083-90; Parsons and Parsons (1997), Curr. Opin. Cell Biol. 9, 187-92; Abu-Ghazaleh et al. (2001), Biochem. J. 360, 255-64; Belsches-Jablonski et al. (2001), Oncogene 20, 1465-75; Avizienyte et al. (2002), Nat. Cell Biol. 4, 632-638; Frame (2002), BBA 1602, 114-30; Kitagawa et al. (2002), J. Biol. Chem. 277, 366-71).

As already mentioned above, the capability of PTKs, e.g. Src, to undergo an auto-phosphorylation reaction is typically dependent on their structural conformation. The structure of Src is composed of a string of functional parts connected together in a single protein chain and comprising from one end of the chain to the other an anchoring segment, an SH3 domain, an SH2 domain, a flexible linker, a kinase domain, carrying a phosphorylation site, and a C-terminal final tail, carrying a further phosphorylation site. Src undergoes a large articulated motion when it turns from its closed into its open conformation and vice versa, thereby opening up or closing its structure. In its closed conformation, the Src protein is inactive and displays a compact protein, wherein the SH3 domain, the SH2 domain, the flexible linker, the kinase domain and the final tail are assembled spherically in close proximity. Thereby, the closed conformation of Src kinase tightly folds upon itself, using the short linker to block the SH3 domain so that it cannot bind to other proteins. The key to transition into the open conformation is a tyrosine residue in the tail. Upon phosphorylation of this tyrosine residue, it binds to the SH2 domain, gluing the whole complex shut. When this regulating phosphate is removed, the tail is released and the whole molecule opens up, unblocking the SH3 binding site and allowing access to the kinase active site. In the open conformation the SH3 domain has a little groove that grabs protein chains, positioning them close enough to the kinase domain to allow the addition of a phosphate group, e.g. by an auto-phosphorylation reaction. The phosphate groups required for auto-phosphorylation are provided by ATP. Alternatively, Src may also switch from its closed into its open conformation upon adding ATP and is thereby capable to undergo an auto-phosphorylation reaction. In an auto-phosphorylation reaction, a non-phosphorylated PTK typically phosphorylates a tyrosine in its kinase domain, which enables this PTK to initiate a subsequent trans-phosphorylation reaction.

Because of its functions, a number of studies have implicated Src in the generation and/or progression of several types of cancer, including breast, hepatic, pancreatic and ovarian cancer and in particular several kinds of leukemia and lymphomas as well as colon cancer (Cartwright et al. (1990), Proc. Natl. Acad. Sci. USA 87, 558-62; Lynch et al. (1993), Leukemia 7, 1416-22; Pories et al. (1998), Gastroenterology 114, 1287-95; Frame (2002), BBA 1602, 114-30; Talamonti et al., J. Clin. Invest., 91, 53 (1993); Lutz et al., Biochem. Biophys. Res. 243, 503 (1998); Rosen et al., J. Biol. Chem., 261, 13754 (1986); Bolen et al., Proc. Natl. Acad. Sci. USA, 84, 2251 (1987); Masaki et al., Hepatology, 27, 1257 (1998); Biscardi et al., Adv. Cancer Res., 76, 61 (1999); Lynch et al., Leukemia, 7, 1416 (1993)) and other disorders. While cellular mechanisms, that typically govern cancer, are generally complex, a principal role has been assigned to deregulated (overexpressed and/or mutated) PTKs and a clear association between Src tyrosine kinase activity and advancing tumor stage in colon cancer has been demonstrated (Talamonti et al. (1993), *J*. *Clin Invest.* 91, 53-60). Consequently, a strong positive correlation between Src activity and tumor progression from non-malignant polyps towards invasive cancers and metastatic foci must be assumed, and activation of Src kinase in primary colorectal carcinoma entails a poor clinical prognosis (Aligayer et al. (2002), *Cancer* 94, 344-51). A reduced activation of Src in human colon and ovarian cancer cells has been shown to reduce their tumorigenicity, suggesting that Src inhibitors could have a potential as anticancer drugs (Staley et al. (1997), *Cell Growth Differ.* 8, 269-74; Wiener et al. (1999), *Clin. Cancer Res.* 5, 2164-70). Colon cancer is a common disease which leads to death in ~50% of the cases as a consequence of metastasis. Mechanism-based cures are highly desired for a more successful treatment of colon cancer and many other cancers as well.

Furthermore, studies with Src-deficient mice demonstrated that Src activity is required for bone resorption by osteoclasts, implying Src inhibitors might be useful in the treatment of osteoporosis (Soriano et al. (1991), *Cell* 64, 693-702; Boyce et al., (1992), *J*. *Clin. lnvest.* 90, 1622-7; Lowell et al. (1996), *Genes Dev.* 10, 1845-57).

As mentioned before, PTKs, in particular Src family members, are involved in generation and/or progression of many different types of diseases and pathologic disorders. In order to develop suitable therapies for such PTK-related diseases strong efforts have been made, e.g. by identifying inhibitors of PTK-activity by means of conventional protein-tyrosine kinase assays. A typical protein-tyrosine kinase assay is based on monitoring kinase activity by determining the amount of substrate converted by the kinase. Accordingly, a PTK-substrate (e.g. peptide or a protein) is incubated along with the kinase under specific conditions, whereby phosphorylation of the substrate (due to kinase activity) is observed in the presence and absence of candidate modulators of the kinase (activity). Substrate conversion into its phosphorylated form by the kinase is measured using an appropriate detection system. Such kinase assays are typically carried out either in a homogeneous batch or on a (solid phase) surface. However, since the kinase as well as its substrate have to be supplied, it is of utmost importance to provide highly purified substrate, which may result in increased costs. Additionally, conventional assays as described above, are in general not suitable to identify test compounds, which exclusively bind to the PTK in its closed conformation(s) prior to phosphorylation of its kinase domain tyrosine residue. As a consequence, these assays are limited to the identification of compounds which are exclusively capable of binding to phosphorylated PTKs. Compounds, which bind to non-phosphorylated PTKs and also represent an interesting class of potential modulators of protein tyrosine kinases, cannot be identified by these assays. Consequently, false negative results may be obtained upon monitoring test compounds by such conventional kinase assays, since less suitable compounds may be identified than possible.

Another approach, representing a modified kinase assay, is disclosed in EP 03 028 713. EP 03 028 713 discloses a method of identifying, selecting and/or characterizing a compound modulating the activity of at least one Src family kinase member. According to EP 03 028 713, a living cell is cultivated in a first step containing at least one nucleic acid coding for a Src family kinase or a mutated Src family kinase. In a second step, the nucleic acid is expressed in the transfected cell and, in a further step, the cell is contacted with at least one test compound. A phenotype change of the cell may be determined (in the case of modulation of the activity of at least one Src family kinase by the test compound as compared to the phenotype of the cell in the preceding step. A change of phenotype indicates that the tested compound modulates the activity of at least one Src family kinase member. However, such a phenotype change as obtained by a method of EP 03 028 713 is triggered by PTK inhibitors, which merely bind to phosphorylated PTKs. Compounds binding to non-phosphorylated PTKs generally remain unidentified.

Summarizing the above, the methods known in the art merely allow to screen for compounds having binding affinity to phosphorylated PTK family members. However, at present no method is available in the art which allows to detect/identify compounds, which modulate the activity of members of the PTK family, and which bind to a non-phosphorylated PTK either typically in its open or atypically in its closed conformation. Accordingly, there is still a strong need for a method fulfilling this criterium.

It is the object of the present invention to provide more elaborate methods for identifying and characterizing compounds modulating the function of PTK family members, in particular of Src family members, and binding to a non-phosphorylated PTK either in its open or closed conformation or alternatively to a non-phosphorylated PTK in its closed conformation.

The underlying object is solved by a method for identifying, selecting and/or characterizing a compound which modulates the activity of at least one protein-tyrosine kinase (PTK) as disclosed herein. The method according to the invention comprises the steps of (a) providing a non-phosphorylated protein-tyrosine kinase; (b) providing a test compound; (c) contacting the non-phosphorylated protein-tyrosine kinase with the test compound; (d) activating auto-phosphorylation of the non-phosphorylated protein-tyrosine kinase; and (e) measuring the degree of phosphorylation of the protein-tyrosine kinase via biochemical methods.

In step (a) of the method according to the present invention a non-phosphorylated protein-tyrosine kinase (PTK) is provided. Non-phosphorylated protein-tyrosine kinases (PTKs), as used in the inventive methods, typically comprise any non-phosphorylated PTK, in particular any mammalian receptor and non-receptor PTK. Preferably, the PTK family kinases are vertebrate, in particular mammalian kinases (e.g. horse, pig, chicken, rat, mouse). The use of human PTK family kinase sequences is most preferred. In a preferred embodiment of the inventive methods the non-phosphorylated PTKs used belong to the Src-family, or are selected from the PTKs FAK, PYK2, Syk, ZAP70, ITK, TEC, TXK, BTK, Etk/BMX, CTK, CSK, Arg, Abl, Fes, Fer. A Src family kinase (non-receptor PTK of the SRC family) comprises any member of the Src family, including but not limited to Src, Yes, Fgr, Fyn, Lck, Hck, Lyn, Blk and Yrk.

In a particular preferred embodiment (wt) Src protein-tyrosine kinases as used in the present inventive method are selected from sequences SEQ ID NO: 4 (wt Src kinase), SEQ lD NO: 5 (wt Yes kinase), SEQ lD NO: 6 (wt Fyn kinase), SEQ ID NO: 7 (wt Yrk kinase), SEQ lD NO: 8 (wt Fgr kinase), SEQ lD NO: 9 (wt Hck kinase), SEQ lD NO: 10 (wt Lyn kinase), SEQ ID NO: 11 (wt Lck kinase), and SEQ lD NO: 12 (wt Blk kinase).

PTKs as used for step (a) of a method according to the present invention are provided in their non-phosphorylated form, which can undergo an auto-phosphorylation reaction, if ATP is added. A non-phosphorylated PTK as used in step (a) of the inventive method, may be present in an open or a closed conformation. ln order to maintain the PTK in this non-phosphorylated form, it is of utmost importance that no ATP is available in step (a) and any subsequent step of the inventive method, until the auto-phosphorylation reaction shall be initiated. The use of non-phosphorylated PTKs in method step (a) enables a skilled person to detect precisely the degree of phosphorylation, e.g. upon addition of ATP, in a final qualitative or quantitative determination step of the inventive method.

Non-phosphorylated PTKs used in a method according to the present invention are preferably selected from naturally occurring wildtype (wt) proteins of the aforementioned PTKs. In order to provide these (wt) PTK proteins for step (a), suitable expression systems are used (see Sambrook et al. (2001), Molecular Cloning: A Laboratory Manual, Cold Spring Harbor Laboratory Press, NY Cold Spring Harbor, NY). Generally, the expression of (wt) protein is based on the coding sequence of naturally occurring nucleic acid sequences. Alternatively, non-phosphorylated PTKs as used in a method according to the present invention, may be encoded by variants of these naturally occurring nucleic acid sequences, encoding the (wt) PTK. Such variants e.g. lead to an improved expression of the encoded fusion protein in a particular host organism. Typically, such variants of naturally occurring nucleic acid sequences can be altered such as to advantageously adapt the codon usage to the respective organism. The encoded (wt) amino acid sequence of these variants is typically identical to those of the corresponding (wt) PTKs, i.e. the base pairs of a triplet of the nucleic acid sequence, which encodes a single amino acid, may be altered in accordance with the degenerate character of the genetic code (without altering the amino acid sequence). Tables for appropriately adjusting protein expression upon altering the encoding nucleic acid sequence due to the codon usage in different organisms are known to a skilled person. Preparation and purification of such nucleic acids and/or variants therefrom is usually performed via standard procedures (see Sambrook et al. (2001), Molecular Cloning: A Laboratory Manual, Cold Spring Harbor Laboratory Press, NY Cold Spring Harbor, NY).

Alternatively, non-phosphorylated PTKs used by inventive methods may be mutated (wt) PTKs (PTK mutants), which show one or more modifications for at least one amino acid as compared to the corresponding wildtype (wt) protein. Modifications may include any possible alteration in the amino acid sequence caused e.g. by additions of amino acids at the N- or C-terminus, or one or more insertions, deletions, substitutions, etc.. Mutants of (wt) PTKs may thus comprise fragments as well as full length sequences of the corresponding native proteins. Fragments of non-phosphorylated PTKs as used in step (a) of the inventive method typically comprise only a part of the full-length sequence of a PTK, e.g. one, two, three, etc. domains of a (wt) PTK. In a preferred embodiment of the inventive method such a fragment of the non-phosphorylated PTK substantially comprises the kinase (catalytic) domain of a (wt)-PTK, e.g. as shown in SEQ ID NO: 13, comprising the catalytic domain of Src-kinase. Mutants of (wt) PTKs as used by the present inventive method typically have an identity of at least 60 %, preferably of at least 70%, 80% or 90%, more preferably at least 95 % and most preferably at least 98 % with the respective (wt) PTKs. In the case of fragments of a non-phosphorylated PTK as used by the inventive method, these fragments typically have an identity of at least 70%, 80% or 90%, preferably at least 95 % and more preferably at least 98 % with the corresponding fragment of the respective (wt) PTK. Preferably, mutants of (wt) PTKs to be used by the inventive assay system are functionally homolog to their wt analogues. A functionally homolog mutant PTK in this context is typically a PTK showing essentially the same activity as its corresponding (wt) PTK, i.e. essentially the same auto-phosphorylation activity. The extent of auto-phosphoryltation activity may be measured by determining the kinetik of an auto-phosphoryltation reaction of such a mutant of (wt) PTK versus (wt) PTK. Functional homolog mutants of (wt) protein-tyrosine kinases as used in a method according to the present invention, comprise typically a characteristic, i.e. at least 90 %, preferably at least 95 % structure and sequence identity with the biologically functional regions of the native (wt) protein-tyrosine kinases.

Moreover, non-phosphorylated PTKs, as used by a method according to the present invention, may additionally comprise a moiety, e.g. for practicability reasons, e.g. for improving protein isolation and purification, for facilitating washing steps to remove unwanted impurities, or for prevention of trans-phosphorylation. Therefore, non-phosphorylated PTKs for use in inventive methods may preferably be expressed or modified with a chemical or biochemical group, a protein, a tag, e.g. selected from Maltose Binding protein (MBP), Gluthation-S-transferase (GST), Chitin Binding domain (CBD), His-tag, Strep-tag II (WSHPQFEK), FLAG-tag [(M)DYKDDDDK)], T7-tag, S-tag, Protein A, PinPoint-tag or Thioredoxine, or any other group suitable for immobilizing a PTK on a solid surface. Consequently, the PTK as used in the present invention may represent a modified protein or a fusion protein and/or may have a chemical or biochemical moiety preferably cleavably and covalently bound to the PTK.

In a preferred embodiment of the inventive method mutated (wt) PTKs, preferably mutated (wt) Src family protein-tyrosine kinases favor the closed, non-phosphorylated conformation. The closed, non-phosphorylated conformation of a PTK, typically cannot be subjected to an auto-phosphorylation reaction until it has switched to an open conformation. However, a closed conformation of a mutated (wt) PTK may atypically be altered into its open conformation by adding ATP. Thus, it is of utmost importance to avoid the presence of ATP when providing a non-phosphorylated PTK in a closed conformation in step (a) and any subsequent step of the inventive method, until the auto-phosphorylation reaction is to be initiated, e.g. in step (d) by adding ATP.

The determination of auto-phosphorylation of both (wt)-PTK and mutant-PTK, which predominantly exists in the closed conformation, allows to characterize test compounds and differentiate identified test compounds with respect to their binding mode (preferential binding to Src in their closed conformation vs. indiscriminative binding). As the non-phosphorylated kinase does not display any auto-phosphorylation during the preincubation process, inhibitors, binding both the open conformation and closed conformation of the kinase may be identified.

In another preferred embodiment of the inventive method the mutated (wt) protein-tyrosine kinases, preferably mutated (wt) Src family protein-tyrosine kinases, may display a hyperactive form of the corresponding (wt) protein-tyrosine kinases.

In another particular preferred embodiment mutated protein-tyrosine kinases as used in an inventive method are selected from sequences SEQ ID NO: 1 (sequence of 6His-Scr v1, human), SEQ ID NO: 2 (sequence of 6His-Scr v2, human), SEQ ID NO: 3 (sequence of 6His-hck, human) or SEQ ID NO: 13 (sequence of 6His-Scr catalytic domain, human).

Non-phosphorylated PTKs, as used in method step (a) of the present invention, may be provided in a liquid phase, for example in solution, or in suspension or can be bound to a solid phase. If the non-phosphorylated PTK is present in solution or suspension, the solution or suspension may contain a buffer suitable to prevent auto-phosphorylation of the PTK. Therefor, it is of utmost importance that the buffer as used by step (a) of the inventive method lacks ATP. Mg²⁺ may be present in a buffer as used in step (a). Alternatively, Mg²⁺ may be added in a subsequent step of the inventive method.

Suitable buffers for use in step (a) of the inventive method may be selected from a PBS buffer, PBS-Tween buffer (PBST), BSA-PBS buffer (PBS buffer with bovine serum albumin (BSA)), FKS-PBS buffer (PBS buffer with fetal calf serum (fcs)), phosphate-buffer, TRIS-HCl buffer, Tris-Phosphat-buffer, or other suitable buffers, such as Dulbeccos's modified Eagle Medium (DMEM) or RPIM with 5-10%FKS. Preferably, such buffers additionally may contain salts and stabilizing agents as appropriate. Particularly preferred buffers are Tris-HCl buffers, e.g. 10-100 mM Tris-HCl, pH 4-9, 0,01-50 mM Mg²⁺, 1-80% DMSO, more preferably 40-60 mM Tris-HCl, pH 6-8, 8-12 mM Mg²⁺, 20-40% DMSO, PBST buffers, or e.g. 10-100 mM Tris-HCl, pH 4-9, 0,01-50 mM Mg²⁺, more preferably 40-60 mM Tris-HCl, pH 6-8, 8-12 mM Mg²⁺, or e.g. 10-100 mM potassium phosphate, pH 4-9, 0,01-500 mM NaCl, 0,001-10% Tween, more preferably 40-60 mM potassium phosphate, pH 6-8, 100-200 mM NaCl, 0,01-0,2% Tween.

In step (a) as well as in steps (b), (c), (d), and (e) of the inventive methods different buffers may be used, e.g. for providing the non-phosphorylated protein-tyrosine kinase, for providing a test compound, for contacting the non-phosphorylated protein-tyrosine kinase with the test compound, for washing steps, for activating auto-phosphorylation of the non-phosphorylated protein-tyrosine kinase, or for measuring the degree of phosphorylation of the protein-tyrosine kinase via biochemical methods. In steps (a), (b), (c), (d), and (e) of the inventive method any of the above mentioned buffers may be used. If a washing step is inserted, any of the above mentioned buffers may be used as a first buffer and, if necessary, be substituted for a second buffer in a washing step, wherein the second buffer comprises the same or different components as the first buffer. The buffers used in any of steps (a), (b), (c), (d) and (e) of the inventive method, may have different pH-values. Preferably, pH-values of solutions or suspensions containing PTKs and/or a test compound in steps (a), (b), (c), (d) and/or (e) typically range between 4-9, preferably between 6-8. The temperature of a solution or suspension containing PTKs and/or a test compound, e.g. a solution or suspension in any of the aforementioned steps (a), (b), (c), (d) and (e), is usually kept in a range of from 0-40°C, preferably between room temperature (approx. 20-25°C) and 37°C. Finally, the amount of non-phosphorylated PTK provided in step (a) typically ranges of from 0,01-100 µM, preferably of from 0,05-50 µM, and more preferably of from 1-30 µM.

A liquid phase as used in step (a) of the inventive method will be typically prepared by adding the non-phosphorylated PTK into a buffer as defined above in a liquid form or in its lyophilized form. When adding the PTK to a buffer in a liquid form, the PTK will preferably be added in a such a concentration as to obtain the above disclosed final concentrations, which e.g. range of from 0,01-100 µM, preferably of from 0,05-50 µM, and more preferably of from 1-30 µM. If the non-phosphorylated PTK is added to the buffer in its lyophilized form, the PTK typically will be added in such a quantified amount as to obtain such a final concentration of PTK in the liquid phase as defined above. Preferably, after adding PTK to the buffer in its lyophilized form, PTK is usually completely dissolved in the liquid phase prior to proceeding with the next step of the inventive method.

As an alternative to providing non-phosphorylated PTK in a liquid phase according to step (a), PTK can be bound to a solid phase. PTK, for example, can be bound to a solid phase in the form of an essentially planar surface or in the form of particles, pearls or the like. Suitable solid phase materials preferably may be obtained from any solid phase, for example, in the form of an essentially planar surface or in the form of particles, pearls or the like, as used in adsorption chromatography, ion-exchange chromatography, affinity chromatography, etc.. More preferably solid phases are Ni-NTA coated plates, in particular Ni-NTA HisSorb Plate, white (Quiagen), Ni-NTA-agarose, beads, magnetic beads.

For providing non-phosphorylated PTKs as used in method step (a) according to the present invention, expression constructs, containing nucleic acids encoding these PTKs, can be generated to enable stable and, if necessary, inducible expression of PTKs and mutated PTKs as mentioned above in cells. These nucleic acids coding for PTKs and mutated PTKs as mentioned above can be contained in a vector.

Such a vector for providing a non-phosphorylated PTK as used in method step (a) of the inventive method is an entity which is capable of being introduced or of introducing the PTKs and/or nucleic acids encoding the PTKs into a host cell for expression. It is preferred that the PTKs or mutated PTKs encoded by the introduced nucleic acid of the invention are expressed within the cell upon introduction of the vector. The vector can exist separately in the cell or can be integrated into the genome of the cell according to the invention.

In a preferred embodiment such a vector as used to provide a non-phosphorylated PTK for step (a) of the present invention comprises plasmids, phagemids, phages, cosmids, artificial mammalian chromosomes, knock-out or knock-in constructs, viruses, in particular adenovirus, vaccinia virus, lentivirus (Chang, L.J. and Gay, E.E. (2001) Curr. Gene Therap. 1:237-251), Herpes simplex virus (HSV-1, Carlezon, W.A. et al. (2000) Crit. Rev. Neurobiol.), baculovirus, retrovirus, adeno-associated-virus (AAV, Carter, P.J. and Samulski, R.J. (2000) J. Mol. Med. 6:17-27), rhinovirus, human immune deficiency virus (HIV), filovirus and engineered versions thereof (see, for example, Cobinger G. P. et al (2001) Nat. Biotechnol. 19:225-30), virosomes, "naked" DNA liposomes, and nucleic acid coated particles, in particular gold spheres. Particularly preferred are viral vectors like adenoviral vectors or retroviral vectors (Lindemann et al. (1997) Mol. Med. 3:466-76 and Springer et al. (1998) Mol. Cell. 2:549-58). Liposomes are usually small unilamellar or multilamellar vesicles made of neutral cationic and/or anionic lipids, for example, by ultrasound treatment of liposomal suspensions. The DNA can, for example, be ionically bound to the surface of the liposomes or internally enclosed in the liposome. Suitable lipid mixtures are known in the art and comprise, for example, cholesterol, phospholipide like, for example, phosphatidylcholin (PC), phosphatidylserin (PS) and the like, DOTMA (1, 2-Dioleyloxpropyl-3-trimethylammoniumbromid) and DPOE (Dioleoylphosphatidylethanolamin) which both have been used on a variety of cell lines.

A large number of specialized prokaryotic vectors have been described (e.g., plasmids such as those capable of replication in E. coli such as, for example, pBR322, ColE1, pSC101, pACYC 184, piVX). Such plasmids are, for example, disclosed by Sambrook, J. et al. (In: Molecular Cloning, A Laboratory Manual, 2nd Ed., Cold Spring Harbor Press, Cold Spring Harbor, N.Y. (2001)). Sambrook, J. et al., herein incorporated by reference, provide a review of the characteristics of mammalian vectors.

For expression in mammalian cells, eukaryotic genes are typically cloned first into a bacterial vector and then subcloned into a vector suitable for eukaryotic expression or, preferably, expressed by a one step process. Thus, although many vectors have been described that are capable of replicating in both prokaryotic and eukaryotic cells, such vectors are designed to express a particular inserted polynucleotide in only one class of cell. Such vectors are illustrated, e.g. by U.S. Pat. No. 4,970,155 (Ikasinski, G. F.), which describes a prokaryotic plasmid that contains eukaryotic transcription and replication elements, such that an inserted polynucleotide can be expressed only in a eukaryotic cell. Similarly, U.S. Pat. No. 5,266,490 (Davis, S. et al.) describes an expression vector that contains an SV40 origin of replication, a eukaryotic transcription unit of the early immediate human cytomegalovirus (CMV) promoter region; and a generic polylinker and an SV40 splice/polyadenylation site. The vector also contains a pBR322 origin of replication and an antibiotic resistance gene under control of a prokaryotic promoter.

Also known are vectors that are capable of expressing an inserted gene in both prokaryotic and eukaryotic cells without any requirement to modify the vector or reclone the inserted gene. He, B. et al. (Gene 164: 75-79 (1995) described expression vectors that direct the synthesis of proteins from a common set of signals in both prokaryotic and eukaryotic cells. To allow transcription from a common promoter the vectors rely upon a phage RNA polymerase. To direct initiation of translation to the same start codon the vectors utilize an internal ribosome entry site from encephalomyocarditis virus that has been modified to include a prokaryotic ribosome-binding site at an appropriate distance upstream from the desired start codon. Mole, S. E. et al. (Nucl. Acids Res. 15: 9090 (1987) describe pSEMCatR1, a prokaryotic-eukaryotic shuttle vector compatible with pUR and lambda gt11 expression systems. Kaehler, R. et al. (DD 206791) discuss a hybrid expression vector which contains prokaryotic and eukaryotic control units directly connected to the sequence being expressed. Alting-Mees, M. A. (Strategies 5:58-61 (1992) describes the Stratagene(R) vector pBK-CMV, which is intended to be suitable for gene expression in both prokaryotes and eukaryotes. However, genes cloned into pBK-CMV are expressed only inefficiently in eukaryotic cells.

Expression vectors within the meaning of the invention are vectors being capable of mediating the expression of a nucleic acid. Preferred expression vectors according to the invention are e.g. pcDNA4/TO, PcDNA3-derivatives, pcDNA5/TO. Further examples are pGene/V5-His, pVg RXR and pIND-based inducible expression vectors.

For the expression constructs as used for providing the non-phosphorylated PTKs as used in method step (a) according to the invention, the pcDNA4/TO (Invitrogen, Carlsbad CA, USA) can be chosen as a preferred expression vector. This vector carries a tet-operator sequence enabling tetracycline (tet)-inducible expression in cells, preferably mammalian cells, of the desired transgene, whose nucleic acid, cDNA, has been cloned into its polylinker. The vector also contains a zeocin resistance gene which allows selection for stably transfected cells with the antibiotic zeocin (zeo). In various experiments of the invention, the human Src cDNA was cloned (directed) into the EcoRl restriction site of the vector's polylinker. In a preferred embodiment the expression of the nucleic acid of the expression construct is induced by adding tetracycline. Preferably, the induced expression causes an overexpression of the nucleic acid in the transfected cells.

A test compound is provided by step (b) of the method according to the present invention. "Compounds" or "test compounds" as used in the inventive method relate to any molecule which may modulate the activity of at least one PTK family kinase or PTK family kinase mutant, in particular at least one Src family kinase or Src family kinase mutant, or a further PTK as mentioned above. Typically, such test compounds can either directly or indirectly modulate at least the activity of one PTK family kinase or PTK family kinase mutant, in particular at least one Src family kinase or Src family kinase mutant. In particular, such test compounds can modulate auto-phosphorylation-competent protein kinases and inhibit the auto-phosphorylation reaction. The effect and potency of test compounds modulating auto-phosphorylation-competent protein kinases thus can be determined by the extent of suppression of such auto-phosphorylation reaction. A test compound as used by step (b) of the inventive method may be a small (organic) chemical entity, peptide antibody or intrabody molecule that effects the function of at least one member of the Src family. Suitable test compounds may be selected from compound collections or designed compounds, for example using combinatorial chemistry. Compound collections may be selected from the Cambridge Small Compound Library, Aldrich Library of Rare Chemicals or compound collections from Reaction Biology Corp. (RBC), ActiMol, AnalytiCon Discovery, Biofocus, BIOMOL Research Laboratories, Chembridge, Comgenex, Microsource /MSDI, Polyphor, Prestwick Chemical, SPECS and Biospecs, TimTec, Tripos, etc.. Preferably, the test compound has a molecular weight of less than 3000, more preferably of less than 1500 Da. A "test compound" as used by a method of the invention may be a peptide or a polypeptide, a modified peptide, e.g. a cyclic peptide or modified at the terminal end groups, e.g. with the introduction of a chloromethyl ketone, aldehyde, or boronic acid group at the C-Terminus or blocked at the N-terminus with a carbobenzyl group, a naturally occurring substance or a conjugate substance comprising at least two components. Test compounds as used in the method according to the present invention may be provided in a liquid phase, for example in solution, preferably in a liquid phase, preferably in aqueous solution or mixture of aqueous solutions with polar organic solvents, e.g. TFE, DMSO, chloroform, etc.. A liquid phase as used by step (b) of the inventive method, preferably an aqueous solution or a mixture of aqueous solutions, typically contains polar organic solvents in a final concentration of from 0-80%(w/v), preferably of from 0-60%(w/v) and more preferably in a concentration of from 0-40%(w/v). A liquid phase may contain buffers as already disclosed for step (a) of the inventive method. Similarly, it is of utmost importance that buffers as used in step (b) of the present invention lack ATP in order to avoid any auto-phosphorylation reaction of the non-phosphorylated PTK by subsequent step (c), when the liquid phase as used by step (b) is combined with the liquid phase as used by step (a) of the inventive method. The liquid phase as used by step (b) of the invention may contain buffers as mentioned above in final concentrations as disclosed above. The final concentrations of the test compound contained in the liquid phase as used by step (b) of the inventive method typically range of from 0,001-1000 µM, preferably of from 0,01-500 µM, and more preferably of from 0,05-10 µM, e.g. the concentration of the test compound is preferably selected such as to result in a final concentration in the liquid phase as used by step (b) e.g. of from 500, 200, 100, 50, 25, 12.5, 6.25, 3.125, 1.0, 0.2, 0.04, or 0.08 µM, after combining the solutions as used in steps (a) or (b) of the inventive method in a further step (c). The liquid phase as used by step (b) of the invention typically will be prepared by adding the test substance to the liquid phase in a liquid form or in a solid form. Prior to addition the test compound is typically contained in a buffer and/or an organic solvent as defined above. Preferably, the test compound will then be completely dissolved in the buffer and/or organic solvent.

Besides non-phosphorylated PTK and a test compound, respectively, the remaining components of a liquid phase as used in steps (a) or (b) of the inventive method may be identical or different to each other. Different to each other preferably means that independent from the other each liquid phase may contain different buffers, organic solvents, etc. as defined above Each liquid phase also may preferably contain independent from the other different final concentrations and/or pH-values in the ranges as defined above. Typically, the non-phosphorylated PTK, contained in the liquid phase as used in step (a), and the test compound, contained in the liquid phase as used in step (b), are present in a molar ratio of from 1 PTK : 1 to 5 test compound. More preferably, non-phosphorylated PTK and test substances are present in the liquid phase as used in step (a) or in step (b), respectively, in a molar ratio of from 1 PTK : 1 to 3 test molecules.

A "modulation of the activity of a PTK" in the sense of the present invention refers to any alteration of the auto-phosphoralytion activity of a PTK or a mutated PTK as used in an inventive method, particularly of a Src family protein tyrosine kinase or a mutated Src family kinase, as disclosed herein, e.g. decrease or increase, inhibit or activate, partially or completely their activity. A modulation of the auto-phosphoralytion activity may occur preferably via so called "direct inhibitors". "Direct inhibitors" directly bind to a domain of a PTK or a mutated PTK, particularly to a domain of a Src family kinase or Src family kinase mutant. Those direct inhibitors typically bind to the enzymatic kinase domain, especially to its ATP binding pocket or its substrate binding site. Nevertheless, effects due to binding of direct inhibitors to other sites or domains of PTKs are conceivable, since they may e.g. exert structural changes on the enzymatic kinase domain, alternatively to the substrate binding site or ATP binding site or the entry of the ATP binding site. Direct inhibitors of e.g. the Src kinase family are e.g. PP1-Chr. (Biomol), PP2 (Biomol) and SU6656.

It shall be understood, that inhibition of auto-phosphorylation activity of a PTK by a test compound typically prevents the PTK of getting phosphorylated in the kinase domain. Since phosphorylation in the kinase domain, preferably via auto-phosphorylation, is essential for a PTK in order to carry out a trans-phosphorylation reaction, modulation of auto-phosphorylation activity of a PTK, i.e. inhibiting auto-phosphorylation activity of a PTK, consequently also leads to an inhibition of trans-phosphorylation activity.

According to step (c) of the inventive method the protein-tyrosine kinase (PTK) as provided by step (a) and the test compound as provided by step (b) of the inventive method are brought into contact with each other, preferably forming a PTK/compound complex. "Contacting" according to the invention means - in its broadest sense - any interaction between a test compound with PTK or mutated PTK, in particular with a Src family protein-tyrosine kinase or a mutated Src family protein-tyrosine kinase, as disclosed herein, whereby any of the two components as provided by steps (a) or (b) of the inventive method may be independently provided in a liquid phase, for example in solution, preferably in a liquid phase containing one of the afore mentioned buffers, in suspension or bound to a solid phase. In order to avoid auto-phosphorylation prior to subsequent step (d), the presence of ATP has to be strictly avoided in buffers used for liquid phases or suspensions as already outlined above. The term "contacting" more specifically comprises any possibile interaction between a test compound and a protein-tyrosine kinase as used in the present inventive method.

As already mentioned above, PTK and test compounds typically are present in the liquid phases as provided by steps (a) or (b), respectively, in a molar ratio of from 1 PTK : 1 to 3 test compounds. Accordingly, the molar ratio of the resulting liquid phase obtained in step (c) preferably contains PTK and test compounds in a molar ratio of from 1 PTK : 1 to 3 test molecules. Also preferred, the concentrations of PTK and test compound in the liquid phases as provided by steps (a) or (b) are selected such as to preferably result in final concentrations for PTK in step (c) of from 0,01-100 µM, preferably of from 0,05-50 µM, and more preferably of from 1-30 µM. The final concentration of the test compound in step (c) preferably range of from 0,001-10 µM, preferably of from 0,01-5 µM, and more preferably of from 0,05-1000 µM. Upon preparing liquid phases as used in steps (a) or (b), dilution effects in subsequent step (c) are to be taken into account. Accordingly, in order to result at the desired concentrations in step (c), typically more concentrated liquid phases as used in step (a) or in step (b) with respect to PTK, test compound, buffer, organic solvent, etc. are provided.

After combining the solutions or suspensions of step (a) containing PTKs (or solutions/suspensions of step (a) containing PTK, being bound to a solid phase) and test compounds of step (b) in step (c), pH-values of the combined solutions or suspensions typically range between 4-9, preferably between 6-8. The temperature of step (c) usually is kept in a range of from 20-40°C, preferably between RT (room temperature, approx. 20-25°C) and 37°C. Incubation times typically range of from 1-400 minutes, preferably between 10 and 30 minutes.

After contacting the protein-tyrosine kinase (PTK) of step (a) and the test compound of step (b) the PTK may be optionally immobilized on a solid phase (step c') in order to avoid trans-activation by PTKs themselves and to enable a readout of the phosphorylation, preferably via an assay system. The immobilisation may occur via a chemical or biochemical group, a protein, a tag, etc.. In a preferred embodiment, the PTK is immobilized on a solid phase via a group selected from Maltose binding protein (MBP), Gluthation-S-transferase (GST), Chitin binding domain (CBD), His-tag, Strep-tag ll (WSHPQFEK), FLAG-tag [(M)DYKDDDDK)], T7-tag, S-tag, Protein A, PinPoint-tag or thioredoxine. Additionally, any other group suitable for immobilizing the PTK, as used in the method of the present invention, may be used.

After immobilizing the PTK in step (c') pH-values of solutions or suspensions containing PTKs and test compounds typically range between 4-9, preferably between 6-8. The temperature in step c') usually is kept in a range of from 20-40°C, preferably between RT (room temperature, approx. 20-25°C) and 37°C. Incubation times in step (c') typically range of from 1 minute up to 400 minutes, preferably between 5 and 60 minutes and more preferably between 10 and 30 minutes.

A solid phase as used in step (c') of the inventive method typically comprises any solid phase suitable to bind a PTK as used in the present invention with a significant strength, i.e. after binding the PTK to a solid phase and an optional washing step preferably no detectable "bleeding" of the PTK bound on the solid-phase material is observed. Suitable solid phase materials preferably may be obtained from any solid phase, for example, in the form of an essentially planar surface or in the form of particles, pearls or the like, as used in adsorption chromatography, ion-exchange chromatography, affinity chromatography, etc.. More preferably solid phases are Ni-NTA coated plates, in particular Ni-NTA HisSorb Plate, white (Quiagen), Ni-NTA-agarose, beads, magnetic beads.

Steps (a), (b) and (c') of the present invention may be carried out simultaneously or in an altered order alternatively to the above described sequences. E.g., the PTK may be provided first (a), thereafter the PTK is immobilized on a solid phase (c') and the test compound is added only after immobilization of the PTK in further step (c). Alternatively, the test compound may be combined with PTK in any conceivable way, preferably as disclosed herein and only thereafter PTKs are immobilized on a solid phase in a separate step.

After immobilizing the non-phosphorylated PTK on a solid surface optionally a washing step (c") may be inserted. Such a washing step preferably removes any non-bound PTK or excessive amounts of test compounds, buffers, detergents, etc.. A washing step also may be used in order to alter the reaction conditions, e.g. the buffer, the pH-value of the buffer, or to obtain a DMSO-free environment, etc.. Buffers suitable for a washing step preferably are the same as already indicated above for providing PTKs and/or test compounds. In order to avoid any auto-phosphorylation prior to step (d) as disclosed below, washing buffers as used prior to step (d) preferably no ATP is available. Washing step (c") may be repeated as often as regarded appropriate by a skilled person, e.g. once, two times, three times, four times, etc..

After a preincubation period, during which PTK molecules have been allowed to form complexes with a test compound and reaching an equilibrium of the above reaction, partial or full auto-phosphorylation activity of the PTK may be initiated according to step (d). Preferably, the initiation of auto-phosphorylation is carried out by adding an initiation buffer containing ATP and, if not yet contained, Mg²⁺, which allows the non-inhibited kinase molecules to auto-phosphorylate themselves in intramolecular mechanism in as detail explained above. The buffer used in step (d) is preferably a Tris-HCl buffer containing ATP and Mg²⁺, such as 10-100 mM Tris-HCl, pH 4-9, 0,01-50 mM Mg²⁺, 10-300 µM ATP, more preferably 40-60 mM Tris-HCl, pH 6-8, 8-12 mM Mg²⁺, 80-120 µM ATP. pH-values obtained in step (d) typically range between 4-9, preferably between 6-8. The temperature is usually kept in a range of from 20-40°C, preferably between room temperature (RT) (approx. 20-25°C) and 37°C. The PTK/compound-complex is then typically incubated in the initiation buffer with incubation times preferably in the range of from 1-600 minutes, preferably between 5 and 40 minutes, more preferably between 10 and 30 minutes. Preferably, the phosphorylation is carried out as an auto-phosphorylation, i.e. the PTK represents its own substrate and phosphorylates itself. This auto-phosphorylation in cis does not depend on molecule diffusion. As a result, this reaction is kinetically preferred, extremely fast and furthermore independent on the protein concentration. Thus auto-phosphorylation of PTKs, which are not inhibited by bound compound, is detectable and typically completed within a short time frame (several minutes in the case of the Src assay example).

In this context it is to be noted that auto-phosphorylation of a kinase according to step (d) of the inventive method is observed only, if the test compound, as used by step (b), does not exhibit a modulating, in particular an inhibiting, effect on the kinase of step (a). In other words, if a test compound of step (b) in fact modulates the kinase of step (a), e.g. inhibits the activity of the PTK, no auto-phosphorylation of the PTK is observed. Accordingly, suitable modulators of PTKs may be identified by the inventive method by testing as to whether auto-phosphorylation of a kinase (provided in step (a)) is diminished or inhibited after contacting same with a test compound (provided in step (b)). Upon analyzing the degree of auto-phosphorylation, a person skilled in the art thus can readily determine as to whether a test compound is capable of inhibiting the auto-phosphorylation reaction or as to whether this compound did not exhibit any modulating effect. In order to evaluate the test results from a method according to the present invention, a negative test can be carried out, e.g. an auto-phosphorylation assay according to the inventive method, wherein step (b) is omitted. By such a negative test, background signals may be determined, if present, and, if necessary, such results may be used as a baseline when evaluating the results from an auto-phosphorylation reaction according to the inventive method.

Typically no additional amounts of non-phosphorylated protein-tyrosine kinase as used in step (a), are added to the activated protein-tyrosine kinase after prior or simultaneously to the auto-phosphorylation of step (d). Additionally, since the PTK is typically immobilized on a solid surface and no additional PTK is added to the activated PTK after step (d), the activated PTK is preferably not hindered due to its immobilisation to undergo an auto-phosphorylation. This flexibility preferably may be achieved by selecting appropriate means for immobilizing the PTK on the solid surface and attaching them at adequate positions on the PTK in order to ensure flexibility, when immobilized, and not to block any phosphorylation site or ATP binding site. Suitable means for immobilizing PTK are typically any of the aforementioned immobilizing groups, in particular any of the aforementioned tags, such as His-tags, Strap-tag II, etc. or solid surfaces such as beads, e.g. as magnetic beads, or Ni-NTA-plates, and will be selected by a person skilled in the art.

By final step (e), the degree of phosphorylation of the protein-tyrosine kinase is measured, preferably via biochemical methods. A suitable biochemical method for measuring the degree of phosphorylation of the protein-tyrosine kinase preferably allows quantitative and/or qualitative determination. In a preferred embodiment, the biochemical methods comprise immunological methods, such as ELISA-assays, Western-Blots, antibody detection, detection via protein protein interactions, e.g. via SH2 domains, pTyr specific peptides, etc, detection of absorption, such as fluorescence detection, fluorescence depolarisaton, chemoluminiscence detection, bioluminescence detection, detection of radioactive ligands, detection via small chemical or biochemical molecules, such as alkaline phosphatases, GFP, EGFP, Gluthathione S-transferase, etc.. In a particularly preferred embodiment, the degree of phosphorylation of the protein-tyrosine kinase is determined by antibody binding, e.g. via detection by enzyme reaction of horse radish peroxidase (HRP)-labelled anti-phospho-tyrosine (a-PY) (HRP-labeled phosphotyrosine specific antibody (Abcam, anti-Phosphotyrosine ab 9329)), via anti-phosphotyrosine, recombinant 4G10 (HRP-conjugate, 16-105, Upstate), via monoclonal anti-phosphotyrosine, peroxidase-conjugate from mouse (purified immunoglobulin, lyophilized powder, clone PT-66, A5965 Aldrich). Particularly in connection with any of the afore mentioned assays, e.g. an antibody binding assay with the above mentioned horse radish peroxidase (HRP) - labelled anti-phospho-tyrosine (a-PY), chemoluminiscent detection reagent, e.g. ECL™ Western Blotting reagents may additionally to the above be added to the bound antibody/PTK complex, whereby detection and probably quantification of the chemoluminiscence intensity is achieved with a chemoluminiscence reader. Other suitable chemoluminiscence agents are LumiGlO® Chemoluminiscent substrate (catalog #:20-212 BIOMOL), CL substrate RTU Applied Biosystems (part number: 106823). In a qualitative type of analysis the amount of auto-phosphorylated protein in the presence of inhibitor is compared with the amount of auto-phosphorylated protein in the absence of inhibitor. Therefore, preferably each a signal of the auto-phosphorylated protein in the presence and in the absence of inhibitor is observed, the difference of which indicates as to whether auto-phosphorylisation has taken place or has been inhibited by the test compound. In a quantitative assay the amount of auto-phosphorylated protein at different inhibitor concentrations is monitored and allows determination of compound IC50 values. Preferably, the test compound and the PTK are incubated in different concentrations and auto-phosphorylation is measured. Therefore, preferably either the final concentrations of PTK in step (d) is kept at a constant level of e.g. from 0,08-500 µM, e.g. 500, 200, 100, 50, 25, 12.5, 6.25, 3.125,1.0, 0.2, 0.04, 0.08 µM, whereas the test compound is provided in step (d) in at least one final concentration, preferably in a dilution series having final concentrations e.g. selected from 0,08-500 µM, particularly from 500, 200, 100, 50, 25, 12.5, 6.25, 3.125,1.0, 0.2, 0.04, 0.08 µM.

The invention also relates to a compound or a pharmaceutically acceptable derivative thereof identified, selected and/or characterized by a method according to the present invention. Preferably, this compound is obtained or obtainable by a method of the invention. Preferably, this compound is suitable for the treatment of diseases, which are at least in part caused by a PTK, in particular by a Src protein tyrosine kinase..

A pharmaceutically acceptable derivative of a test compound according to the invention relates to any non-toxic salt, ester, salt of an ester or other derivative of a compound of this invention that, upon administration to a recipient, is capable of providing, either directly or indirectly, a compound of this invention or an inhibitorily active metabolite or residue thereof.

Pharmaceutically acceptable salts of the test compounds of this invention include those derived from pharmaceutically acceptable inorganic and organic acids and bases.

Diseases, which are at least in part caused by a PTK as used in the inventive method, are preferably any diseases or other deleterious conditions in which at least one PTK is known or will be known to play a role. Accordingly, the test compounds of the invention are useful for treating diseases or conditions that are known to be affected by the activity of one or more PTKs.

The invention also relates to the use of a test compound or a pharmaceutically acceptable derivative thereof according to the invention for the preparation of a pharmaceutical composition for the treatment of diseases, which are caused at least in part by a PTK, preferably cancer, diseases caused by metabolic defects, diabetes, particularly diabetes type 1 and 2, diseases caused by developmental defects or degeneration, hypercalcemia, restenosis, osteoporosis, osteoarthritis, symptomatic treatment of bone metastasis, rheumatoid arthritis, inflammatory bowel disease, multiple sclerosis, psoriasis, lupus, graft vs. host disease, T-cell mediated hypersensitivity disease, Hashimoto's thyroiditis, Guillain-Barre syndrome, chronic obstructive pulmonary disorder, contact dermatitis, Paget's disease, asthma, ischemic or reperfusion injury, allergic disease, atopic dermatitis or allergic rhinitis.

Furthermore, the invention also relates to a pharmaceutical composition for the treatment of diseases (or for manufacturing drugs for the treatment of diseases), which are caused at least in part by a PTK, preferably cancer, diseases caused by metabolic defects, diabetes, particularly diabetes type 1 and 2, diseases caused by developmental defects or degeneration, hypercalcemia, restenosis, osteoporosis, osteoarthritis, symptomatic treatment of bone metastasis, rheumatoid arthritis, inflammatory bowel disease, multiple sclerosis, psoriasis, lupus, graft vs. host disease, T-cell mediated hypersensitivity disease, Hashimoto's thyroiditis, Guillain-Barre syndrome, chronic obstructive pulmonary disorder, contact dermatitis, Paget's disease, asthma, ischemic or reperfusion injury, allergic disease, atopic dermatitis or allergic rhinitis.

Preferably, the test compounds and/or pharmaceutical compositions according to the invention or identified by methods according to the invention are administered for the treatment of various forms of non-solid and solid cancers. Examples for cancers which can be treated by the test compounds and/or pharmaceutical compositions according to the invention are Hodgkin lymphoma, non-Hodgkin lymphoma, histocytic lymphoma, cancers of the brain (glioblastomas), ovarian, genitourinary tract, colon, liver, colorectal tract, pancreas, breast, prostate, lymphatic system, stomach, larynx and lung, including lung adenocarcinoma and small cell lung cancer and/or skin, e.g. melanoma or non-melanoma skin cancer, including basal cell and squamous cell carcinomas.

A pharmaceutically acceptable carrier, adjuvant, or vehicle according to the invention refers to a non-toxic carrier, adjuvant or vehicle that does not destroy the pharmacological activity of the compound with which it is formulated. Pharmaceutically acceptable carriers, adjuvants or vehicles that may be used in the compositions of this invention include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat.

The pharmaceutical compositions of the present invention may be administered orally, parenterally, by inhalation spray, topically, rectally, nasally, buccally, vaginally or via an implanted reservoir.

The term parenteral as used herein includes subcutaneous, intravenous, intramuscular, intra-articular, intra-synovial, intrasternal, intrathecal, intrahepatic, intralesional and intracranial injection or infusion techniques. Preferably, the compositions are administered orally, intraperitoneally or intravenously. Sterile injectable forms of the compositions of this invention may be aqueous or oleaginous suspension. These suspensions may be formulated according to techniques known in the art using suitable dispersing or wetting agents and suspending agents. The sterile injectable preparation may also be a sterile injectable solution or suspension in a non-toxic parenterally-acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium.

For this purpose, any bland fixed oil may be employed including synthetic mono- or diglycerides. Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions may also contain a long-chain alcohol diluent or dispersant, such as carboxymethyl cellulose or similar dispersing agents that are commonly used in the formulation of pharmaceutically acceptable dosage forms including emulsions and suspensions. Other commonly used surfactants, such as Tweens, Spans and other emulsifying agents or bioavailability enhancers which are commonly used in the manufacture of pharmaceutically acceptable solid, liquid, or other dosage forms may also be used for the purposes of formulation.

The pharmaceutically acceptable compositions of this invention may be orally administered in any orally acceptable dosage form including, but not limited to, capsules, tablets, aqueous suspensions or solutions. In the case of tablets for oral use, carriers commonly used include lactose and corn starch. Lubricating agents, such as magnesium stearate, are also typically added. For oral administration in a capsule form, useful diluents include lactose and dried cornstarch. When aqueous suspensions are required for oral use, the active ingredient is combined with emulsifying and suspending agents. If desired, certain sweetening, flavouring or colouring agents may also be added.

Alternatively, the pharmaceutically acceptable compositions of this invention may be administered in the form of suppositories for rectal administration. These can be prepared by mixing the agent with a suitable non-irritating excipient that is solid at room temperature but liquid at rectal temperature and Therefore will melt in the rectum to release the drug. Such materials include cocoa butter, beeswax and polyethylene glycols.

The pharmaceutically acceptable compositions of this invention may also be administered topically, especially when the target of treatment includes areas or organs readily accessible by topical application, including diseases of the eye, the skin, or the lower intestinal tract. Suitable topical formulations are readily prepared for each of these areas or organs.

Topical application for the lower intestinal tract can be effected in a rectal suppository formulation (see above) or in a suitable enema formulation. Topically-transdermal patches may also be used.

For topical applications, the pharmaceutically acceptable compositions may be formulated in a suitable ointment containing the test compound as identified by an inventive method suspended or dissolved in one or more carriers. Carriers for topical administration of the test compounds of this invention include, but are not limited to, mineral oil, liquid petrolatum, white petrolatum, propylene glycol, polyoxyethylene, polyoxypropylene compound, emulsifying wax and water. Alternatively, the pharmaceutically acceptable compositions can be formulated in a suitable lotion or cream containing the test compound suspended or dissolved in one or more pharmaceutically acceptable carriers. Suitable carriers include, but are not limited to, mineral oil, sorbitan monostearate, polysorbate 60, cetyl esters wax, cetearyl alcohol, 2-octyldodecanol, benzyl alcohol and water.

For ophthalmic use, the pharmaceutically acceptable compositions may be formulated as micronized suspensions in isotonic, pH adjusted sterile saline, or, preferably, as solutions in isotonic, pH adjusted sterile saline, either with or without a preservative such as benzylalkonium chloride. Alternatively, for ophthalmic uses, the pharmaceutically acceptable compositions may be formulated in an ointment such as petrolatum.

The pharmaceutically acceptable compositions of this invention may also be administered by nasal aerosol or inhalation. Such compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other conventional solubilizing or dispersing agents.

Most preferably, the pharmaceutically acceptable compositions of this invention are formulated for oral administration.

The amount of the test compounds of the present invention that may be combined with the carrier materials to produce a composition in a single dosage form will vary depending upon the host treated, the particular mode of administration. Preferably, the compositions should be formulated so that a dosage of between 0.01-100 mg/kg body weight/day of the inhibitor can be administered to a patient receiving these compositions. Preferred dosages range from 0.1-5 mg/kg body weight/day, even further preferred dosages from 1 - 5 mg/kg body weight/day.

It has to be noted that a specific dosage and treatment regimen for any particular patient will depend upon a variety of factors, including the activity of the specific compound employed, the age, body weight, general health, sex, diet, time of administration, rate of excretion, drug combination, and the judgment of the treating physician and the severity of the particular disease being treated. The amount of a test compound of the present invention in the composition will also depend upon the particular test compound in the composition.

Finally, the present invention also relates to kits suitable in a method according to the present invention for identifying, selecting and/or characterizing a compound which modulates the activity of at least one protein-tyrosine kinase (PTK). Such a kit may comprise one ore more of the inhibitors, identified by a method according to the present invention, and optionally buffers and instructions for use.

The following figures and examples are thought to illustrate the invention and should not be construed to limit the scope of the invention thereon. All references cited by the disclosure of the present application are hereby incorporated in their entirety by reference.

### Description of the Figures:

- Figure 1:: shows a schematic drawing of the assay
- Figure 2:: shows a bar plot of the chemoluminiscence signal in dependence of the amount of kinase used for immobilization of Src kinase (Srcv1) on the Ni-NTA coated 96 wells. With increasing amount of kinase, the signal increases, as the binding sites on the well surface become saturated. In the case of the kinase which was autoactivated by incubation with ATP a correlation between kinase amount, used for immobilization on the Ni-NTA plate and signal can be observed (see also Example 2.2).
- Figure 3:: shows bar plots of the inhibition of Src auto-phosphorylation by kinase inhibitors in Figures 3A and 3A (see Figures 3A and 3B and Example 2.3).
- Figure 3A:: shows a bar plot of the inhibition of Src kinase (100µl, 2.0 µM) with kinase inhibitor (PP1, 1.25 mM, Biomol, CAS: 172889-26-8). In the experiment Src kinase (100µl, 2.0 µM) was incubated with kinase inhibitor (PP1, 1.25 mM, Biomol) and activated with ATP (100 µM). A clear inhibition of the kinase activity is regarded (left bar), whereas in a control experiment containing no inhibitor but 100 µM ATP no signal was detected (middle bar). A control experiment containing no inhibitor and no ATP also resulted in no signal (right bar) (see also Example 2.3).
- Figure 3B:: shows a bar plot of the inhibition of Src kinase (100µl, 1.0 µM) with different kinase inhibitors 100 µl src kinase (1.0 µM): from left to right: compound1, compound2, PP1, and a control containing no inhibitor. PP1 showed the strongest inhibition of Src kinase followed by compound 2 and 1 (see also Example 2.3).

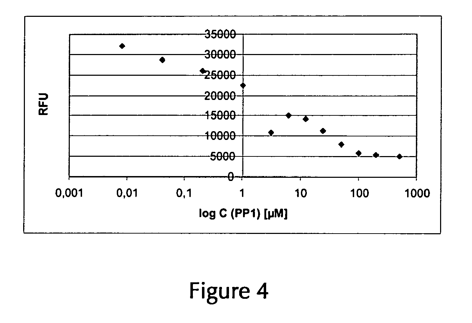

- Figure 4:: shows a graph of a dose response curve obtained from a quantitative analysis of the inhibition of Src kinase with inhibitor PP1. Therefore, 200 µl kinase (0.5 µM) were incubated with a dilution series of PP1 (500, 200, 100, 50, 25, 12.5, 6.25, 3.125, 1.0, 0.2, 0.04, 0.08 µM). On the y-axis the chemoluminiscence signal is displayed, on the x-axis the logarithmic compound concentration in µM is displayed. From the dose response curve obtained by this experiment an IC₅₀- value for PP1 can be determined (IC₅₀~ 10 µM). Thus it can be concluded that the autoactivation assay can be used for quantitative analysis. (see also Example 2.4).
- Figure 5:: shows a graph of several dose response curves (Figures 5A-C) obtained from quantitative analysis of the inhibition of different amounts of Src kinase with inhibitor PP1 and 6151. The test compounds PP1 and 6151 were titrated (500, 200, 100, 50, 25, 12.5, 6.25, 3.125,1.0, 0.2, 0.04, 0.08 µM) at three different kinase concentrations (0.1, 0.25 and 0.5 µM Src kinase) The chemoluminiscence signal is displayed on the y-axis, the logarithmic compound concentration in µM on the x-axis (see also Example 2.5).
- Figure 6:: shows a graph of several dose response curves obtained from quantitative analysis of the inhibition of Src kinase Srcv1 and of different amounts of Src kinase Srcv2 with inhibitor PP1 and 6151 (500, 200, 100, 50, 25, 12.5, 6.25, 3.125,1.0, 0.2, 0.04, 0.08 µM). On the y-axis the chemoluminiscence signal is displayed, on the x-axis the logarithmic compound concentration in µM is displayed. (see also Example 2.6)
- Figure 7:: shows several graphs of dose response curves (Figures 7A-C) obtained from quantitative analysis of the inhibition of Src kinase Srcv2 (0,25 µM) with inhibitors PP1, 5953 and 6151 (500, 200, 100, 50, 25, 12.5, 6.25, 3.125,1.0, 0.2, 0.04, 0.08 µM). On the y-axis the chemoluminiscence signal is displayed, on the x-axis the logarithmic compound concentration in µM is displayed. Each of the investigated inhibitors showed inhibition of Srcv2 kinase (see also Example 2.7.1).

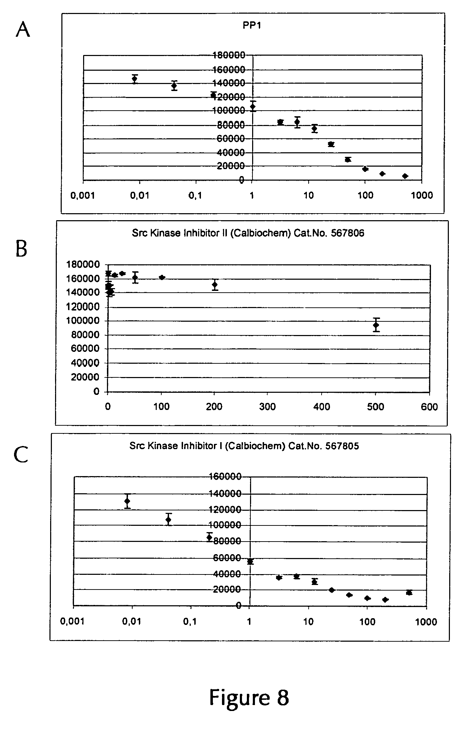

- Figure 8:: shows several graphs of IC 50 titration curves (Figures 8A-C) of the two src inhibitors Src kinase inhibitor I (Calbiochem, Cat. No. 567805) and Src Kinase Inhibitor II (Calbiochem, Cat. No. 567806). The two src kinase inhibitors provided by Calbiochem show the same inhibition profile in the src autoactivation assay as described in literatur. While the inhibitor I shows a stronger inhibition than PP1, the inhibitor II shows weak to no inhibition in the autoactivation assay. On the y-axis the chemoluminiscence signal is displayed, on the x-axis the logarithmic compound concentration in µM is displayed (see also Example 2.7.2).
- Figure 9:: shows a bar plot of an analysis of test compounds performed with the method according to the present invention. The test compounds had a final concentration of 500 µM in the assay. The DMSO concentration was 30 %.
Test compounds were defined as hits if the residual activity was beyond a defined threshold.
- Figure 10:: shows several graphs of dose response curves (Figures 10A-F) obtained from quantitative analysis of the inhibition of Src kinase Srcv2 (0,25 µM) with dilution series of inhibitors found as hits in the qualitative assay (500, 200, 100, 50, 25, 12.5, 6.25, 3.125,1.0, 0.2, 0.04, 0.08 µM), for lC50 determination. The standard deviation is shown by error bars. On the y-axis the chemoluminiscence signal is displayed, on the x-axis the logarithmic compound concentration in µM is displayed (see also Example 2.7.4).

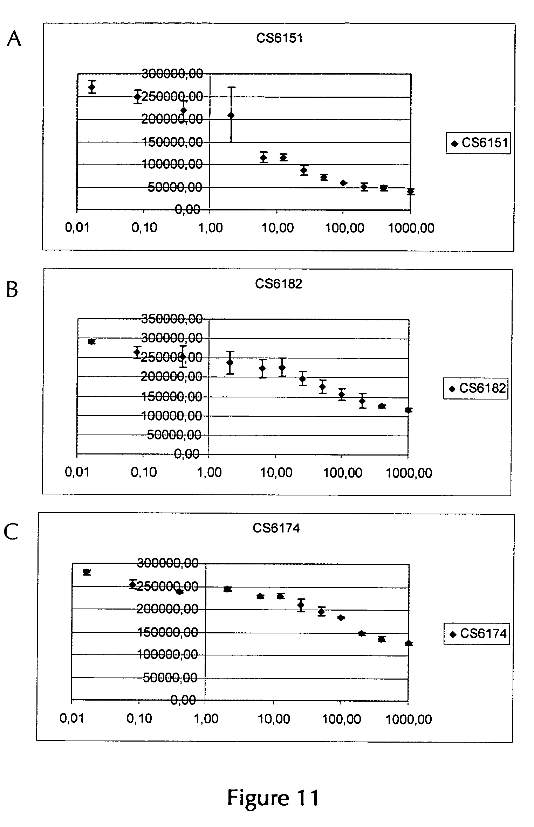

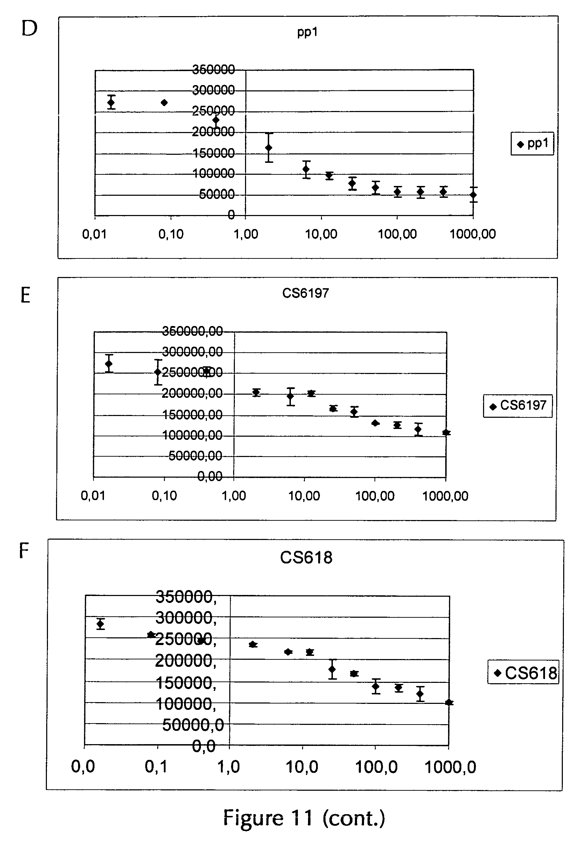

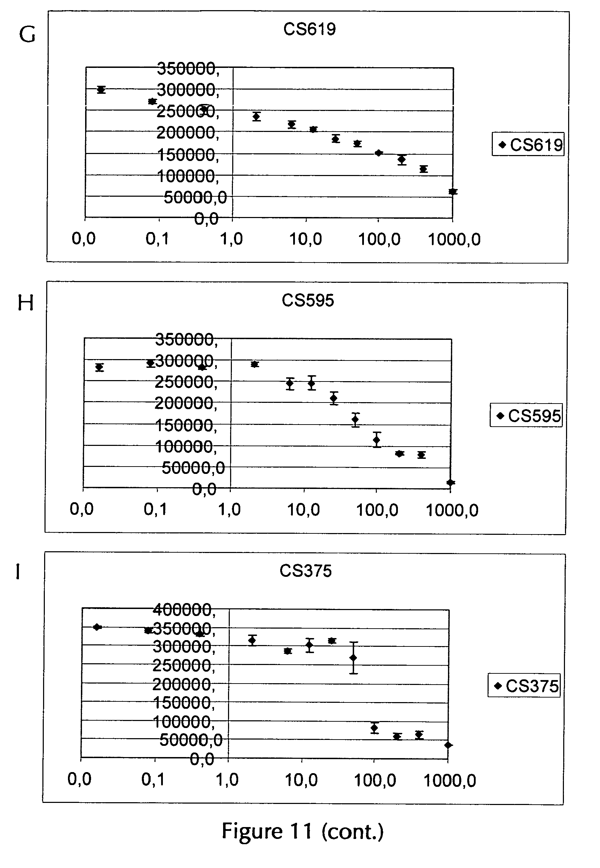

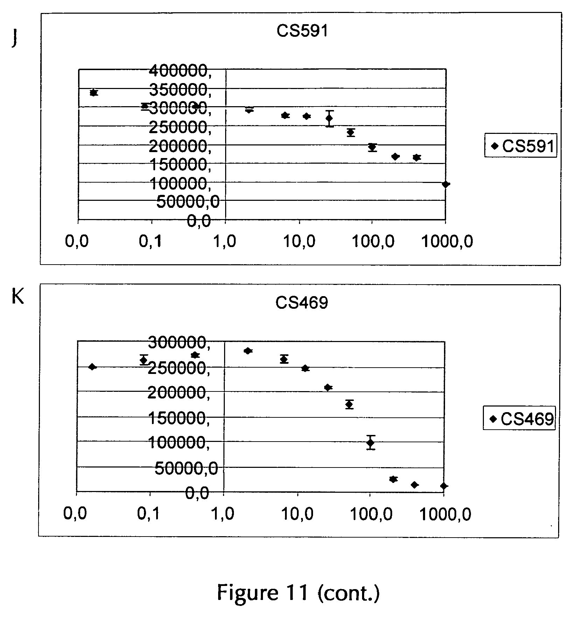

- Figure 11:: shows several graphs of dose response curves (Figures 11A-K) obtained from quantitative analysis of the inhibition of Src kinase Hck with dilution series of the inhibitors 65151, 6182, 6174, PP1, 6197, 6185, 6199, 5950, 3752, 5913 and 4695 (500, 200, 100, 50, 25, 12.5, 6.25, 3.125,1.0, 0.2, 0.04, 0.08 µM) for lC50 determination. The measurements were carried out as triplicates, where the standard deviation is shown by error bars. On the y-axis the chemoluminiscence signal is displayed, on the x-axis the logarithmic compound concentration in µM is displayed (see also Example 2.8).
- Figure 12:: shows a plot showing the readout of kinase activity using an alpha screen kinase assay (for detailed description visit Perkin Elemer web site) for testing the maximum concentration of DMSO at which Src kinase still exhibits activity. The kinase dilution series were carried out at increasing DMSO concentrations (0-80%, Figure 12a) (0-50%, Figure 12b) (see also Example 3.3).
- Figure. 13:: shows a SDS PAGE of eluate fractions after Ni-NTA Chromatography. The following samples are shown from left to right: protein marker M7 (Sigma, MW-SDS-701), flow through fraction, eluate fractions (see also Example 1.2.2).
- Figure. 14:: SDS PAGE of eluate fractions after Ni-NTA Chromatography. The following samples are shown from left to right: protein marker M7 (Sigma, MW-SDS-701), sample load, eluate fractions (see also Example 1.2.3).
- Figure 15:: SDS PAGE of eluate fractions after Ni-NTA Chromatography. The following samples are shown from left to right: protein marker M7 (Sigma, MW-SDS- 701), flow through fraction, sample load, eluate fractions (see also Example 1.3.5).
- Figure. 16:: SDS PAGE of eluate fractions after Ni-NTA Chromatography. The following samples are shown from left to right: protein marker M7 (Sigma, MW-SDS-701), sample load, eluate fractions (see also Example 1.3.6).

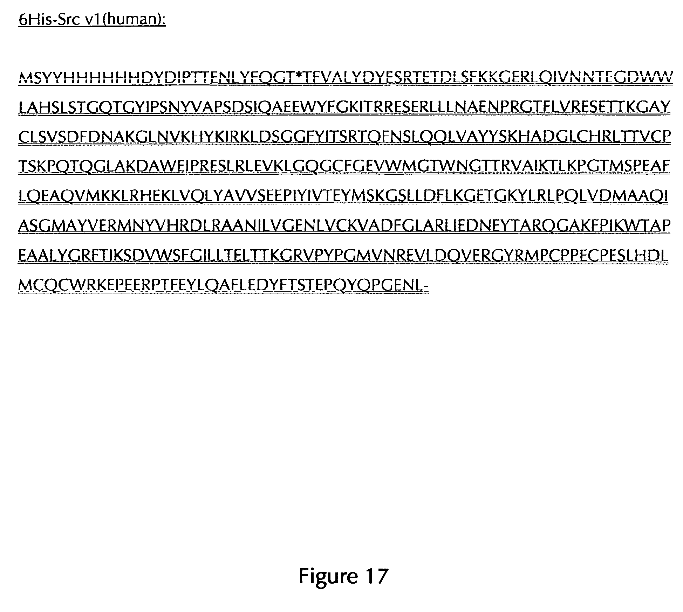

- Figure 17:: Peptide sequence of 6His-Src v1 (human); the number of amino acids is 474, T* = T87 of human Scr, the His6-tag is underlined, the TEV-cleavage site is double underlined.

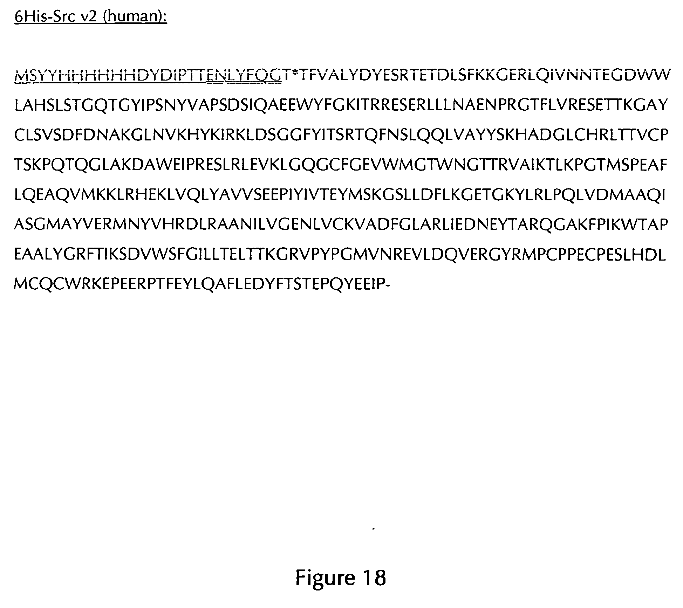

- Figure 18:: Peptide sequence of 6His-Src v2 (human) ; the number of amino acids is 472, T* = T87 of human Scr, the His6-tag is underlined, the TEV-cleavage site is double underlined.

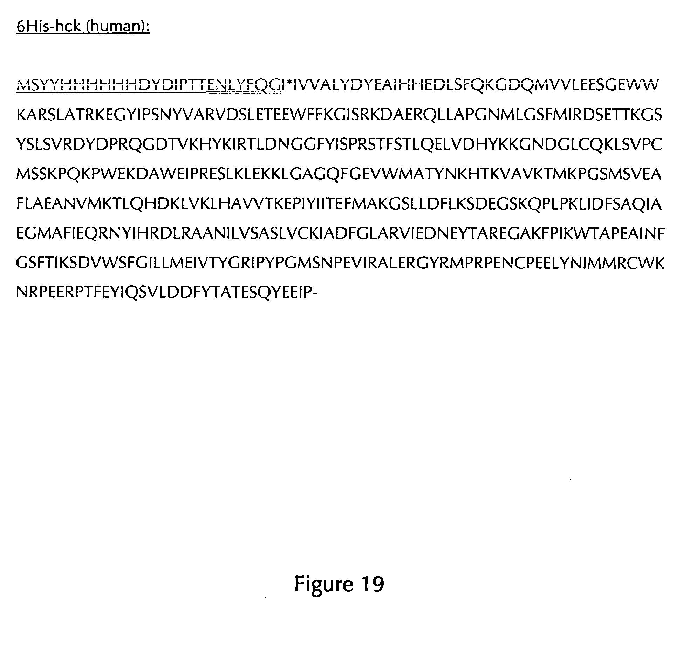

- Figure 19:: Peptide sequence of 6His-Hck (human) ; the number of amino acids is 470, l* = 180 of human Hck, the His6-tag is underlined, the TEV-cleavage site is double underlined.
- Figure 20a-d:: Sequence alignment of protein-tyrosine kinases from the Src family (Src, Yes, Fyn, Yrk, Fgr, Hck, Lyn, Lck and Blk)

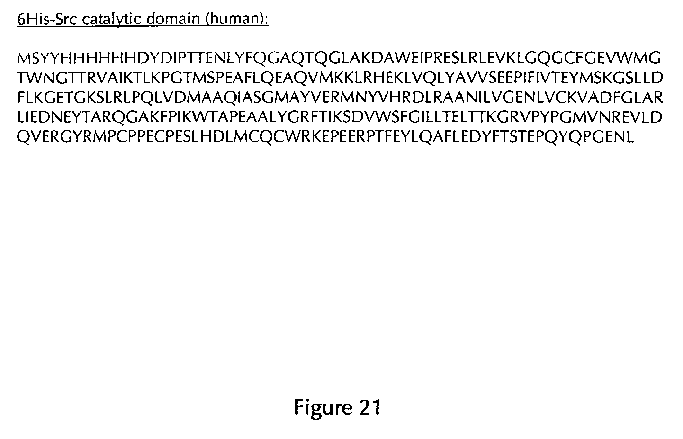

- Figure. 21:: shows the sequence of 6His-Scr domain, comprising the catalytic domain of Src-kinase (see also SEQ ID NO: 13 and Example 4) ; the number of amino acids is 308, Q* = Q254 of human Scr, The His6-tag is underlined, the TEV-cleavage site is double underlined.
- Figure. 22A:: shows a SDS PAGE of eluate fractions of the catalytic domain of Src-kinase after anion exchange chromatography. The following samples are shown from left to right: sample load (lane 1), eluate fractions (lanes 2-10), protein marker M7 (lane 11) (Sigma, MW-SDS-701) (see Examples 4.1.1-4.1.6).
- Figure.22B:: shows a chromatogram of a gelfiltration chromatography of the catalytic domain of Src-kinase, the second peak corresponds to the of the catalytic domain of Src-kinase (see Examples 4.1.1-4.1.6).
- Figure 23:: shows a bar plot of a comparison of full length src kinase and the phosphatase treated catalytic domain of Src-kinase in an autoactivation assay. The PP1 titration curves were carried out with the full length Src kinase and phosphatase treated catalytic domain of src kinase. As a result the phosphatase treated inactive catalytic domain of Src shows similar results in the autoactivation assay as full-length Src. Similar dose response curves were obtained for both proteins (see Example 4.2).

### Examples

### 1. Preparation of PTK proteins and plasmids encoding therefore

### 1.1 Cloning and Expression of Src kinase v1 and v2

Both Src kinase proteins used in the methods according to the present invention contain a N-terminal His₆-tag to allow Ni-NTA affinity purification and immobilization of the proteins on Ni-NTA coated plates. Srcv1 is the wild type form of Src kinase while Srcv2 contains a C-terminal modification (Y530-EEIP in srcv2 instead of Y530-QPGENL in srcv1). Due to this modification at the C-terminus of Srcv2 the affinity of pY-530 to Src SH2 domain is increased, which in turn leads to stronger inactivation of Src v2. Thus this modification allows expression and purification of a homogeneously C-terminal (at Y530) phosphorylated Src kinase, which is present predominatly in its closed conformation.

### 1.1.1 Srcv1 (n-terminally truncated):

1 Srcv1 (n-terminally truncated) was prepared by amplifying the insert, matching exactly to the c-Src-construct mentioned in "Sicheri et al., 1997, with proofreading pwo-polymerase for subcloning including the primers ATATAAGCTTCTAGAGGTTCTCCCC-GGGCTG (L-1611 hSrc-Hind3+) (SEQ ID NO: 14) and ACCACCTTTGTGGCCCTCTATGACTATGAGTC (SEQ ID NO: 15) (U-src-259blunt). Afterwards, a restriction digest of the PCR (Hindlll) insert and pFastBacHta-vector (Ehel/Hindlll) was performed. Vector and insert were ligated, transformed in E. coli Mach1 (invitrogen), and proofed by sequencing. The resulting transfer plasmid, named pFastBacHTa-c-src(h)-FL-var3-clone8, was then transformed into E. coli DH10Bac. Bacmid DNA was isolated resulting in bacmid DNA bMON14272-HTa-SrcFLVar3 8.1.

Baculoviruses were generated, containing srcFLVar3, strictly according to Invitrogen Bac to Bac System and expressed in Sf9 insect cells: Sf9 cells were cultivated in 200ml suspension cultures (Sf900ll medium - Invitrogen - containing 6-8% FCS GOLD - PAA - and 1% Pluronic - Sigma). At a density of 1,3-1,75 x 10⁶ cells/ml, cells were infected and harvested 72 h post infection.

### 1.1.2 Srcv2 (n-terminally truncated, c-terminal variation):

Srcv2(n-terminally truncated, c-terminal variation) was prepared by amplifying the insert. The insert was amplified with proofreading pwo-polymerase for subcloning using primers carrying spacer and variation, e.g. ATATCCATGGGTCTGGCCGG-TGGAGTGACCACCTTTGTGGCCCTCTATGACTATGAGTCTAGG (U-hSrc-Var4) (SEQ ID NO: 16) and TATACTCGAGTTAAGGGATCTCTTCGTACTGGGGCTCGGTGGACGTG-AAGTAGTCCTCCAGG (L-hSrc-Var4) (SEQ ID NO: 17).

Afterwards, a restriction digest of both the PCR insert and pFastBacHta-vector was performed with Ncol/Xhol. A Ligation of vector and insert was carried out and a transformation in E. coli Mach1 (invitrogen), proofed by sequencing. The resulting transfer plasmid: pFastBacHTa-src(h)-var4-clone7 was then transformed into E. coli DH10Bac. Bacmid DNA was isolated resulting in bacmid DNA bMON14272-HTa-src(h)-var4-clone 7-4 and bMON14272-HTa-src(h)-var4-clone 7-6. Generation of baculoviruses containing srcFLVar3 was strictly performed according to Invitrogen Bac to Bac System and expression in Sf9 insect cells: Sf9 cells were cultivated in monolayers (Sf900ll medium - Invitrogen - containing 6-8% FCS GOLD - PAA - and 0,1% Pluronic - Sigma), the cells were infected at a density of 2 x 10⁶ cells/ml and harvested 72 h post infection.

### 1.2 Purification of src kinase v1

### 1.2.1 Lysis of cell pellets

Pellets resulting from 2000 ml Sf9 cell culture were resuspended in 100 ml ice cooled lysis buffer (20mM Tris, pH 8.3, 10% glycerol , 2mM TCEP, 4µl Benzonase (250 u/µl Merck 1.01656.0001), 0.2mM AEBSF, 0.05% Triton-X-100). 100ml raw extract were filled in a rosette glas vial on ice. Cell lysis was carried out under the following conditions: pulse: 50% per second, duration: 4min - brutto, power: 8, size of rosette vessel: ~150ml. Afterwards, the lysate was clarified by centrifugation for 30 min at 4 °C at ~41800 x g (Evolution RC centrifuge, Sorvall SA-600 rotor). The general yield of cell lysate after this procedure is ~400ml-600 ml

### 1.2.2 Ni-NTA affinity chromatography

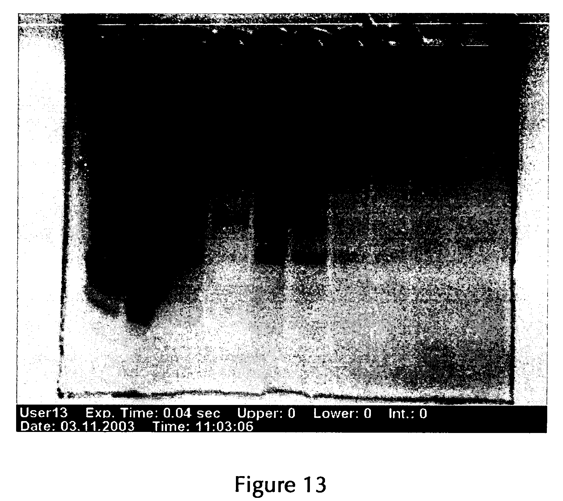

The obtained celllysate (400 ml) was loaded onto a Ni-NTA column (bed volume ~10ml Ni-NTA superflow matrix, Quiagen), which was subsequently equilibrated in IMAC-A bufferA (50 mM NaH₂PO₄, pH 8.0, 300 mM NaCl, 10 mM Imidazol, 5% Glycerol). The sample was applied to column using the system pump of the Äkta Prime (Pharmacia) with a flow rate of 10 ml/min. Afterwards sample loading was finished the column was extensively washed with IMAC-A buffer until baseline was reached. Afterwards a step gradient (0- 10% IMAC-B buffer (50 mM NaH₂PO₄, pH 8.0, 300 mM NaCl, 500 mM Imidazol, 5% Glycerol)) and a linear gradient ,(10%-100% in 100 ml, IMAC-B buffer) was performed. Eluate fractions were collected in 10 ml fractions. The Puritiy of eluate fractions was checked by SDS-PAGE (see Figure 13) and eluate fractions were pooled afterwards. The general yield of after this procedure is 100-150 ml. The results are presented in Figure. 13.

### 1.2.3 Gelfiltration

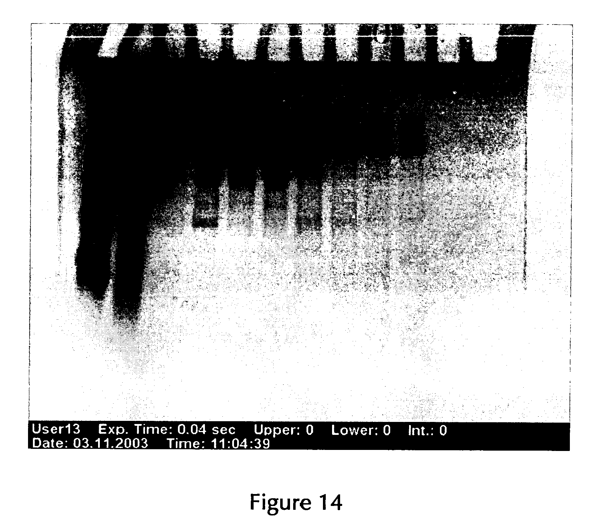

Gelfiltration was carried out using the Äkta purifier 100 (Pharmacia). The volume of the protein solution obtained after the IMAC purification step was reduced from ~140 ml to 7 ml using Centriprep YM-10 concentrators (Amicon). 5 ml of the protein solution were directly loaded onto a Superdex 16/60 75pg gel filtration column (Pharmacia), using a 10 ml syringe. The column was pre-equilibrated with running buffer (25mM Hepes pH 7.4, 40 mM NaCl, 0.05 mM EDTA, 10% Glycerol, 1.0 mM TCEP) and the gel filtration chromatography was carried out with a flow rate of 2.0 ml/min with the appropriate running buffer. The collected eluate fractions had a volume of 5.0 ml. Puritiy of eluate fractions was checked by SDS-PAGE (see Figure 14) The general yield after this procedure was 5 ml purified protein solution(C= 2.4 mg/ml; total amount 12 mg). The protein sample was aliquoted (50 µl), shock frozen in liquid nitrogen and stored at -80°C. The results are shown in Figure 14.

### 1.3 Purification of src kinase v2

### 1.3.1 Lysis of cell pellets

Pellets resulting from 2000 ml insect cell culture expression were resuspended in 100 ml ice cooled lysis buffer (20mM Tris, pH 8.3, 10% glycerol , 2mM TCEP, 4µl Benzonase (250 u/µl Merck 1.01656.0001), 0.2mM AEBSF, 0.05% Triton-X-100). 100ml raw extract were filled in rosette glas vial on ice. Cell lysis was carried out under the following conditions: pulse: 50% per second, duration: 4min - brutto, power: 8, size of rosette vessel: ~150ml. Afterwards lysate was clarified by centrifugation for 30 min at 4 °C at ~41800 x g (Evolution RC centrifuge, Sorvall SA-600 rotor). The general yield of cell lysate after this procedure is -600 ml.

### 1.3.2 lon exchange (26/10 HiLoadQ Sepharose)

Ion exchange chromatography was carried out using Äkta purifier 100 (Pharmacia).The obtained celllysate (600 ml) was loaded onto a 26/10 HiLoadQ Sepharose column (Pharmacia), which was subsequently equilibrated with buffer A (20mM Tris pH8.3, 5% glycerol, 0.5mM TCEP).The sample was applied onto the column with a flow rate of 3 ml/min. Afterwards sample loading was finished the column was extensively washed with bufferA until baseline was reached. Afterwards a linear gradient (0%-100% in 100 ml, bufferB (20mM Tris pH8.3, 5% glycerol, 0.5mM TCEP, 1 M NaCl)) was performed. Eluate fractions were collected in 10 ml fractions.

### 1.3.3 Ni-NTA affinity chromatography

The Ni-NTA affinity chromatography was carried out using Äkta prime (Pharmacia Amersham). The eluate fraction obtained from the ion exchange step (200 ml) was loaded onto a HisTrap HP Ni-NTA column (bed volume ~6ml, PharmaciaAmersham), which was subsequently equilibrated in BufferA (50mM sodium phosphate pH 8.0, 500mM NaCl, 20mM imidazole, 5% glycerol, 0.5mM TCEP). The sample was applied to column using the system pump of the Äkta Prime (Pharmacia) with a flow rate of 3 ml/min. Afterwards, the column was extensively washed with Buffer A until baseline was reached. Afterwards a step gradient (0-7% IMAC-B) and a linear gradient, (7-100% in 100 ml, buffer-B(50mM sodium phosphate pH 8.0, 500mM NaCl, 500mM imidazole, 5% glycerol, 0.5mM TCEP)) was performed. Eluate fractions were collected in 10 ml fractions.

### 1.3.4 Buffer change with HiPrep 26/10 desalting column

The desalting gel filtration chromatography was carried out using Äkta purifier 100 (Pharmacia Amersham) and HiPrep 26/10 desalting gelfiltration column (Amersham). The pooled eluate fractions obtained after the Ni-NTA chromatography were loaded onto the column (sample volume = 8 ml, flow rate 2.0 ml/min), which was pre-equilibrated in running buffer (20mM Tris pH 8.3, 5% glycerol, 0.5mM TCEP).

### 1.3.5 Ion exchange MonoQ HR10/10

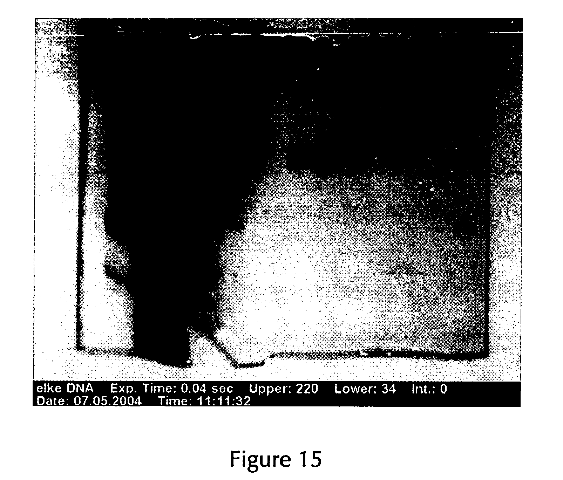

The ion exchange chromatography was carried out using Äkta purifier 100 (Pharmacia Amersham) and a MonoQ HR10/10 anion exchange column (Pharmacia Amersham), which was operated at a flow rate of 2.0 ml/min. The pooled eluate fractions obtained after the buffer change step (V=24 ml) were loaded onto the column, which was subsequently preequilibrated with BufferA (20mM Tris pH8.3, 5% glycerol, 0.5mM TCEP). A linear gradient from 0-20 % BufferB (20mM Tris pH8.3, 5% glycerol, 0.5mM TCEP, 1M NaCl; V=320 ml) was performed and eluate fractions were checked by SDS-PAGE on puritiy. The results are shown in Figure 15.

### 1.3.6 Gelfiltration (HiLoad 26/60 superdex 75pg)

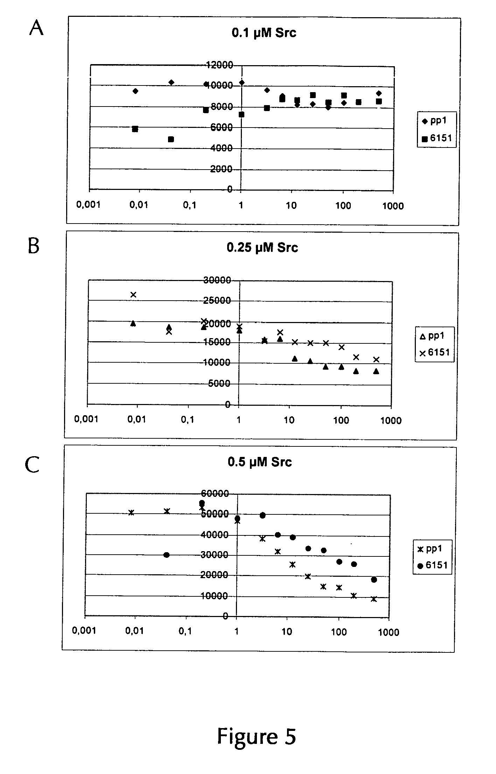

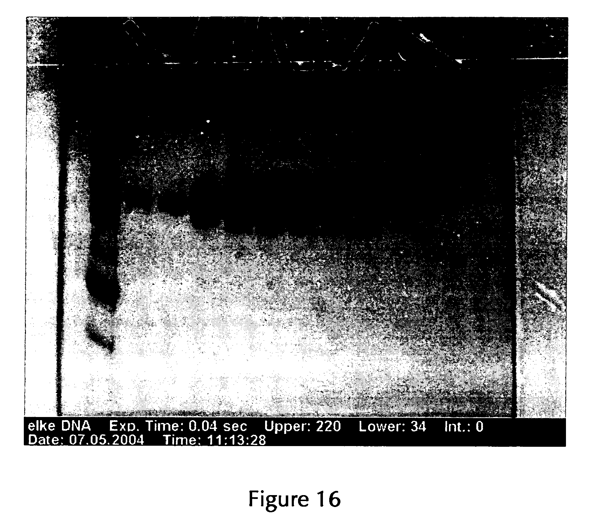

Gelfiltration was carried out using the Äkta purifier 100 (Pharmacia) and a HiLoad 26/60 superdex 75pg gel filtration column (Pharmacia Amersham), which was operated at a flow rate of 2.0 ml/min. The pooled eluate fractions obtained after the anion exchange step (V=5 ml) were directly loaded onto a Superdex 16/60 75pg gel filtration column (Pharmacia), using a 10 ml syringe. The column was preequelibrated with running buffer (25mM Hepes pH 7.4, 40 mM NaCl, 0.05 mM EDTA, 10% Glycerol, 1.0 mM TCEP) and the gel filtration chromatography was carried out with a flow rate of 2.0 ml/min with the appropriate running buffer. The collected eluate fractions had a volume of 5.0 ml. Puritiy of the eluate fractions were checked by SDS-PAGE analysis (Figure. 5). The general yield of after this procedure was 5 ml purified protein solution(C= 1.0 mg/ml; total amount 27 mg). The protein sample was aliquoted (50 µl), shock frozen in liquid nitrogen and stored at -80°C. The results are presented in Figure 16.

### 2. Protein-tyrosine kinase auto-phosphorylation assay:

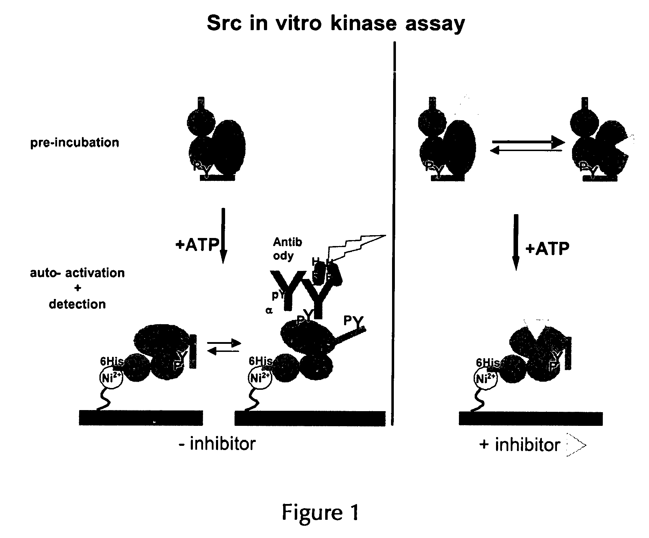

A general principle of the assay performed using a method according the present invention is shown in Figure 1. In the following, exemplary auto-phosphorylation assays using the methods according to the present invention are shown.

### 2.1 Assay Protocol for Src protein tyrosine kinase auto-phosphorylation assay

In a concrete assay protocol 180 µl of 0.25 µM of Src kinasev2 in incubation buffer (50 mM Tris/HCl, pH 7.2, 10 mM MgCl₂, 30% DMSO) are preincubated in a 96 well plate (Nunc, 96Microwell™ Platten konischer Boden, V96, Cat.no. 442587). with 20 µl compound solution (in 100 % DMSO) at the desired concentration for 20 min at room temperature on a rotation shaker (Edmund Bühler, Labortechnik Materialtechnik, Johanna Otto, GmbH) at 300 rpm. After mixing the kinase with the compound the final DMSO concentration was 40%. Then, 190 µl of the preincubated compound/kinase solution are transfered to 96 well Ni-NTA plates (Ni-NTA HisSorb™ plates, white, Qiagen (cat.No.35081) and incubated for 30 min at room temperature on a rotation shaker at 300 rpm to allow binding of the kinase to the wells. The kinase/compound solution containing unbound kinase and compound is carefully replaced by 200 µl of DMSO free initiation buffer (50 mM Tris/HCl, pH 7.2, 10 mM MgCl₂, 100 µM ATP) per well and the plate is incubated for 20 min at room temperature on a rotation shaker at 300 rpm.

The initiation buffer is then removed carefully and the plate is washed two times with PBS-Tween (PBS-T) buffer (50 mM potassium phosphate, pH 7.2, 150 mM NaCl, 0.05 % Tween 20) (200 µl per well per washing step).

Afterwards 200 µl of a solution containing HRP-coupled anti p-Tyr antibody (1:2000 dilution of a polyclonal antiserum (1.0 mg/ml) (Abcam, anti-Phosphotyrosine ab 9329) in PBS-T, 0.5 % bovine serum albumin (BSA) (AppliChem GmbH Albumin Fraktion V, pH 7.0, A1391,0500) is added per well and the plate is incubated for 30 min at room temperature on a rotation shaker at 300 rpm.

The antibody solution is removed carefully and the plate is washed three times with 200 µl PBS-T buffer (200 µl per well per washing step).

Afterwards a chemoluminiscent detection reagent is added (ECL™ Western Blotting Detection Reagents, RPN2106, Amersham Biosciences, 200µl per well) and chemoluminiscence intensitiy is detected and quantified in a plate reader (Wallac 1420 Multilabel counter Victor²).

### 2.2 Verification of src kinase auto-phosphorylation and immobilization on Ni-NTA coated plates

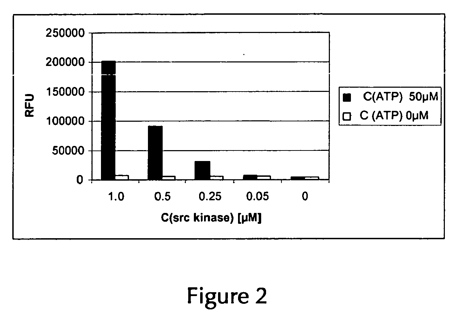

The assay will be performed as a solid phase assay, which can be done by immobilisation of the kinase (Srcv1) via its His₆-tag on Ni-NTA coated 96 wells (Qiagen), as this assay system allows washing steps. Thus it was evaluated, whether it is possible to detect auto-phosphorylated src kinase, immobilized on Ni-NTA coated plates with an HRP labeled anti p-Tyr antibody. Therefore, Src kinase (1.0µM) was incubated for 30 min either in the presence and absence of 50 µM ATP at RT in incubation buffer (50 mM Tris/HCl, pH 7.2, 10 mM MgCl₂). Afterwards the solution was diluted to 1.0, 0.5, 0.25 and 0.0 µM final src protein concentration in PBS and incubated for 30 min (V= 200µl) on NiNTA coated plates at RT on a rotation shaker (300 rpm). After removal of the supernatant and 3 times washing with 200 µl PBS-T, immobilized, phosphorylated src kinase was detected. Therefore, a solution of HRP-labeled anti p-Tyr antibody (Abcam) (200 µl, 1:2000 diluted in PBST-0.5% BSA) was added to the wells and the plate was incubated for 30 min at RT °C, 300 rpm. After removal of the supernatant and 3 times washing with 200 µl PBS-T, chemoluminiscence development buffer was added (200 µl of a 1:1 mixture of ECL substrate, Amersham) and chemoluminiscence signal was quantified using Wallac1420 Multilabel counter (Victor²). The result of the experiment is shown in Figure 2. The diagram shows the chemoluminiscence signal in dependence of the amount of kinase used for immobilization on the Ni-NTA coated 96 wells. With increasing amount of kinase, the signal increases, as the binding sites on the well surface become saturated. In the case of the kinase which was autoactivated by incubation with ATP a correlation between kinase amount, used for immobilization on the Ni-NTA plate and signal can be observed. Thus it is possible to auto-phosphorylate Src and detect auto-phosphorylated Src kinase, that is immobilized and on Ni-NTA plates.

### 2.3 Inhibition of Src auto-phosphorylation by kinase inhibitors

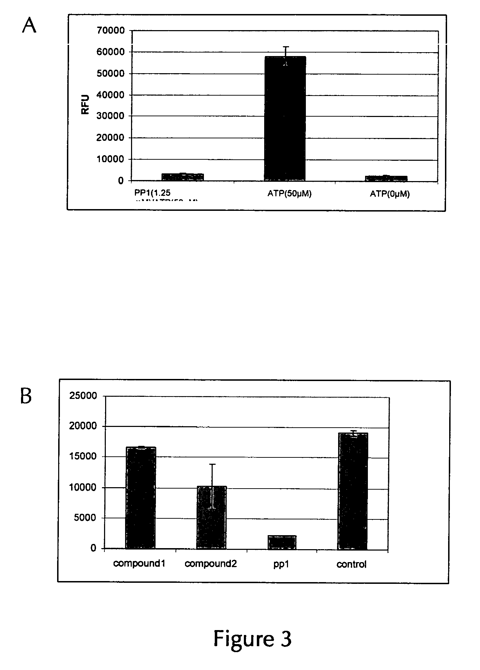

In order to use this assay for identification and characterization of test compounds modulating the kinase activity, it was tested whether auto-phosphorylation can be inhibited by PP1 (Biomol, El-275), a Src kinase inhibitor, binding to the ATP binding site of Src irrespective of its conformation (i.e. closed/open), and other test compounds known to bind only to the src kinase in its closed conformation. The data show that PP1 potently inhibits auto-phosphorylation of src kinase (Srcv1), while compound1 and compound2 showed an inhibition of around 30% (see Figures 3a,3b).

For a first experiment Src kinase (100µl, 2.0 µM) was preincubated for 30 min in the presence of kinase inhibitors (PP1, 1.25 mM) in incubation buffer 50 mM Tris/HCl, pH 7.2, 10 mM MgCl₂, 10% DMSO) in 96 well v-plates (Nunc). Afterwards 100 µl assay buffer containing 100 µM ATP was added (except for the negative control which was incubated with 100 µl assay buffer without ATP) and the mixture was transfered to Ni-NTA coated plates and further incubated for 20 min at RT on a rotation shaker (300 rpm). The degree of src kinase auto-phosphorylation was detected with an anti p-Tyr-antibody as described above (see Figure 3a).

In a second experiment 100 µl src kinase (1.0 µM) were incubated for 30 min with PP1, compound1 and compound2 (1.25 mM) in incubation buffer (50 mM Tris/HCl, pH 7.2, 10 mM MgCl2, 30% DMSO) in 96 well v-plates (Nunc). The solution was transfered on NiNTA plates and incubated for 30 min with 100 µl assay buffer containing 100 µM ATP for the auto-phosphorylation of src. The degree of src kinase auto-phosphorylation was detected with an anti p-Tyr-antibody as described above (see Figure 3b).

### 2.4 Quantitative analysis of inhibition of src autoactivation with kinase inhibitor PP1

It was also tested whether the assay is not only suitable for qualitative analysis but also can be used for quantitative analysis of kinase inhibitior profiles. Therefore, in a scout experiment a PP1 dilution at a constant kinase concentration (Src v1) was prepared, which lead to the PP1 IC₅₀ titration curve. In particular, 200 µl kinase (0.5 µM) were incubated for 30 min with a dilution series of PP1 (500, 200, 100, 50, 25, 12.5, 6.25, 3.125, 1.0, 0.2, 0.04, 0.08 µM) in incubation buffer buffer (50 mM Tris/HCl, pH 7.2, 10 mM MgCl₂, 30% DMSO) in 96 well v-plates (Nunc). 10% DMSO on Ni-NTA plate. Afterwards solution was replaced by 200 µl initiation buffer (assay buffer containing 100µM ATP to the wells and incubated for 7 minutes. Auto-phosphorylation of src was detected with an anti p-Tyr-antibody as described above. The results are shown in Figure 4. On the y-axis the chemoluminiscence signal is displayed, on the x-axis the logarithmic compound concentration in µM is displayed. From the dose response curve obtained by this experiment an IC₅₀ value for PP1 can be determined (IC₅₀~ 10 µM). Thus it can be concluded that the autoactivation assay can be used for quantitative analysis.

### 2.5 Signal/background optimization

In order to optimize the assay performance, the signal-to-background ratio of the assay should be increased. Therefore, it was tested whether it is possible to increase the signal to background ratio of the assay by increasing the amount of kinase used in the assay, as by increasing the amount of kinase an higher occupation of the Ni-NTA coated plates could be achieved. To approve this, dilution series of PP1 and the kinase inhibitor 6151 were carried out at different kinase concentrations (Srcv1). The test compounds PP1 and 6151 were thus titrated (500, 200, 100, 50, 25, 12.5, 6.25, 3.125, 1.0, 0.2, 0.04, 0.08 µM) in the auto-phosphorylation assay at three different kinase concentrations (0.1, 0.25 and 0.5 µM Src kinase) 10 µl of 10-fold compound stock solution in 100% DMSO were incubated with 200 µl kinase at the desired concentration in incubation buffer (50 mM Tris/HCl, pH 7.2, 10 mM MgCl₂, 30% DMSO) in 96 well v-plates (Nunc) for 20 min at RT. Afterwards the solution was transferred to 96 well Ni-NTA plate and incubated for 30 min at RT on a rotation shaker at 300 rpm. The solution was replaced by 200 µl assay buffer, containing 100µM ATP and the plate was incubated for 30 min at RT on a rotation shaker. The degree of src kinase auto-phosphorylation was detected with an anti p-Tyr-antibody as described above. The results are displayed in Figures 5a and b. The chemoluminiscence signal is displayed on the y-axis of each figure, the logarithmic compound concentration in µM on the x-axis. As shown, the best signal/noise ratios were obtained at a protein concentration of 0.5 µM Srcv1.

As described above Srcv2 has the advantage over Srcv1 that it is present predominantly in its closed conformation, as its C-terminal modification leads to an increased affinity of pY-530 to the internal SH2 domain of Src, which in turn keeps the kinase in its closed conformation. Thus it was assumed that the use of Srcv2 instead of Srcv1 should give better results in the assay, as during preincubation of the kinase with compound the kinase is predominantly present in its closed conformation and thus the background signal should be decreased.

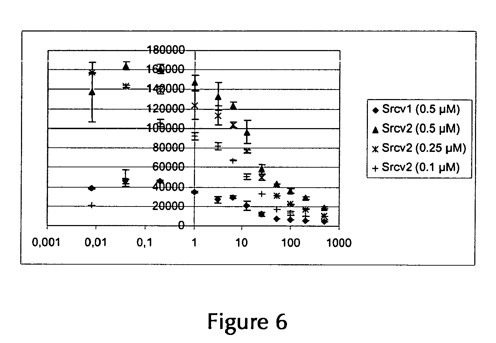

To evaluate whether better results were obtained if Srcv2 is used instead of Srcv1PP1 titration curves were carried out at different protein concentrations of Srcv1 and Srcv2. Therefore, Srcv1 and Srcv2 were compared in the autoactivation assay, wherein two test compounds (PP1, CS6151 binding the closed conformation only) were titrated (500, 200, 100, 50, 25, 12.5, 6.25, 3.125, 1.0, 0.2, 0.04, 0.08 µM) at three different kinase concentrations (0.1, 0.25 and 0.5 µM Src kinase v1) and the mutated form Src v2 (0.5 µM), in order to compare the assay performance with these two proteins.10 µl of 10 fold compound stock in 100% DMSO were incubated with 200 µl kinase at the desired concentration in incubation buffer (50 mM Tris/HCl, pH 7.2, 10 mM MgCl₂, 30% DMSO) in 96 well v-plates (Nunc) for 20 min in 96 v well plates at RT. Afterwards the solution was transferred to 96 well Ni-NTA plate and incubated for 30 min at RT on a rotation shaker (300 rpm). Solution was removed and initiation buffer (200 µl assay buffer, containing 100µM ATP) was added and the plate was incubated for 30 min at RT on a rotation shaker (300 rpm). The degree of src kinase auto-phosphorylation was detected with an anti p-Tyr-antibody as described above. The result of the experiment is shown in Figure 6. On the y-axis the chemoluminiscence signal is displayed, on the x-axis the logarithmic compound concentration in µM is displayed. As shown, if Srcv2 is used instead of Srcv1, much better titration curves were obtained even at lower protein concentrations (even with 0.25 µM Srcv2 a four-fold signal increase was observed). Thus for optimizing the assay performance, Srcv2 will be used for further experiments.

The increased signal intensity at low inhibitor concentrations observed for Srcv2 might be explained with the fact that Srcv2 is expressed and purified with a phosphorylation at Y530, while Srcv1 is completly non phosphorylated (this data can be confirmed by x-ray structure analysis). It can be assumed that during autoactivation pY-530 is released from the internal SH2 domain and can be detected by an anti p-Tyr antibody. Thus in the case of Srcv2 two instead of one P-Tyr's were detected per activated kinase molecule and this might lead to a stronger signal in the assay.

### 2.7 Further examples of qualitative and quantitative analysis of src inhibitors tested in the method according to the present invention

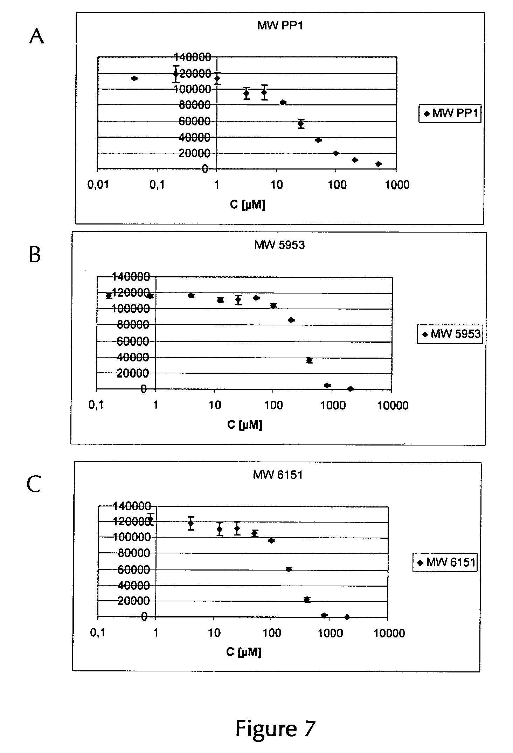

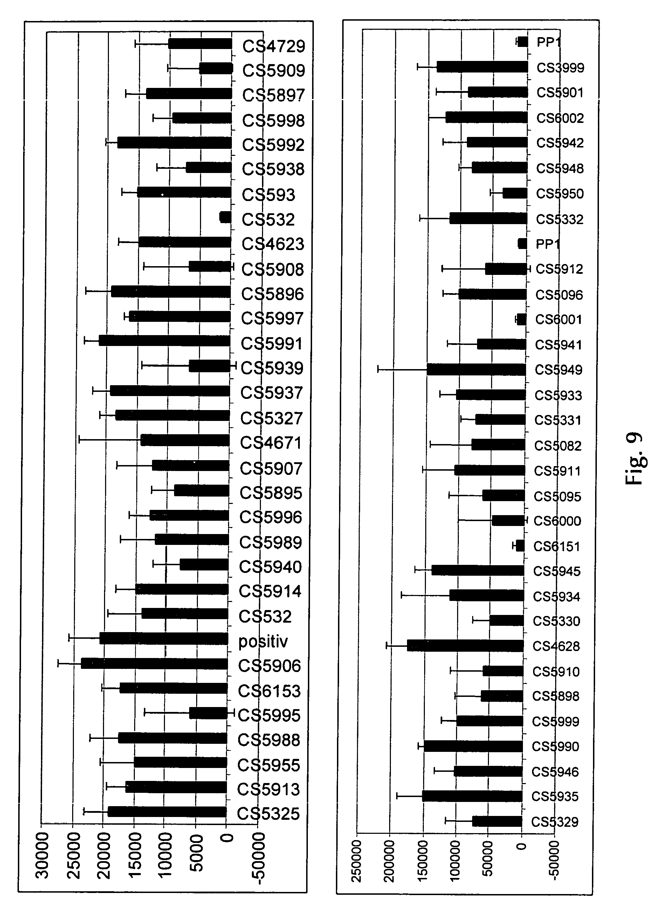

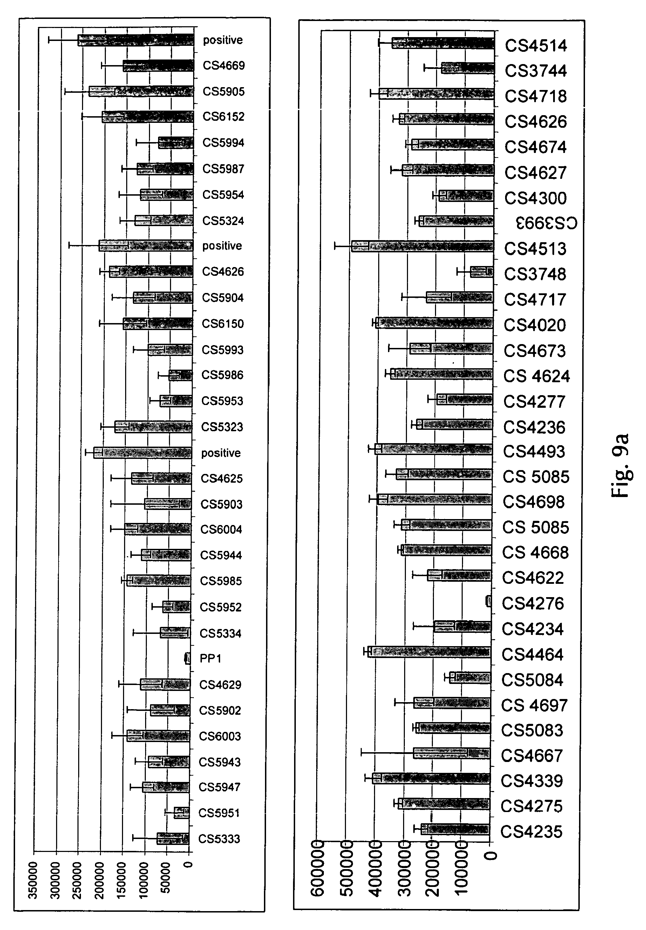

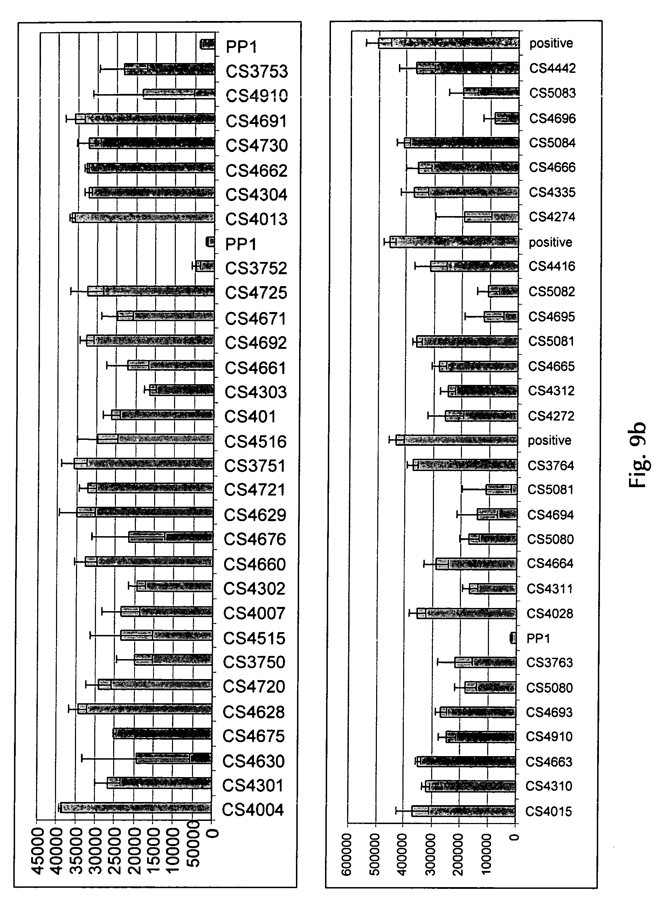

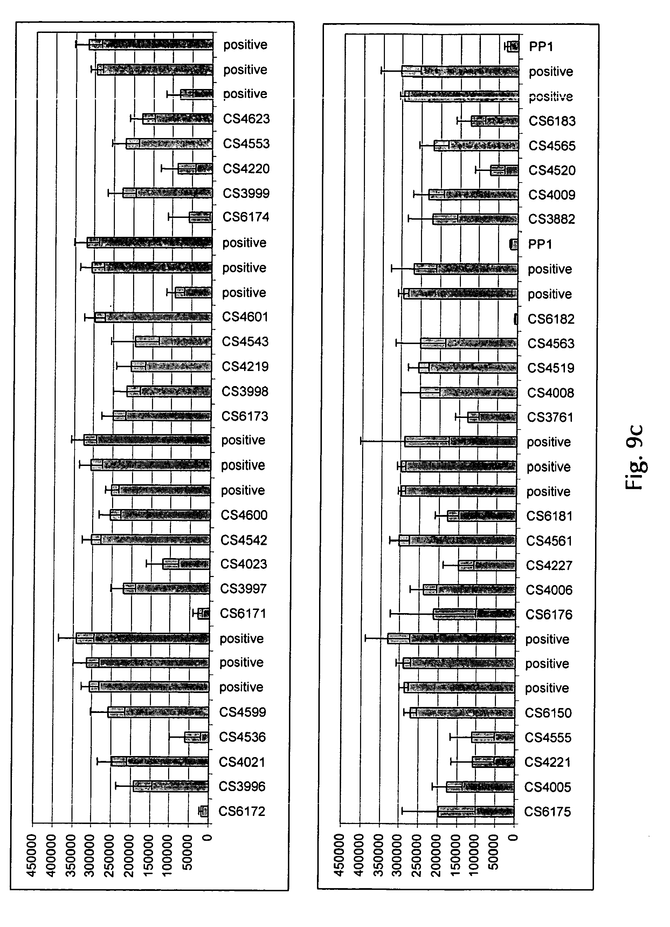

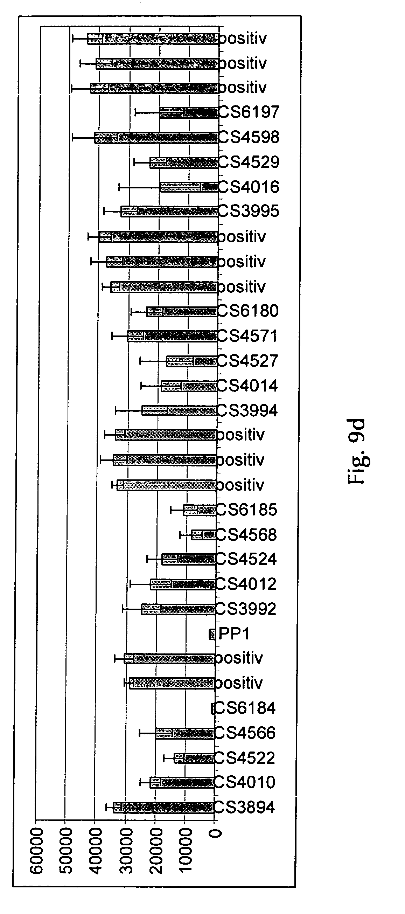

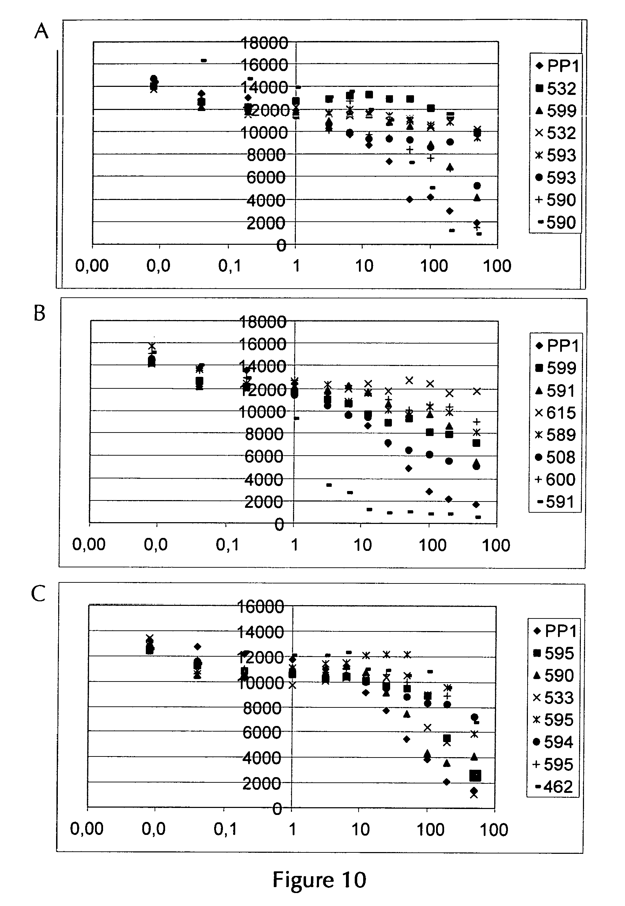

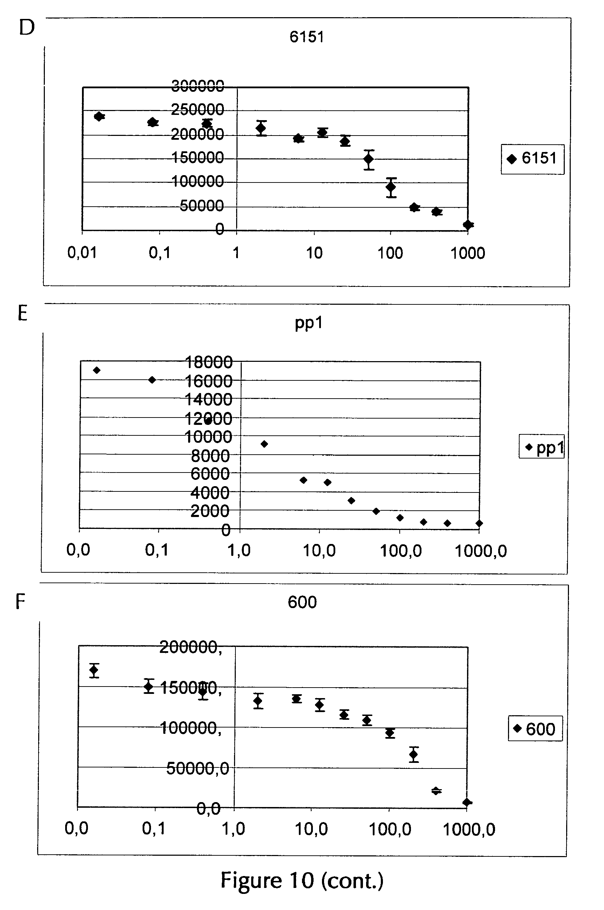

2.7.1 The assay was used for qualitative and quantitative analysis of different compound classes. One of the compound classes binds to the closed conformation. of src kinase only. The assay was performed either manually or in a semiautomatic approach using the EP3 robotor (Perkin Elmer) for automated pipetting steps. Furthermore, commercially available Src inhibitors (PP1, (Biomol) and Src inhibitor I (Calbiochem, Cat. No. 567805) and Src inhibitor II (Calbiochem, Cat. No. 567806) were analysed for their inhibitory profile in the Src autoactivation assay as reference control.
   The test compounds were titrated in the autoactivation assay with (0.25 µM Srcv2 kinase). 10 µl of 10 fold compound stock in 100% DMSO were incubated with 200 µl kinase at the desired concentration in assay buffer without ATP, 30% DMSO for 20 min in 96 well plates at RT. Afterwards the solution was transferred to 96 well Ni-NTA plate and incubated for 30 min at RT on a rotation shaker. Solution was removed and 200 µl assay buffer, containing 100µM ATP was added and the plate was incubated for 30 min at RT on a rotation shaker. Solution was removed and wells were washed 3 times with 200µl PBST. Afterwards 200 µl 1:2000 diluted anti p-Tyr antibody in PBST-0.5% BSA was added and the plate was incubated for 30 min at RT on a rotation shaker. The degree of src kinase auto-phosphorylation was detected with an anti p-Tyr-antibody as described above. The results are shown in Figure 7. On the y-axis the chemoluminiscence signal is displayed, on the x-axis the logarithmic compound concentration in µM is displayed. Each of the investigated inhibitors showed inhibition of Srcv2 kinase.
2.7.2 In a further experiment, IC 50 titration curves of the two src inhibitors Src kinase inhibitor I (Calbiochem, Cat. No. 567805); Src Kinase Inhibitor II (Calbiochem, Cat. No. 567806) were performed in duplicates. The two src kinase inhibitors provided by Calbiochem show the same inhibition profile in the src autoactivation assay as described in literatur. The results are presented in Figure 8. While the inhibitor I shows a stronger inhibition than PP1, the inhibitor II shows weak to no inhibition in the autoactivation assay. On the y-axis the chemoluminiscence signal is displayed, on the x-axis the logarithmic compound concentration in µM is displayed.
2.7.3 In an additional experiment 96 potential inhibitors were analyzed using the method according to the present invention as disclosed above. The test compounds had a final concentration of 500 µM in the assay. The DMSO concentration was 30 %. Test compounds were defined as hits if the residual activity was beyond a defined threshold (see Figure 9).
2.7.4 Finally, dilution series of test compounds found as hits in the qualitative assay, for lC50 determination were performed using the same reaction conditions as disclosed above. The measurements were carried out either as unicates or as triplicates. The results are shown in Figure 10. The standard deviation is shown by error bars. On the y-axis the chemoluminiscence signal is displayed, on the x-axis the logarithmic compound concentration in µM is displayed.

### 2.8 Examples of qualitative and quantitative analysis of hck inhibitors tested in the auto-phosphorylation assay

Quantitative compound screening was also carried out with a 2^{nd} protein tyrosine kinase, called hck using identical reaction conditions as disclosed above for Srcv2. For this experiment a mutated form of hck was used having the same C-terminal modification as Srcv2. The results are shown in Figure 11. The diagrams show dilution series of test compounds for IC50 determination with the kinase hck. The measurements were carried out as triplicates, where the standard deviation is shown by error bars. On the y-axis the chemoluminiscence signal is displayed, on the x-axis the logarithmic compound concentration in µM is displayed.

### 3. Further analysis

### 3.1 Statisitcal analysis of the auto-phosphorylation assay: Determination of z'

In order to get information about the quality of the assay the z' was determined for the assay. For determination of the maximal counts src v2 in the absence of inhibitor was used. For determination of the assay minimum, inhibition of srcv2 with 500 µM PP1 was carried out.

**Table1: The test compounds PP1 and 6151 were titrated (500, 200, 100, 50, 25, 12.5, 6.25, 3.125, 1.0, 0.2, 0.04, 0.08 µM) in the auto-phosphorylation assay at three different kinase concentrations**

| | PP1 (500 µM) | PP1 (0 µM) | PP1 (500 µM) | PP1 (0 µM) |
|---|---|---|---|---|
| | 5816 | 156929 | 6060 | 124288 |
| | 5922 | 144133 | 5465 | 130523 |
| | 5781 | 146417 | 5523 | 130217 |
| | 6275 | 139552 | 5752 | 140377 |
| mean | 5948 | 146758 | 5700 | 131351 |
| standard deviation | 196 | 6371 | 234 | 5773 |
| Z' | 0,87 | | 0,86 | |

### 3.2 DMSO tolerance of src kinase

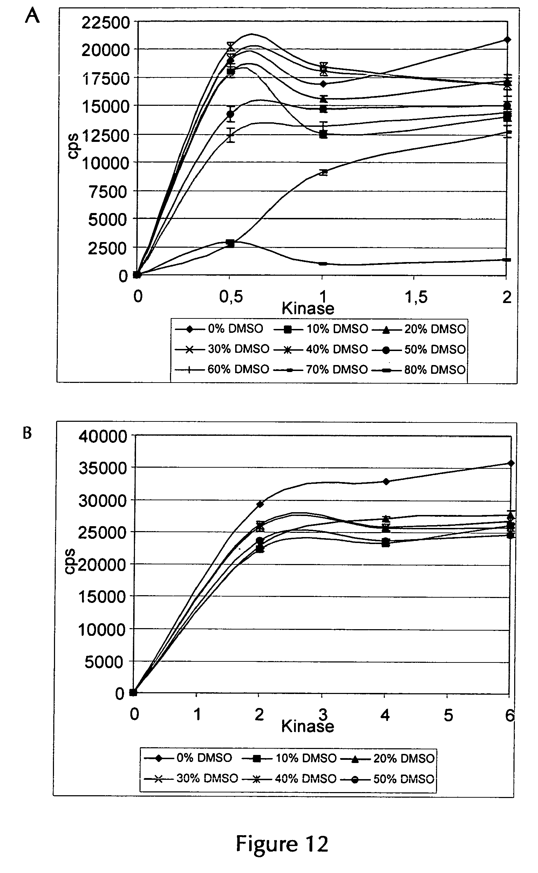

That src kinase tolerates up to 40% DMSO during kinase reaction was shown in a kinase activity (assay alpha screen kinase assay, for detailed description visit Perkin Elemer web site), which monitored phosphorylation of a synthetic poly GT peptide over the time. After kinase reaction the assay solution was adjusted to 10 % DMSO before performing the alpha screen as the alphascreen itself tolerates up to 10% DMSO only. To ensure that, the kinase has a high DMSO tolerance, kinase dilutions were carried out using lower kinase concentrations and DMSO concentrations up to 80%. Therefore 20 µl Kinase were mixed with 20 µl peptide solution and incubated for 30 min at 27.2 °C. Kinase reaction was stopped by addition of 60 µl alpha screen buffer. Suspension was incubated for 45 min at 27.2 °C 3 x 25 µl of each time point were transfered to a 384 well opti plate Readout of assay was carried out at the fusion alpha screen reader using the kinase program (laser exposure time 1s). Kinase dilution series were carried out at increasing DMSO concentrations (0-50%). At the kinase concentrations used in the assay no significant effect of DMSO could be observed. At DMSO concentrations up to 40 % no decrease in kinase activity could be observed. DMSO concentrations > 40 % lead to a decrease in kinase activity. Thus the kinase reaction can be carried out in the presence of 30 % DMSO without affecting the kinase activity. The results are shown in Figures 12a and b, displaying the readout of kinase activity.

### 4. Comparison of full length Src kinase and the phosphatase treated catalytic domain of Src kinase in the autoactivation assay

Similar experiments as shown above for Src and Hck were carried out for a fragment of Src kinase comprising the catalytic domain of Src kinase (SEQ ID NO: 13)

### 4.1 Purification and dephosphorylation of Src catalytic domain

### 4.1.1 Lysis of E. coli BL21 STAR(DE3) cells

Pellets resulting from 5000 ml *E.coli* BL21 STAR(DE3) culture were resuspended in 250 ml ice cooled lysis buffer (20mM Tris, pH 8.3, 10% glycerol , 2mM TCEP, 4µl Benzonase (250 u/µl Merck 1.01656.0001), 0.2mM AEBSF, 0.05% Triton-X-100). 100ml raw extract were filled in rosette glas vial on ice. Cell lysis was carried out under the following conditions: pulse: 50% per second, duration: 4min - brutto, power: 8, size of rosette vessel: ~150ml. Afterwards lysate was clarified by centrifugation for 35 min at 4 °C at ~41800 x g (Evolution RC centrifuge, Sorvall SA-600 rotor). Supernatant was pooled and filtered through a sartorius 0.45µm CA membrane with 5µm GF prefilter. The general yield of cell lysate after this procedure is ~400ml-600 ml.

### 4.1.2 Ni affinity chromatography

The obtained cell lysate (400 ml) was loaded onto a Ni-NTA column (bed volume ~6ml His Trap matrix, Amersham), which was subsequently equilibrated in IMAC-A bufferA (50 mM NaH₂PO₄, pH 8.0, 300 mM NaCl, 10 mM Imidazol, 5% Glycerol1 mM TCEP). The sample was applied to column using the system pump of the Äkta Purifier (Pharmacia) with a flow rate of 3 ml/min. Afterwards sample loading was finished the column was extensively washed with IMAC-A buffer until baseline was reached. Afterwards a step gradient (0- 10% IMAC-B buffer (50 mM NaH₂PO₄, pH 8.0, 500 mM NaCl, 500 mM Imidazol, 5% Glycerol, 1mM TCEP)) and a linear gradient ,(10%-100% in 100 ml, IMAC-B buffer) was performed. Eluate fractions were collected in 10 ml fractions. Puritiy of eluate fractions was checked by SDS-PAGE and eluate fractions were pooled subsequently. The general yield of after this procedure is 100-150 ml.

### 4.1.3 Desalting

For the next purification step a buffer change in 20mM Tris pH 8.3, 50mM NaCl, 10% glycerol, 1 mM TCEP was carried out. Therfore the sample (V 10) was loaded onto a desalting column (26/10, Pharmacia), preequalibrated in 20mM Tris pH 8.3, 50mM NaCl, 10% glycerol, 1 mM TCEP. The sample was eluted at a flow rate of 1-1.5ml/min and the corresponding eluate fractions were pooled.

### 4.1.4 Dephosphorylation with lambda phosphatase

The kinase was dephosphorylated by adding 1/15 molar lambda phosphatase to the kinase solution. The solution was incubated in the presence of 2mM MnCl₂ at 4°C over night for dephosphorylation.

### 4.1.5 lon exchange

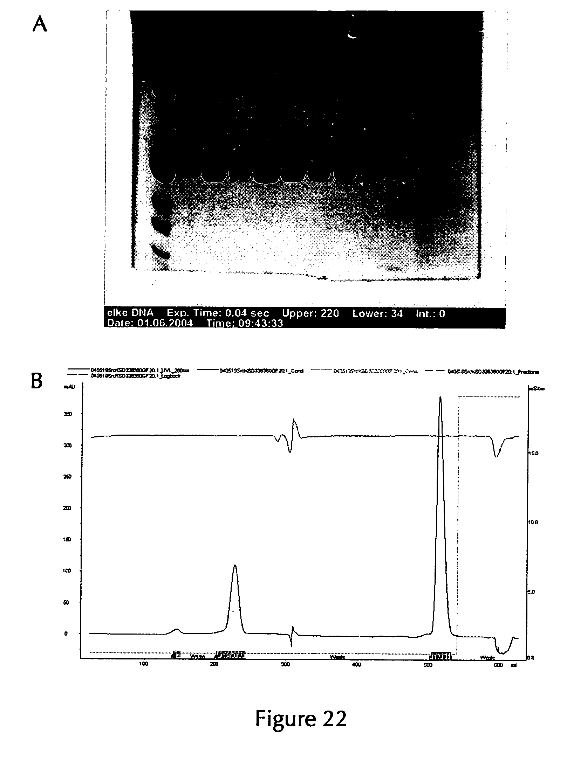

Ion exchange chromatography was carried out using the Äkta purifier 100 (Pharmacia) at a flow rate of 1.5 ml/min. The protein solution obtained after the desalting step was directly loaded onto a MonoQ HR 10/10 column (Pharmacia), preequelibrated with loading buffer (20mM Tris pH 8.3, 5% glycerol, 1 mM TCEP). After extensive washing with loading buffer the protein was eluted in a linear gradient 0-30% elution buffer (20mM Tris pH 8.3, 5% glycerol, 1 M NaCl, 1 mM TCEP) in 480 ml. Puritiy of eluate fractions was checked by SDS-PAGE (see Figure 22A).

### 4.1.6 Gelfiltration

Gelfiltration was carried out using the Äkta purifier 100 (Pharmacia). The volume of the protein solution obtained after the ion exchange chromatography step was reduced to -5 ml using Centriprep YM-10 concentrators (Amicon). The protein solution were directly loaded onto a HiLoad 26/10 superdex 200pg gel filtration column (Pharmacia), using a 10 ml syringe. The column was preequelibrated with running buffer (25mM Hepes pH 7.4, 40 mM NaCl, 0.05 mM EDTA, 10% Glycerol, 1.0 mM TCEP) and the gel filtration chromatography was carried out with a flow rate of 2.0 ml/min with the appropriate running buffer. The collected eluate fractions had a volume of 5.0 ml. The general yield of after this procedure was 5 ml purified protein solution(C= 2.4 mg/ml; total amount 12 mg). (see Figure 22B) The protein sample was aliquoted (50 µl), shock frozen in liquid nitrogen and stored at -80°C.

### 4.2 Autoactivation assay with full length Src kinase and phosphatase treated catalytic domain of src kinase.

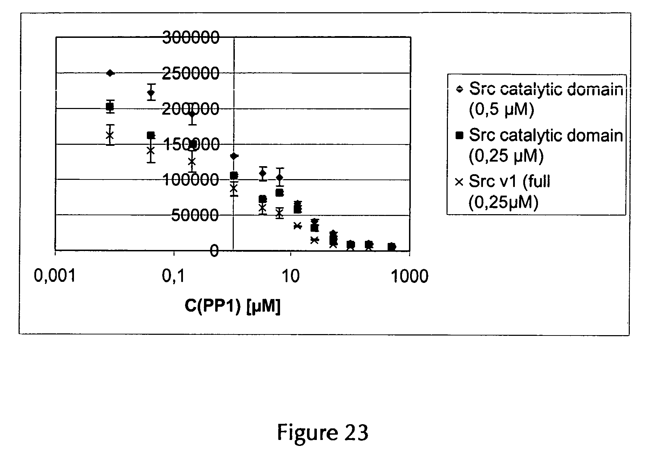

PP1 titration curves were carried out with full length Src kinase and phosphatase treated catalytic domain of src kinase (6His-Scr catalytic domain) in the same manner as shown above for full-length Src kinase. As a result, the phosphatase treated inactive catalytic domain of Src can be also used in the autoactivation assay and shows a similar activity. Similar dose response curves were obtained for both proteins (see Figure 23 for full-length Src kinase and catalytic domain of Src kinase (6His-Scr catalytic domain)).

### Advantages of the present invention:

The method according to the present invention for identifying, selecting and/or characterizing a compound, which modulates the activity of at least one protein-tyrosine kinase (PTK), displays several important advantages over other kinase assays of the prior art:
i) The inventive method provides the possibility to determine the degree of auto-phosphorylation of a non-phosphorylated PTK and also to quantify the potency of test compounds binding to the open and in particular to the closed conformation of a PTK.
ii) Many protein kinases use as a substrate(s) some of their own sequences (auto-phosphorylation), which allows to determine and quantify the degree of their activity in vitro, obviating the need for any external substrate. The inventive method thus avoids costs and labor for external substrate (usually peptide).
iii) As the auto-phosphorylation according to the inventive method is a reaction of 0^{th} order, it is independent of the kinase concentration and thus easy to set up and evaluate. Kinetic aspects, like Kₘ for the substrate, which can complicate an assay, do not play a role in the inventive method.
iv) Due to immobilization of the kinase on a solid phase, trans-phosphorylation of kinase molecules is excluded, which provides for a direct correlation of compound inhibition and phosphorylation signal. Furthermore, the phosphorylation signal is solely an auto-phosphorylation signal.
v) As auto-phosphorylation is an intramolecular reaction the kinase auto-phosphorylation can be carried out in very short time frames (within minutes), which is an advantage if instable protein and/or test compounds are used.
vi) For many protein kinases autoactivation by auto-phosphorylation occurs as first step and therefore is an important event potentiating kinase activity. Thus the kinase autoactivation is a physiological relevant readout, whereas the use of isolated synthetic peptide substrates, which is currently state-of-the-art, may entail artefactual results.
vii) Substrate-compound interactions, potentially leading to false positive or false negative hits in screening assays, can be excluded as no substrate binding to the kinase protein occurs.
viii) Due to its simple set-up (see iii) the method according to the present invention can easily be employed in high throughput screening assays (HTS).

## Claims

1. Method for identifying, selecting and/or characterizing a compound which modulates the activity of at least one protein-tyrosine kinase (PTK), comprising the steps of:
a) Providing a non-phosphorylated protein-tyrosine kinase; and
b) Providing a test compound; and
c) Contacting the non-phosphorylated protein-tyrosine kinase with the test compound; and
d) Activating auto-phosphorylation of the non-phosphorylated protein-tyrosine kinase; and
e) Measuring the degree of phosphorylation of the protein-tyrosine kinase via biochemical methods.

2. Method according to claim 1, wherein after step (c) the non-phosphorylated protein-tyrosine kinase is immobilized on a solid surface.

3. Method according to any of claims 1 or 2, wherein the immobilization occurs via a group selected from: Maltose Binding protein (MBP), Gluthation-S-transferase (GST), Chitin Binding domain (CBD), His-tag, Strep-tag II (WSHPQFEK), FLAG-tag [(M)DYKDDDDK)], T7-tag, S-tag, Protein A, PinPoint-tag or Thioredoxine.

4. Method according to any of claims 1 to 3, wherein a washing step is inserted after step (c).

5. Method according to any of claims 1 to 4, wherein auto-phosphorylation of the non-phosphorylated protein-tyrosine kinase is initiated by adding ATP.

6. Method according to any of claims 1 to 5, wherein the biochemical method for determining the degree of phosphorylation of the protein-tyrosine kinase is suitable for quantitative and/or qualitative determination.

7. Method according to any of claims 1 to 6, wherein the biochemical method for determining the degree of phosphorylation of the protein-tyrosine kinase is selected from immunological methods, ELISA-assays, Western-Blots, antibody detection, detection via protein protein interactions, detection of absorption, such as fluorescence detection, fluorescence depolarisaton, chemoluminiscence detection, bioluminescence detection, detection of radioactive ligands, detection via small chemical or biochemical molecules, such as alkaline phosphatases, GFP or EGFP, Gluthathione S-transferase, or antibody binding assays.

8. Method according to any of claims 1 to 7, wherein the degree of phosphorylation of the protein-tyrosine kinase is determined by enzyme reaction of horse radish peroxidase (HRP)-labelled anti-phospho-tyrosine (a-PY), or anti-phosphotyrosine recombinant 4G10, or monoclonal anti-phosphotyrosine peroxidase-conjugate from mouse.

9. Method according to any of claims 1 to 8, wherein the protein-tyrosine kinase is selected from a wildtype (wt) protein-tyrosine kinase.

10. Method according to claim 9, wherein the (wt) protein-tyrosine kinase is selected from the family of Src kinases.

11. Method according to claim 10, wherein the wildtype (wt) Src family kinase is selected from Src, Fyn, Yes, Fgr, Lyn, Hck, Lck, Blk and Yrk.

12. Method according to any of claims 9 to 11, wherein the (wt) Src protein-tyrosine kinase is derived from any of SEQ ID NOs: 4 to 12.

13. Method according to any of claims 1 to 8, wherein the protein-tyrosine kinase is selected from a mutated (wt) protein-tyrosine kinase.

14. Method according to claim 13, wherein the mutated (wt) protein-tyrosine kinase is selected from the family of Src kinases.

15. Method according to claim 14, wherein the mutated (wt) Src family kinase displays a hyperactive form of a Src family kinase.

16. Method according to any of claims 13 to 15, wherein the mutated Src protein-tyrosine kinase is derived from any of SEQ ID NOs: 1 to 3 or 13.

17. Compound identified, selected and/or **characterized by** a method of any of claims 1 to 16.

18. Compound according to claim 17 as a medicament, particularly for the treatment of diseases, which are caused at least in part by a PTK, preferably cancer, diseases caused by metabolic defects, diabetes, particularly diabetes type 1 and 2, diseases caused by developmental defects or degeneration, hypercalcemia, restenosis, osteoporosis, osteoarthritis, symptomatic treatment of bone metastasis, rheumatoid arthritis, inflammatory bowel disease, multiple sclerosis, psoriasis, lupus, graft vs. host disease, T-cell mediated hypersensitivity disease, Hashimoto's thyroiditis, Guillain-Barre syndrome, chronic obstructive pulmonary disorder, contact dermatitis, Paget's disease, asthma, ischemic or reperfusion injury, allergic disease, atopic dermatitis or allergic rhinitis.

19. Use of a compound according to claim 17 for preparing a pharmaceutical composition for the treatment of diseases, which are caused at least in part by a PTK, preferably cancer, diseases caused by metabolic defects, diabetes, particularly diabetes type 1 and 2, diseases caused by developmental defects or degeneration, hypercalcemia, restenosis, osteoporosis, osteoarthritis, symptomatic treatment of bone metastasis, rheumatoid arthritis, inflammatory bowel disease, multiple sclerosis, psoriasis, lupus, graft vs. host disease, T-cell mediated hypersensitivity disease, Hashimoto's thyroiditis, Guillain-Barre syndrome, chronic obstructive pulmonary disorder, contact dermatitis, Paget's disease, asthma, ischemic or reperfusion injury, allergic disease, atopic dermatitis or allergic rhinitis.

20. Pharmaceutical composition containing a compound according to claim 17 and a pharmaceutically acceptable carrier, adjuvant or vehicle.

21. Pharmaceutical composition according to claim 20, wherein the pharmaceutical composition is suitable for the treatment of diseases, which are caused at least in part by a PTK, preferably cancer, diseases caused by metabolic defects, diabetes, particularly diabetes type 1 and 2, diseases caused by developmental defects or degeneration, hypercalcemia, restenosis, osteoporosis, osteoarthritis, symptomatic treatment of bone metastasis, rheumatoid arthritis, inflammatory bowel disease, multiple sclerosis, psoriasis, lupus, graft vs. host disease, T-cell mediated hypersensitivity disease, Hashimoto's thyroiditis, Guillain-Barre syndrome, chronic obstructive pulmonary disorder, contact dermatitis, Paget's disease, asthma, ischemic or reperfusion injury, allergic disease, atopic dermatitis or allergic rhinitis.
